# EUROPÄISCHE PATENTSCHRIFT

(11) **EP 0 880 509 B1**
(45) Veröffentlichungstag und Bekanntmachung des Hinweises auf die Patenterteilung: **12.11.2003**
(21) Anmeldenummer: 97904373.4
(22) Anmeldetag: 03.02.1997
(51) Int. Cl.: C07D 251/18, A01N 43/68, C07D 405/14, C07D 409/12, C07D 409/14, C07D 411/12, C07D 405/12, C07D 401/12, C07D 413/12, C07D 417/12, C07D 403/12

(54) **2,4-DIAMINO-1,3,5-TRIAZINE, VERFAHREN ZU DEREN HERSTELLUNG UND DEREN VERWENDUNG ALS HERBIZIDE UND PFLANZENWACHSTUMSREGULATOREN**
2,4-DIAMINO-1,3,5-TRIAZINES, PROCESSES FOR THEIR PREPARATION AND THEIR USE AS HERBICIDES AND PLANT-GROWTH REGULATORS
2,4-DIAMINO-1,3,5-TRIAZINES, PROCEDES PERMETTANT DE LES PREPARER ET DE LES UTILISER COMME HERBICIDES ET REGULATEURS DE CROISSANCE VEGETALE

(30) Priorität: 06.02.1996 DE 19604191
(43) Veröffentlichungstag der Anmeldung: 02.12.1998
(73) Patentinhaber: Bayer CropScience GmbH, 65929 Frankfurt/Main (DE)
(72) Erfinder: GIENCKE, Wolfgang, D-65719 Hofheim (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9700472
(87) Internationale Veröffentlichungsnummer: WO97029095

(56) Entgegenhaltungen:
- EP-A- 0 043 194
- EP-A- 0 213 080
- EP-A- 0 283 522
- EP-A- 0 338 793
- EP-A- 0 352 613
- EP-A- 0 361 577
- EP-A- 0 411 153
- EP-A- 0 436 435
- EP-A- 0 483 667
- EP-A- 0 534 859
- EP-A- 0 589 037
- EP-A- 0 620 220
- EP-A- 0 661 274
- EP-A- 0 678 579
- EP-A- 0 683 236
- EP-A- 0 749 970
- WO-A-95/08527
- WO-A-95/24393
- CH-A- 485 647
- DE-A- 19 522 137
- FR-A- 2 000 204
- GB-A- 1 166 538
- US-A- 3 821 228
- US-A- 3 829 573
- US-A- 3 929 784
- DATABASE WPI Week 9628 Derwent Publications Ltd., London, GB; AN 96-272740 XP002030579 & JP 08 113 554 A (ZERIA SHINYAKU KOGYO K.K.) , 7.Mai 1996
- CHEMICAL ABSTRACTS, vol. 92, no. 9, 3.März 1980 Columbus, Ohio, US; abstract no. 69219v, XP002030574 & XENOBIOTICA, Bd. 9, Nr. 5, 1979, Seiten 323-332, N.M. WOOLHOUSE AND AL.:
- CHEMICAL ABSTRACTS, vol. 92, no. 23, 9.Juni 1980 Columbus, Ohio, US; abstract no. 191078p, XP002030575 & JOURNAL OF MEDICINAL CHEMISTRY, Bd. 23, Nr. 5, 1980, WASHINGTON US, Seiten 502-505, J.G. CANNON ET AL.:
- CHEMICAL ABSTRACTS, vol. 87, no. 5, 1.August 1977 Columbus, Ohio, US; abstract no. 33435v, XP002030576 & JOURNAL OF MEDICINAL CHEMISTRY, Bd. 20, Nr. 6, 1977, WASHINGTON US, Seiten 771-776, R.F. BORNE ET AL.:
- CHEMICAL ABSTRACTS, vol. 85, no. 17, 25.Oktober 1976 Columbus, Ohio, US; abstract no. 122909d, XP002030577 & KRATK. TEZISY - VSES. SOVESHCH. PROBL. MEKH. GETEROLITICHESKIKH REAKTS., 1974, Seiten 103-104, A.G. SAKHABUTDINOV ET AL.:
- CHEMICAL ABSTRACTS, vol. 70, no. 1, 6.Januar 1969 Columbus, Ohio, US; abstract no. 3950r, XP002030578 & JOURNAL OF MEDICINAL CHEMISTRY, Bd. 11, Nr. 6, 1968, WASHINGTON US, Seiten 1161-1164, W.B. WRIGHT JR.:

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß in 6-Stellung substituierte 2-Amino-4-(phenoxyalkyl-amino)-1,3,5-triazine herbizide und pflanzenwachstumsregulierende Eigenschaften besitzen; vgl. WO 94/24086, EP-A-509544, EP-A-492615. Weiterhin sind bereits 2-Amino-4-[arylamino- oder (hetero)arylalkyl-amino]-6-haloalkyl-1,3,5-triazine mit herbizider Wirkung bekannt; vgl. US-A-3,816,419, WO 90/09378, WO 88/02368.

Die bekannten Wirkstoffe mit einer 4-[(Hetero)arylalky)-amino]-gruppe enthalten dabei als "Alkyl" jeweils eine Meₜhylenbrücke, welche gegebenenfalls noch verzweigt substituiert ist.

Die bekannten Wirkstoffe weisen bei ihrer Anwendung teilweise Nachteile auf, sei es unzureichende herbizide Wirkung gegen Schadpflanzen, zu geringes Spektrum der Schadpflanzen, das mit einem Wirkstoff bekämpft werden kann, oder zu geringe Selektivität in Nutzpflanzenkulturen. Andere Wirkstoffe lassen sich wegen schwer zugänglicher Vorprodukte und Reagenzien im industriellen Maßstab nicht wirtschaftlich herstellen oder besitzen nur unzureichende chemische Stabilitäten.

Aufgabe der Erfindung ist es, alternative Wirkstoffe vom Typ der 2,4-Diamino-1,3,5-Triazine bereitzustellen, die als Herbizide oder Pflanzenwachstumsregulatoren eingesetzt werden können.

In der Deutschen Patentanmeldung Nr. 19 522 137.0 werden unter anderen Herbizide des genannten Typs vorgeschlagen, die in 6-Stellung am Triazinring einen gegebenenfalls substituierten Cycloalkylrest oder einen Heterocyclus mit einem Sauerstoff-, Stickstoff- oder Schwefelatom enthalten und in 4-Stellung einen Phenylalkylrest mit einer linearen Propylenbrücke aufweisen, die gegebenenfalls noch verzweigt substituiert ist.

Gegenstand der vorliegenden Erfindung sind Verbindungen der Formel (I) und deren Salze, 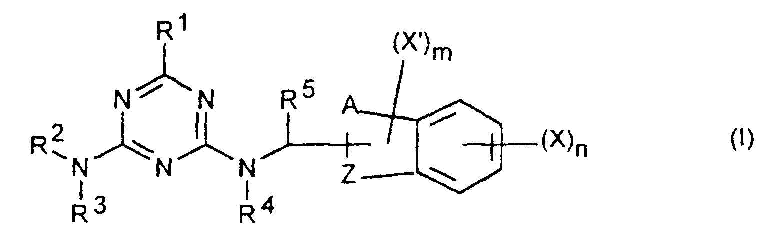 worin
- R¹: (C₁-C₆)Alkyl,
das unsubstituiert oder durch einen oder mehrere Reste aus der Gruppe Halogen, Cyano, Nitro, Thiocyanato, Hydroxy, (C₁-C₄)Alkoxy, (C₁-C₄)Alkylthio, (C₁-C₄)Alkylsulfinyl, (C₁-C₄)-Alkylsulfonyl, (C₂-C₄)Alkenyl, (C₂-C₄)Alkinyl, Phenyl, das unsubstituiert oder substituiert ist, und (C₃-C₉)Cycloalkyl, das unsubstituiert oder substituiert ist, und Heterocyclyl mit 3 bis 6 Ringatomen und 1 bis 3 Heteroringatomen aus der Gruppe N, O und S, wobei der Ring unsubstituiert oder substituiert ist, substituiert ist, oder
einen carbocyclischen oder heterocyclischen Rest mit 3 bis 6 Ringatomen, der unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind,
substituiert ist, wobei Substituenten paarweise auch einen carbocylischen oder heterocyclischen Ring mit 3 bis 6 Ringatomen bilden können, der unsubstituiert oder substituiert ist,
vorzugsweise R¹ einen Rest mit insgesamt 1 bis 20 C-Atomen, insbesondere 1 bis 10 C-Atomen,
- R² und R³: jeweils unabhängig voneinander Wasserstoff, Amino oder Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest oder
R² und R³ gemeinsam mit dem Stickstoffatom der Gruppe NR²R³ einen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder substituiert ist,
- R⁴: Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest,
- R⁵: Wasserstoff, Nitro, Cyano, Thiocyanato oder einen Rest der Formel -B²-Y², wobei B² und Y² wie weiter unten definiert sind,
- A-Z: eine divalente Brücke der Formel

-(CH₂)ₐ- ,

worin a die ganze Zahl 2, 3 oder 4 ist,

-W¹-(CH₂)_{b}-W²-,

worin b die ganze Zahl 1 oder 2 ist und W¹, W² unabhängig voneinander O oder S bedeuten,

-CH₂CH₂CH₂-W³- ,

worin W³ O oder S bedeutet,
- (X')ₘ: m Substituenten X' und dabei X' jeweils unabhängig voneinander Halogen, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, [(C₁-C₄)Alkyl]-carbonyl, [(C₁-C₄)Alkoxy]-carbonyl oder [(C₁-C₄)Alkylthiol-carbonyl, wobei die kohlenwasserstoffhaltigen Teile in den letztgenannten 9 Resten unsubstituiert oder substituiert sind, oder die Oxogruppe,
- (X)ₙ: n Substituenten X und dabei X jeweils unabhängig voneinander Halogen, Nitro, Cyano, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder substituiert sind, oder einen Rest der Formel -B³-Y³, wobei B³ und Y³ wie unten definiert sind,
oder zwei benachbarte Reste X gemeinsam einen ankondensierten Cyclus mit 4 bis 6 Ringatomen, der carbocyclisch ist oder Heteroringatome aus der Gruppe 0, S und N enthält und der unsubstituiert oder durch einen oder mehrere Reste aus der Gruppe Halogen, (C₁-C₄)Alkyl und Oxo substituiert ist,
- B¹,B²,B³: jeweils unabhängig voneinander eine direkte Bindung oder eine divalente Gruppe der Formel -O-, -S(O)ₚ-, -S(O)ₚ-O-, -O-S(O)ₚ-, -CO-, -O-CO-, -CO-O-, -NR'-, -O-NR'-, -NR'-O-, -NR'-CO- oder -CO-NR'-, wobei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist,
- Y¹,Y²: jeweils unabhängig voneinander H oder einen acyclischen Kohlenwasserstoffrest, beispielsweise jeweils mit 1 bis 20 C-Atomen, vorzugsweise mit 1 bis 10 C-Atomen, oder einen cyclischen Kohlenwasserstoffrest mit 3 bis 8 C-Atomen, vorzugsweise 3 bis 6 C-Atomen, oder einen heterocyclischen Rest mit 3 bis 9 Ringatomen und 1 bis 3 Heteroringatomen aus der Gruppe N, O und S, wobei jeder der drei letztgenannten Reste unsubstituiert oder substituiert ist,
- Y³: einen aromatischen, gesättigten oder teilgesättigten carbocyclischen oder heterocyclischen Rest bedeutet, wobei der cyclische Rest substituiert oder unsubstituiert ist,
- m: eine ganze Zahl von Null bis zur Anzahl der Wasserstoffatome im Grundkörper der divalenten Brücke, vorzugweise 0, 1 oder 2, insbesondere 0,
- n: 0, 1, 2, 3 oder 4, vorzugsweise 0, 1 oder 2,
bedeuten.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, daß bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl in Form von (C₃-C₄)Alkenyl bedeutet vorzugsweise einen Alkenylrest mit 3 oder 4 C-Atomen, bei dem die Mehrfachbindung nicht zwischen C-1 und C-2 liegt, wobei C-1 das C-Atom mit der Position von "yl" bezeichnet. Entsprechendes gilt für (C₃-C₄)Alkinyl.

Cycloalkyl bedeutet ein carbocyclisches, gesättigtes Ringsystem mit vorzugsweise 3-8 C-Atomen, z.B. Cyclopropyl, Cyclopentyl oder Cyclohexyl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CH₂CF₃, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthy', Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.

Ein heterocyclischer Rest oder Ring (Heterocyclyl) kann gesättigt, ungesättigt oder heteroaromatisch sein; er enthält vorzugsweise ein oder mehrere Heteroeinheiten im Ring, d.h. Heteroatome oder Ringglieder, welche auch substituierte Heteroatome einschließen, vorzugsweise aus der Gruppe N, O, S, SO, SO₂; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Monound Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch ein oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze von besonderem Interesse, worin
- R¹: (C₁-C₄)Alkyl,
das unsubstituiert oder durch einen oder mehrere Reste aus der Gruppe Halogen, (C₁-C₄)Alkoxy, (C₁-C₄)Alkylthio, (C₁-C₄)Alkylsulfonyl, Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl und (C₁-C₄)Alkylsulfonyl substituiert ist, und (C₃-C₉)Cycloalkyl, das unsubstituiert oder durch einen oder mehrere Reste (C₁-C₄)Alkyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, (C₁-C₄)Alkyl und Oxo substituiert ist,
substituiert ist, oder (C₃-C₇)Cycloalkyl, (C₃-C₇)Cycloalkenyl oder einen heterocyclischen Rest mit 3 bis 6 Ringatomen und 1 bis 3 Heteroringatomen aus der Gruppe N, O und S, wobei der Cycloalkylrest oder der heterocyclische Rest jeweils unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist und/oder Substituenten aufweist, die paarweise einen ankondensierten Benzolring oder einen ankondensierten oder in Spiroform verknüpften Ring aus der Gruppe (C₃-C₇)Cycloalkyl, (C₃-C₇)Cycloalkenyl und einen heterocyclischen Ring mit 3 bis 6 Ringatomen bilden können, der unsubstituiert oder durch einen oder mehrere Reste aus der Gruppe Halogen, (C₁-C₄)Alkyl, (C₁-C₄)Haloalkyl und Oxo substituiert ist, oder Phenyl, das unsubstituiert oder substituiert ist,
vorzugsweise R¹ einen Rest mit insgesamt 1 bis 20 C-Atomen, insbesondere 1 bis 10 C-Atomen,
- R² und R³: jeweils unabhängig voneinander Wasserstoff, Amino oder Alkylamino oder Dialkylamino mit jeweils 1 bis 4 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest oder
R² und R³ gemeinsam mit dem Stickstoffatom der Gruppe NR²R³ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
- R⁴: Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest,
- R⁵: Wasserstoff, Nitro, Cyano, Thiocyanato oder einen Rest der Formel -B²-Y², wobei B² und Y² wie weiter unten definiert sind,
- A-Z: eine divalente Brücke der Formel

-(CH₂)ₐ-,

worin a die ganze Zahl 2, 3 oder 4 ist,

-W¹-(CH₂)_{b}-W²-,

worin b die ganze Zahl 1 oder 2 ist und W¹, W² unabhängig voneinander S oder O bedeuten,

-CH₂CH₂CH₂-W³-,

worin W³ O oder S bedeutet,
- (X')ₘ: m Substituenten X' und dabei X' jeweils unabhängig voneinander Halogen, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder durch einen oder mehrere Reste aus der Gruppe Halogen, (C₁-C₄)Alkoxy und (C₁-C₄)Alkylthio substituiert sind, oder die Oxogruppe,
- (X)ₙ: n Substituenten X und dabei X jeweils unabhängig voneinander Halogen, Nitro, Cyano, (C₁-C₆)Alkyl, (C₁-C₆)Alkoxy, (C₁-C₄)Alkylcarbonyl, (C₁-C₄)Alkyloxycarbonyl oder (C₁-C₄)Alkylthiocarbonyl, wobei die letztgenannten fünf Reste unsubstituiert oder durch Halogen oder (C₁-C₄)Alkoxy substituiert sind, einen Rest der Formel -B³-Y³, wobei B³ und Y³ wie unten definiert sind, oder
zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
- B¹,B²,B³: jeweils unabhängig voneinander eine direkte Bindung oder eine divalente Gruppe der Formel -O-, -S-, -CO-, -O-CO-, -CO-O-, -NR'-, -NR'-CO- oder -CO-NR'-, wobei R' H oder (C₁-C₄)Alkyl ist,
- Y¹,Y²: jeweils unabhängig voneinander H oder einen acyclischen Kohlenwasserstoffrest mit 1 bis 6 C-Atomen, einen cyclischen Kohlenwasserstoffrest mit 3 bis 6 C-Atomen oder einen heterocyclischen Rest mit 3 bis 9 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
- Y³: (C₃-C₆)Cycloalkyl, das unsubstituiert oder durch Reste aus der Gruppe (C₁-C₄)Alkyl und (C₁-C₄)Haloalkyl substituiert ist, oder Phenyl, das unsubstituiert oder durch Reste aus der Gruppe Halogen, (C₁-C₄)Alkyl, (C₁-C₄)Haloalkyl und (C₁-C₄)Alkoxy substituiert ist,
- m: 0, 1 oder 2, vorzugsweise 0 oder 1, insbesondere 0,
- n: 0, 1, 2, 3 oder 4, vorzugsweise 0, 1 oder 2,
bedeuten.

Von besonderem Interesse sind weiterhin erfindungsgemäße Verbindungen der Formel (I) und deren Salze, worin
- R¹: (C₁-C₄)Alkyl,
das unsubstituiert oder durch einen oder mehrere Reste aus der Gruppe Halogen, (C₁-C₄)Alkoxy, (C₁-C₄)Alkylthio und Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Nitro, Cyano, [(C₁-C₂)Alkyl]-carbonyl, Formyl, Carbamoyl, Mono- und Di-[(C₁-C₂)alkyl]-aminocarbonyl, (C₁-C₄)Alkylsulfonyl und (C₃-C₇)Cycloalkyl, das unsubstituiert oder durch einen oder mehrere Reste (C₁-C₄)Alkyl substituiert ist,
substituiert ist, oder
(C₃-C₇)Cycloalkyl oder einen heterocyclischen Rest mit 3 bis 6 Ringatomen und 1 oder 2 Heteroringatomen aus der Gruppe N, O und S, wobei der Cycloalkylrest oder der heterocyclische Rest jeweils unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist und/oder Substituenten aufweist, die paarweise einen in Spiroform verknüpften Ring aus der Gruppe (C₃-C₇)Cycloalkyl bilden können, der unsubstituiert oder durch einen oder mehrere Reste aus der Gruppe Halogen, (C₁-C₄)Alkyl, (C₁-C₄)Haloalkyl und Oxo substituiert ist,
- R² und R³: unabhängig voneinander Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocyclyl-(C₁-C₄)alkyl, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder Phenyl, Phenoxy, Phenylcarbonyl, Phenoxycarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 17 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
R² und R³ gemeinsam mit dem Stickstoffatom der Gruppe NR²R³ einen heterocyclischen Rest mit 3.bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
- R⁴: Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocyclyl-(C₁-C₄)alkyl mit 3 bis 9 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder
Phenyl, Phenoxy, Phenylcarbonyl, Phenoxycarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyctyl, Heterocyclylamino, Heterocyclyloxy, Heterocyclylthio, oder einen der letztgenannten 17 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält,
- R⁵: Wasserstoff, (C₁-C₄)Alkyl, (C₁-C₄)Haloalkyl oder (C₃-C₇)Cycloalkyl,
- A-Z: eine divalente Brücke der Formel

-CH₂CH₂-, -CH₂CH₂CH₂-, -CH₂CH₂CH₂CH₂-,

-S-CH₂S-, -S-CH₂-O-, -O-CH₂-O-,

-S-CH₂CH₂S-, -S-CH₂CH₂-O-, -O-CH₂CH₂-O-,

-CH₂CH₂CH₂-O-, -CH₂CH₂CH₂-S-,
- (X')ₘ: m Substituenten X' und dabei X' jeweils unabhängig voneinander Halogen, (C₁-C₄)Alkyl, (C₂-C₄)Alkenyl, (C₂-C₄)Alkinyl, (C₁-C₄)Alkoxy, (C₂-C₄)Alkenyloxy, (C₂-C₄)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder durch einen oder mehrere Reste aus der Gruppe Halogen substituiert sind, oder die Oxogruppe,
- (X)ₙ: n Substituenten X und dabei X jeweils unabhängig voneinander Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder Phenyl, Phenoxy, Phenylcarbonyl, Phenoxycarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 17 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
- B¹: eine direkte Bindung oder eine divalente Gruppe der Formel -O-, -S-, -CO-, -O-CO-, -CO-O-, -NR'-, -NR'-CO- oder -CO-NR'-, wobei R' H oder (C₁-C₄)Alkyl ist,
- Y¹: H oder einen acyclischen Kohlenwasserstoffrest mit 1 bis 4 C-Atomen, einen cyclischen Kohlenwasserstoffrest mit 3 bis 6 C-Atomen oder einen heterocyclischen Rest mit 3 bis 6 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, Hydroxy, Amino, Mono- und Dialkylamino, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
bedeuten.

Von besonderem Interesse sind weiterhin erfindungsgemäße Verbindungen der Formel (I) und deren Salze, worin
- R¹: (C₁-C₄)Alkyl, (C₁-C₄)Haloalkyl, Benzyl, [(C₃-C₆)Cycloalkyl]-(C₁-C₂)-alkyl, (C₃-C₆)Cycloalkyl oder einen heterocyclischen Rest mit 3 bis 6 Ringatomen und 1 oder 2 Heteroringatomen aus der Gruppe N und O, vorzugsweise ein Sauerstoffatom als Heteroringatom, wobei der Cycloalkylrest oder der heterocyclische Rest jeweils unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist und/oder Substituenten aufweist, die paarweise einen in Spiroform verknüpften Ring aus der Gruppe (C₃-C₇)Cycloalkyl bilden können, der unsubstituiert oder durch einen oder mehrere Reste aus der Gruppe Halogen, (C₁-C₄)Alkyl, (C₁-C₄)Haloalkyl und Oxo substituiert ist,
- R² und R³: unabhängig voneinander Wasserstoff, Amino, Formyl, (C₁-C₄)Alkyl, (C₁-C₄)Alkoxy-(C₁-C₄)alkyl, (C₃-C₆)Alkenyl, (C₃-C₆)Alkinyl, (C₁-C₄)Alkylamino-(C₁-C₄)alkyl, Di-[(C₁-C₄)alkyl]-amino-(C₁-C₄)alkyl oder Phenyl, Phenyl-(C₁-C₄)alkyl, Phenoxycarbonyl oder Phenoxycarbonyl oder einen der letztgenannten vier Reste, der im Phenylteil bis zu dreifach durch Reste aus der Gruppe Halogen, (C₁-C₄)Alkyl, (C₁-C₄)Alkoxy und (C₁-C₄)Alkoxy-carbonyl substituiert ist, oder
R² und R³ gemeinsam mit dem Stickstoffatom der Gruppe NR²R³ einen heterocyclischen Rest mit 3 bis 6 Ringatomen und 1 bis 2 Heteroringatomen, wobei neben dem N-Atom das gegebenenfalls weitere Heteroringatom aus der Gruppe N und O ausgewählt ist und der Rest unsubstituiert oder durch einen oder mehrere Reste aus der Gruppe Halogen, (C₁-C₄)Alkyl und Oxo substituiert ist,
- R⁴: Wasserstoff, Amino, Formyl, (C₁-C₄)Alkyl, Di-[(C₁-C₄)alkyl]-amino, (C₁-C₄)Alkoxy-(C₁-C₄)alkyl, (C₃-C₆)Alkenyl, (C₃-C₆)Alkinyl, (C₁-C₄)Dialkylamino-(C₁-C₄)alkyl, Phenyl, Phenoxy-(C₁-C₄)alkyl, Phenyl-(C₁-C₄)alkyl, Phenoxy-carbonyl, Phenylamino-carbonyl oder einen der letztgenannten fünf Reste, der im Phenylteil einfach bis dreifach durch Reste aus der Gruppe Halogen, (C₁-C₄)Alkyl, (C₁-C₄)Alkoxy und (C₁-C₄)Alkoxy-carbonyl substituiert ist,
- R⁵: Wasserstoff, (C₁-C₄)Alkyl, (C₁-C₄)Haloalkyl oder (C₃-C₇)Cycloalkyl,
- A-Z: eine divalente Brücke der Formel

-CH₂CH₂-, -CH₂CH₂CH₂-, -CH₂CH₂CH₂CH₂-,

-S-CH₂CH₂S-, -S-CH₂CH₂-O-, -O-CH₂CH₂-O-,

-CH₂CH₂CH₂-O-, -CH₂CH₂CH₂-S-,
- (X')ₘ: m Substituenten X' und dabei X' jeweils unabhängig voneinander Halogen, (C₁-C₄)Alkyl oder (C₁-C₄)Alkoxy,
- (X)ₙ: n Substituenten X und dabei X jeweils unabhängig voneinander Halogen, Hydroxy, (C₁-C₄)Alkyl oder (C₁-C₄)Alkoxy
- B¹: eine direkte Bindung oder eine divalente Gruppe der Formel -O-, -S- oder -O-CO-,
- Y¹: H, (C₁-C₄)Alkyl , (C₃-C₆)Cycloalkyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, Mono- und Dialkylamino substituiert ist,
bedeuten.

Bevorzugte erfindungsgemäße Verbindungen der Formel (I) und deren Salze sind solche, worin
- R¹: (C₁-C₄)Alkyl, (C₁-C₄)Haloalkyl oder [(C₃-C₆)-cycloalkyl]-methyl, vorzugsweise -CH₃, -CH₂F, -CHF₂, -CF₃, -CH₂Cl, -CHCl₂, -CCl₃, -CH₂Br, -CHBr₂, -CH₂CH₃, -CH₂CH₂F, -CF₂CHF₂, -CH₂CH₂Cl, -CH₂CH₂Br, -CH(CH₃)₂, -CF(CH₃)₂, -C(CH₃)₂Cl oder Cyclopropylmethyl,
- R² und R³: unabhängig voneinander Wasserstoff, Formyl oder (C₁-C₄)Alkyl oder
R² und R³ gemeinsam mit dem Stickstoffatom der Gruppe NR²R³ einen heterocyclischen Rest mit 4 bis 6 Ringatomen und 1 bis 2 Heteroringatomen, wobei neben dem N-Atom das gegebenenfalls weitere Heteroringatom aus der Gruppe N und O ausgewählt ist,
- R⁴: Wasserstoff oder (C₁-C₄)Alkyl,
- R⁵: H, (C₁-C₄)Alkyl oder (C₁-C₄)Haloalkyl, vorzugsweise H, CH₃, C₂H₅, n- oder i-C₃H₇, n-Butyl oder CF₃, insbesondere CH₃ oder C₂H₅,
- A-Z: eine divalente Brücke der Formel

-CH₂CH₂-, -CH₂CH₂CH₂-, -CH₂CH₂CH₂CH₂-,

-S-CH₂CH₂S, -O-CH₂CH₂-O-,

-CH₂CH₂CH₂-O-, -CH₂CH₂CH₂-S-,
- (X')ₘ: m Substituenten X' und dabei X' jeweils unabhängig voneinander Halogen oder (C₁-C₄)Alkyl,
- (X)ₙ: n Substituenten X und dabei X jeweils unabhängig voneinander Halogen, Hydroxy, (C₁-C₄)Alkyl oder (C₁-C₄)Alkoxy
bedeuten.

Bevorzugt sind auch erfindungsgemäße Verbindungen der Formel (I) und deren Salze, die ein oder mehrere der Merkmale aus den obengenannten bevorzugten Verbindungen enthalten.

Gegenstand der vorliegenden Erfindung sind auch Verfahren zur Herstellung der Verbindungen der allgemeinen Formel (I) oder deren Salze, dadurch gekennzeichnet, daß man
a) eine Verbindung der Formel (II),

   R¹ - Fu (II)

   worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet,
   mit einem Biguanidid der Formel (III) oder einem Säureadditionssalz hiervon 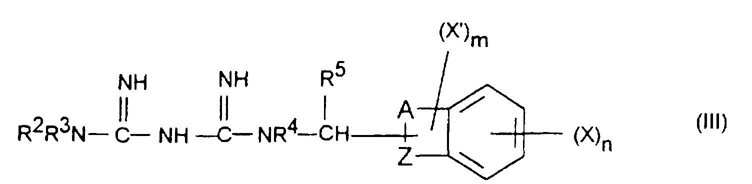 umsetzt oder
b) eine Verbindung der Formel (IV), 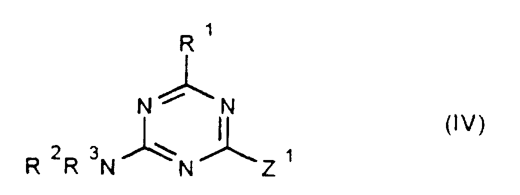 worin Z¹ einen austauschfähigen Rest oder eine Abgangsgruppe, z.B. Chlor, Trichlormethyl, (C₁-C₄)Alkylsulfonyl und unsubstituiertes oder substituiertes Phenyl-(C₁-C₄)alkylsulfonyl oder (C₁-C₄)Alkylphenylsulfonyl, bedeutet, mit einem geeigneten Amin der Formel (V) oder einem Säureadditionssalz hiervon 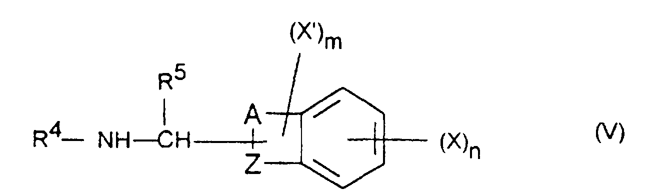 umsetzt,
wobei in den Formeln (II), (III), (IV) und (V) die Reste R¹, R², R³, R⁴, R⁵, A, X', X, m und n wie in Formel (I) definiert sind.

Die Umsetzung der Verbindungen der Formel (II) und (III) erfolgt vorzugsweise basenkatalysiert in einem inerten organischen Lösungsmittel, wie z.B. Tetrahydrofuran (THF), Dioxan, Acetonitril, Dimethylformamid (DMF), Methanol und Ethanol, bei Temperaturen zwischen -10 °C und dem Siedepunkt des Lösungsmittel, vorzugsweise bei 20 °C bis 60 °C; falls Säureadditionssalze der Formel (III) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei beispielsweise im Bereich von 0,1 bis 3 Moläquivalenten bezogen auf die Verbindung der Formel (III) eingesetzt. Die Verbindung der Formel (II) kann im Verhältnis zur Verbindung der Formel (III) beispielsweise äquimolar oder mit bis zu 2 Moläquvälenten Überschuß eingesetzt werden. Grundsätzlich sind die entsprechenden Verfahren in der Literatur bekannt (vergleiche: Comprehensive Heterocyclic Chemistry, A.R. Katritzky, C.W. Rees, Pergamon Press, Oxford, New York, 1984, Vol.3; Part 2B; ISBN 0-08-030703-5, S.290).

Die Umsetzung der Verbindungen der Formel (IV) und (V) erfolgt vorzugsweise basenkatalysiert in einem inerten organischen Lösungsmittel, wie z.B. THF, Dioxan, Acetonitril, DMF, Methanol und Ethanol, bei Temperaturen zwischen -10°C und dem Siedepunkt des jeweiligen Lösungsmitteis oder Lösungsmittelgemisches, vorzugsweise bei 20°C bis 60°C, wobei die Verbindung (V), falls als Säureadditionssalz eingesetzt, gegebenenfalls in situ mit einer Base freigesetzt wird. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei in der Regel im Bereich von 1 bis 3 Moläquivalenten bezogen auf die Verbindung der Formel (IV) eingesetzt, die Verbindung der Formel (IV) kann beispielsweise äquimolar zur Verbindung der Formel (V) oder mit bis zu 2 Moläquvalenten Überschuß eingesetzt werden. Grundsätzlich sind die entsprechenden Verfahren aus der Literatur bekannt (vgl. Comprehensive Heterocyclic Chemistry, A.R. Katritzky, C.W. Rees, Pergamon Press, Oxford, New York, 1984, Vol.3; Part 2B; ISBN 0-08-030703-5, S. 482).

Die Edukte der Formeln (II), (III), (IV) und (V) sind entweder kommerziell erhältlich oder können nach oder analog literaturbekannten Verfahren hergestellt werden. Die Verbindungen können beispielsweise auch nach einem der nachfolgend beschriebenen Verfahren hergestellt werden.

Die Verbindung der Formel (IV), oder eine direkte Vorstufe davon, läßt sich beispielweise wie folgt herstellen:
1. Durch Reaktion einer Verbindung der Formel (II) mit einem Amidino-thioharnstoff-Derivat der Formel (VI), 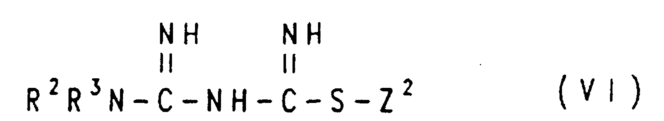 worin Z² (C₁-C₄)-Alkyl oder Phenyl-(C₁-C₄)-alkyl bedeutet und R² und R³ wie in Formel (I) definiert sind, werden Verbindungen der Formel (IV) erhalten, in denen Z¹ = -SZ² bedeutet.
2. Durch Umsetzung eines Amidins der Formel (VII) oder eines Säureadditionssalzes davon, 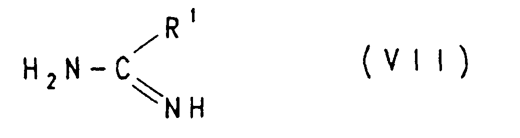 worin R¹ wie in Formel (I) definiert ist,
   mit einem N-Cyanodithioiminocarbonat der Formel (VIII), 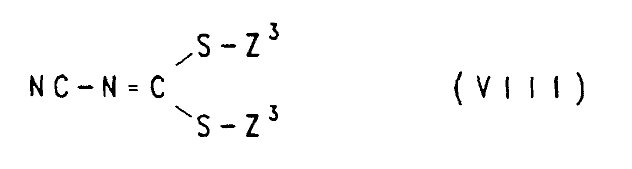 worin Z³ (C₁-C₄)-Alkyl oder Phenyl-(C₁-C₄)-alkyl bedeutet, werden Verbindungen der Formel (IV) erhalten, worin Z¹ = -S-Z³ bedeutet.
3. Durch Umsetzung eines Alkali-dicyanamids mit einem Carbonsäurederivat der genannten Formel (II) werden Verbindungen der Formel (IV) erhalten, worin Z¹ = NH₂ bedeutet,
4. Durch Umsetzung von Trichloracetonitril mit einem Nitril der Formel (IX),

   R¹ - CN (IX)

   worin R¹ wie in Formel (I) definiert ist, werden zunächst Verbindungen der Formel (X), 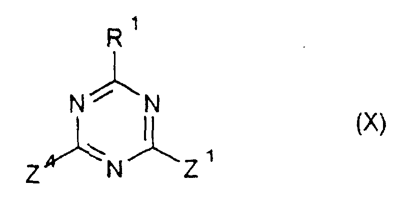 worin Z¹ und Z⁴ jeweils CCl₃ bedeuten, erhalten, welche durch nachfolgende Umsetzung mit Verbindungen der Formel HNR²R³ (R² und R³ wie in Formel (I)), zu Verbindungen der Formel (IV), worin Z¹ = CCl₃ bedeutet, führen.

Die Umsetzung der Carbonsäurederivate der Formel (II) mit den Amidinothioharnstoff-Derivaten der Formel (VI) erfolgt vorzugsweise basenkatalysiert in einem organischen Lösungsmittel, wie z.B. Aceton, THF, Dioxan, Acetonitril, DMF, Methanol, Ethanol, bei Temperaturen von -10°C bis zum Siedepunkt des Lösungsmittel, vorzugsweise bei 0°C bis 20°C. Die Umsetzung kann aber auch in Wasser oder in wässrigen Lösungsmitttelgemischen mit einem oder mehreren der obengenannten organischen Lösungsmitteln erfolgen. Falls (VI) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei z.B. im Bereich von 1 bis 3 Moläquivalenten bezogen auf die Verbindung der Formel (VI) eingesetzt. Verbindungen der Formel (II) und (VI) können beispielsweise äquimolar oder mit bis zu 2 Moläquivalenten Überschuß an Verbindung der Formel (II) eingesetzt werden. Grundsätzlich sind die entsprechenden Verfahren literaturbekannt (vergl.: H. Eilingsfeld, H. Scheuermann, Chem. Ber.; 1967, 100, 1874).

Die Umsetzung der Amidine der Formel (VII) mit den N-Cyanodithioiminocarbonaten der Formel (VIII) erfolgt vorzugsweise basenkatalysiert in einem inerten organischen Lösungsmittel, wie z.B. Acetonitril, DMF, Dimethylacetamid (DMA), N-Methylpyrrolidon (NMP), Methanol und Ethanol, bei Temperaturen von -10°C bis zum Siedepunkt des Lösungsmittels, vorzugsweise bei 20°C bis 80°C. Falls (VII) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei z. B. in Bereich von 1 bis 3 Moläquivalenten bezogen auf die Verbindung der Formel (VIII) eingesetzt, Verbindungen der Formel (VII) und (VIII) können in der Regel äquimolar oder mit 2 Moläquivalenten Überschuß an Verbindung der Formel (II) eingesetzt werden. Grundsätzlich sind die entsprechenden Verfahren literaturbekannt (vergl.: T.A. Riley, W.J. Henney, N.K. Dalley, B.E. Wilson, R.K. Robins; J. Heterocyclic Chem.; 1986, 23 (6), 1706-1714).

Die Herstellung von Zwischenprodukten der Formel (X) mit Z¹ = Chlor kann durch Reaktion von Alkali-dicyanamid mit einem Carbonsäurederivat der Formel (II), wobei dann Fu bevorzugt die funktionelle Gruppe Carbonsäurechlorid oder Carbonsäureamid bedeutet, erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -10°C und dem Siedepunkt des Lösungsmittels, vorzugsweise bei 20°C bis 80°C, wobei die entstehenden Intermediate in situ mit einem geeigneten Chlorierungsreagenz wie beispielsweise Phosphoroxychlorid chloriert werden können. Geeignete Säuren sind z.B. Halogenwasserstoffsäuren, wie HCI, oder auch Lewis-Säuren, wie z.B. AlCl₃ oder BF₃ (vergl. US-A-5095113, DuPont).

Die Herstellung von Zwischenprodukten der Formel (X) mit Z¹, Z⁴ = Trihalogenmethyl kann durch Reaktion der entsprechenden Trihalogenessigsäurenitrile mit einem Carbonsäurenitril der Formel (IX) erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -40 °C und dem Siedepunkt des Lösungsmittels, vorzugsweise bei -10 °C bis 30 °C. Geeignete Säuren sind z.B. Halogenwasserstoffsäuren wie HCI oder auch Lewis-Säuren wie z.B. AlCl₃ oder BF₃ (vgl. EP-A-130939, Ciba Geigy).

Zwischenprodukte der Formel (IV), worin Z¹ = (C₁-C₄)Alkylmercapto oder unsubstituiertes Phenyl-(C₁-C₄)-alkylmercapto ist, können in einem inerten organischen Lösungsmittel wie z .B. Toluol, Chlorbenzol, chlorierten Kohlenwasserstoffen oder anderen bei Temperaturen zwischen -40 °C und dem Siedepunkt des Lösungsmittel, vorzugsweise bei 20 °C bis 80 °C, mit einem geeigneten Chlorierungsreagenz wie z.B. elementarem Chlor oder Phosporoxychlorid zu reaktionsfähigeren Chlortriazinen der Formel (IV), worin Z¹ = CI ist, überführt werden (vgl. J.K. Chakrabarti, D.E. Tupper; Tetrahedron 1975, 31(16), 1879-1882).

Zwischenprodukte der Formel (IV), wobei Z¹ = (C₁-C₄)Alkylmercapto oder unsubstituiertes oder substituiertes Phenyl-(C₁-C₄)-alkylmercapto oder (C₁-C₄)Alkyl-phenylthio ist, können in einem geeigneten Lösungsmittel wie z.B. chlorierten Kohlenwasserstoffen, Essigsäure, Wasser, Alkoholen, Aceton oder Mischungen hiervon bei Temperaturen zwischen 0°C und dem Siedepunkt des Lösungsmittels, vorzugsweise von 20°C bis 80°C, mit einem geeigneten Oxidationssreagenz wie z.B. m-Chlorperbenzoesäure, Wasserstoffperoxid, Kaliumperoxomonosulfat oxidiert werden (vergl.: T.A. Riley, W.J. Henney, N.K. Dalley, B.E. Wilson, R.K. Robins; J. Heterocyclic Chem.; 1986, 23 (6), 1706-1714).

Die Biguanidide der Formel (III) können hergestellt werden, indem man Cyanoguanidine der Formel (XI) 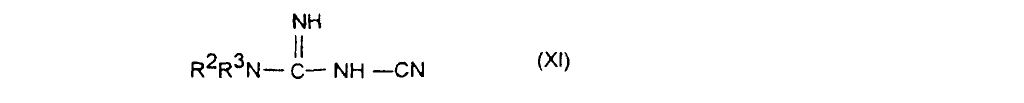 worin R² und R³ wie in Formel (I) definiert sind, mit Aminen der genannten Formel (V), vorzugsweise in Form der Hydrohalogenide oder anderer Säureadditionssalze, umsetzt;
die Amine werden auch in dem alternativen Verfahren b) zur Herstellung von Verbindungen (I) eingesetzt.

Die Umsetzung der Verbindungen (III) und (V) erfolgt in der Regel, indem man die Verbindung (III) mit dem Hydrochlorid des Amins der Formel (V) in Gegenwart eines inerten organischen, vorzugsweise höher siedenden Lösungsmittels bei Temperaturen von 50°C bis zur Siedetemperatur des Lösungsmittels, insbesondere 60 bis 150°C reagieren läßt. Als Lösungsmittel kommen die bereits erwähnten organischen Lösungsmittel in Frage, beispielsweise THF oder Dichlorbenzol.

Zur Herstellung von Zwischenprodukten der Formel (V) 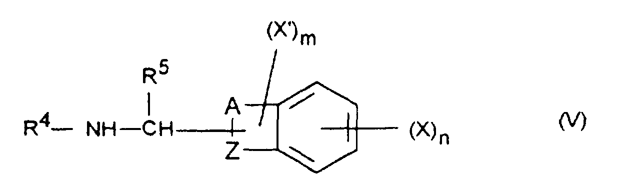 eignen sich Verfahren, wobei man Verbindungen der Formel (XII) 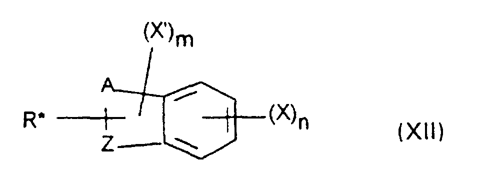 in denen A, X, X', Z, m und n wie in Formel (V) definiert sind und
1. in denen R* eine Gruppe der Formel 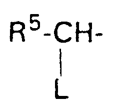 bedeutet,
   worin L eine Abgangsgruppe ist, beispielsweise Halogen wie Chlor, Brom oder lod oder die Mesylgruppe ist, mit Ammoniak oder einem Amin R⁴-NH₂ umsetzt oder
2. in denen R* eine Gruppe der Formel R⁵-CO- bedeutet, mit Ammoniak in Gegenwart einer Wasserstoffquelle, z.B. eines Reduktionsmittels wie Raney Nickel,
   reduktiv aminiert und im Falle R⁴ ungleich Wasserstoff entsprechend derivatisiert,
3. in denen R* eine Gruppe der Formel R⁵-C(=NOH)- bedeutet, mit Wasserstoff in Gegenwart eines Katalysators reduziert und die erhaltene Verbindung (V) im Falle R⁴ ungleich Wasserstoff entsprechend derivatisiert,
wobei in den Formeln der Varianten 1 bis 3 R⁵ jeweils wie in Formel (V) definiert ist.

Zur Herstellung von Zwischenprodukten der Formel (V) und deren Säureadditionssalzen eignet sich beispielsweise ein Verfahren, wobei Ketone der Formel (XIII), 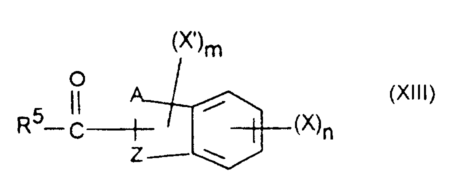 in denen
R⁵, A, X, X', Z, m und n wie in Formel (V) definiert sind, entweder direkt nach der genannten Variante 2 reduktiv aminiert werden oder zunächst mit Hydroxylaminhydrochlorid unter Standardbedingungen, z.B. in wäßriger alkoholischer Lösung in Gegenwart einer Base, zum entsprechenden Oxim umgesetzt werden, das dann nach der genannten Variante 3 zur Verbindung der Formel V, R⁴ = H reduziert wird. Erforderlichenfalls wird zu anderen Verbindungen R⁴ = H derivatisiert.

Die Reaktionen der Varianten 1 bis 3 sind im Prinzip bekannt und in EP-0492615 am Beispiel der Herstellung von Phenoxyalkylaminen in analoger Weise beschrieben. Die dort gewählten Reaktionsbedingungen können in der Regel auch bei der Herstellung von Verbindungen (V) aus Verbindungen der Formel (XIII) angewendet werden. Die Ketone der Formel (XIII) sind literaturbekannt (vgl. JACS 97, 347 (1975); Bull. Soc. Chim. Fr. 1973 (Pt.2), 1285; Gazz. Chim. Ital. 107, 271 (1977); Heterocycles 26, 645 (1987)) oder können analog den literaturbekannten Verfahren hergestellt werden.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzin und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100°C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkaliund Erdalkalihydride, z.B. NaH, Alkali- und Erdalalkoholate, z.B. Natriummethanolat, Kalium-tert.Butylat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen monound dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.
Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff; versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron-ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 31.0, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulcotrione, sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thizopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt,

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiel A1

### 2-Amino-4-(1-fluor-1-methyl-ethyl)-6-[1-(indan-2-yl)-1-ethylamino]-1,3,5-triazin (siehe Tabelle 2, Beispiel 2.17)

Zu 7,0 g (0,025 mol) 1-Biguanidino-1-(indan-2-yl)ethanhydrochlorid in 50 ml Methanol und 7 g gemahlenem Molekularsieb 3Å wird eine aus 1,2 g (0,05 mol) Natrium und 100 ml Methanol hergestellte Methanolatlösung gegeben. Danach fügt man 6,0 g (0,045 mol) 1-Fluor-isobuttersäureethylester hinzu und rührt 2 Stunden bei 25°C und dann 4 Stunden bei 65°C. Die Reaktionsmischung wird filtriert, das Filtrat eingeengt und der Rückstand in Ethylacetat aufgenommen. Es wird mit Wasser gewaschen, die organische Phase abgetrennt und mit Natriumsulfat getrocknet. Das Trockenmittel wird abfiltriert und das Lösungsmittel im Vakuum eingedampft. Nach Reinigung mit Säulenchromatographie (Laufmittel: Essigsäureethylester) erhält man 4,8 g (61 % d.Th.) 2-Amino-4-(1-fluor-1-methyl-ethyl)-6-[1-(indan-2-yl)-1-ethylamino]-1,3,5-triazin.

### Beispiel A2

### 2-Amino-4-isopropyl-6-[1-(1-methylbenzimidazol-2-yl)-1-ethylamino]-1,3,5-triazin (siehe Tabelle 16, Beispiel 16.3)

2,6 g (0,015 mol) 2-Amino-4-chlor-6-isopropyl-1,3,5-triazin und 4,2 g (0,03 mol) K₂CO₃ werden in 50 ml Acetonitril vorgelegt. Zu dieser Lösung fügt man 3,8 g (0,015 mol) 1-Methylbenzimidazol-hydrobromid hinzu und erhitzt anschließend 3 Stunden am Rückfluß. Danach werden alle festen Bestandteile abgesaugt und das Filtrat einrotiert. Der Rückstand wird mittels Säulenchromatographie (Laufmittel: Essigsäureethylester) gereinigt. Man erhält 4,1 g (88 % d.Th.) 2-Amino-4-isopropyl-6-[1-(1-methyl-benzimidazol-2-yl)-1-ethylamino]-1,3,5-triazin.

Die in den Tabellen 1 bis 17 beschriebenen Verbindungen erhält man gemäß oder analog zu den vorstehenden Beispielen A1 und A2. In der Tabelle bedeuten:

| | |
|---|---|
| Nr. = | Beispiel oder Beispielnummer |
| Phys. Daten = | Charakteristische physikalische Daten der Verbindung |
| Me = | Methyl |
| OMe = | Methoxy |
| Et = | Ethyl |
| Pr = | Propyl |
| i-Pr = | Isopropyl |
| c-Pr = | Cyclopropyl |
| 1-Me-c-Pr = | 1-Methyl-cyclopropyl |
| c-C₅H₉ = | Cyclopentyl |
| c-C₄H₇ = | Cyclobutyl |
| c-C₆H₁₁ = | Cyclohexyl |
| t-Bu = | tertiär-Butyl |
| Ph = | Phenyl |
| (X)ₙ = | Position und Art des Substituenten am Phenylring (Position 1 = Bindung zu A); "-" = kein Substituent am Phenylring (n = 0) |

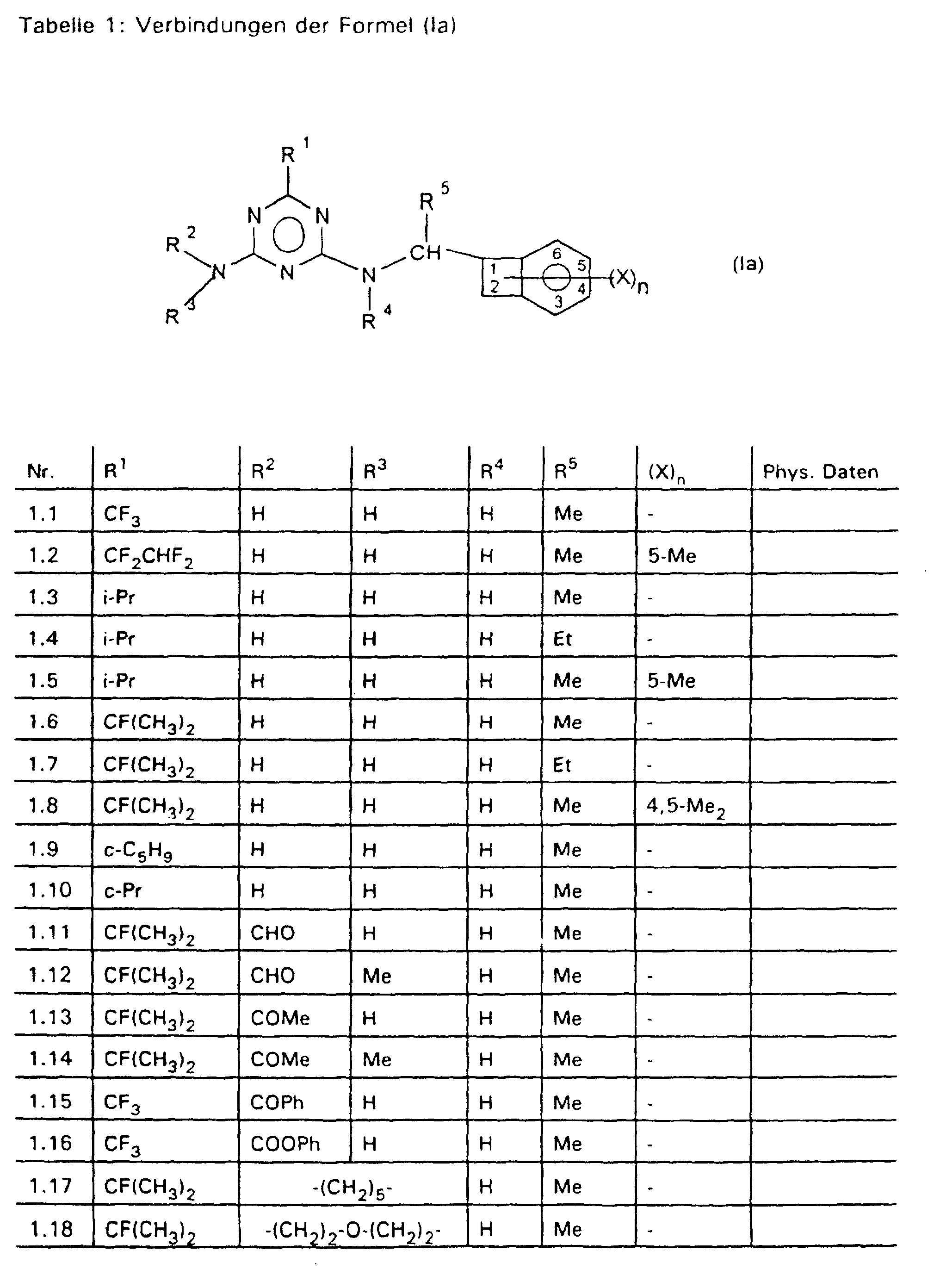

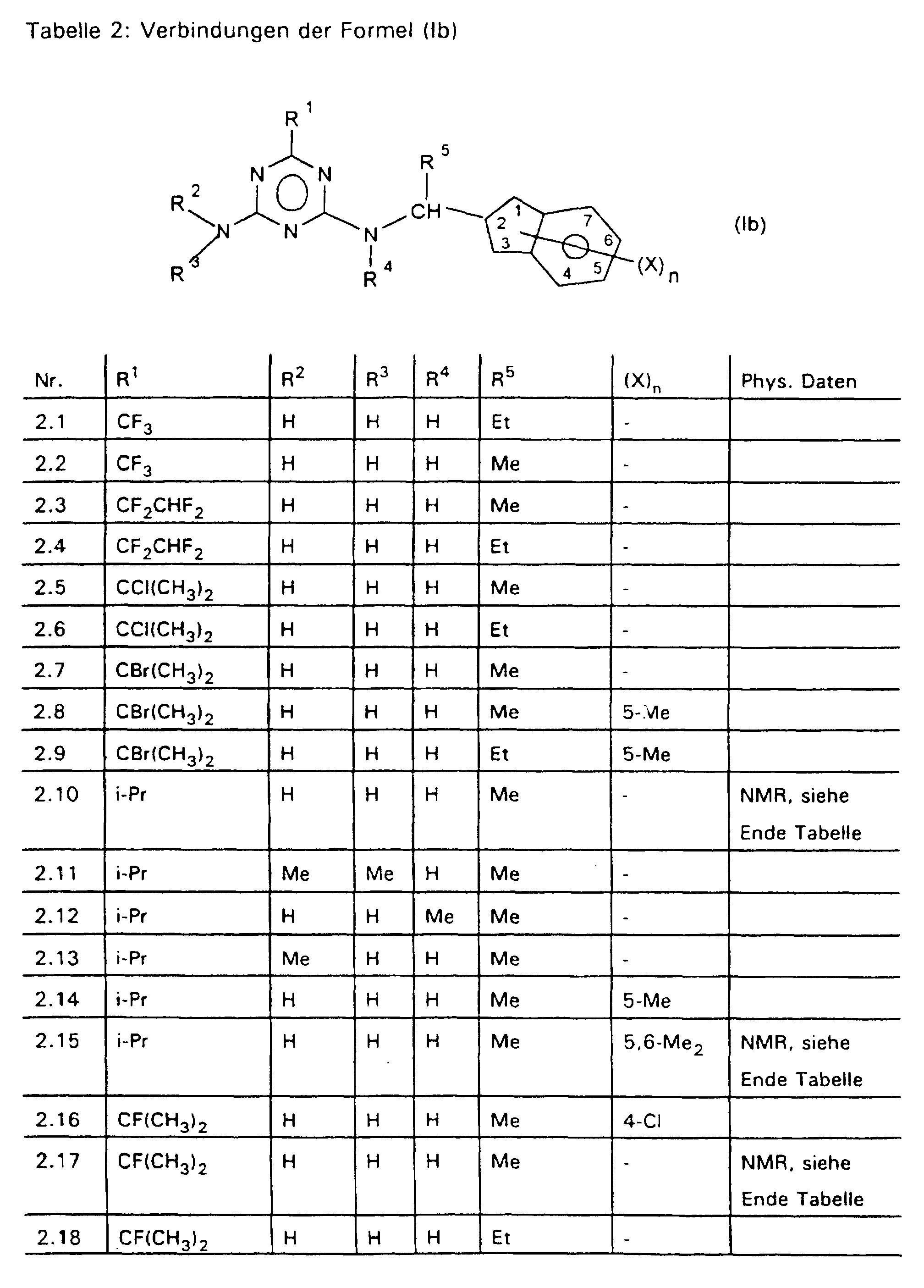

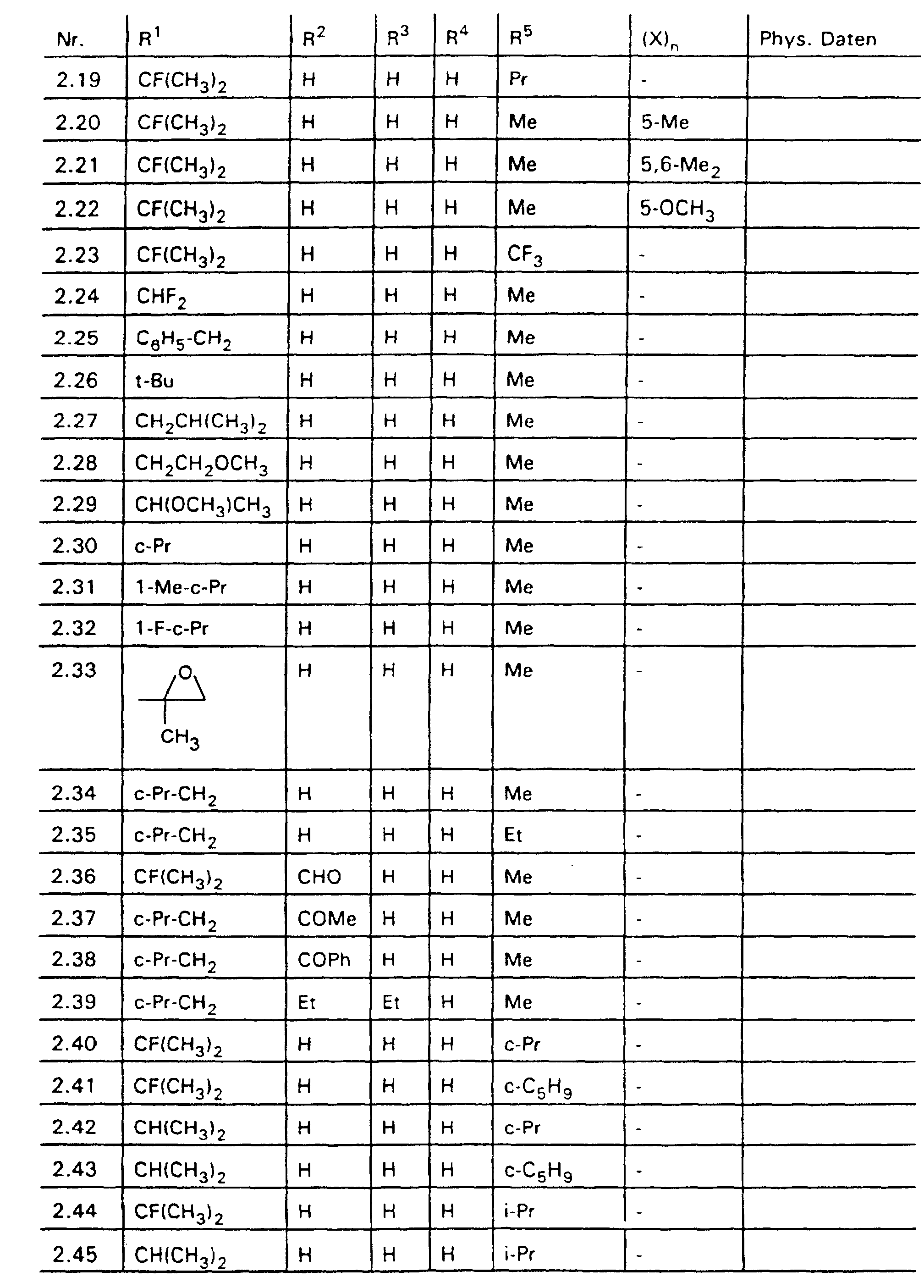

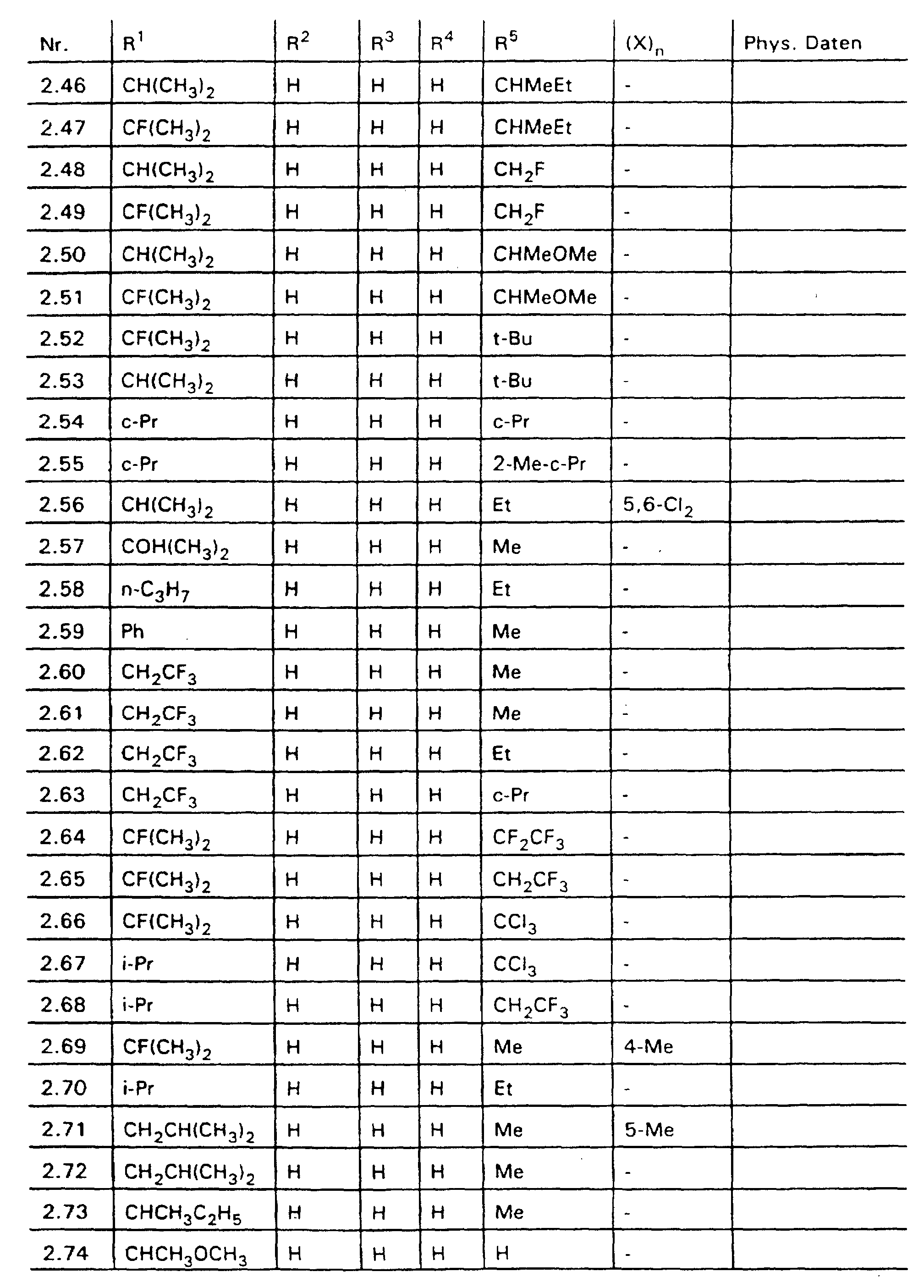

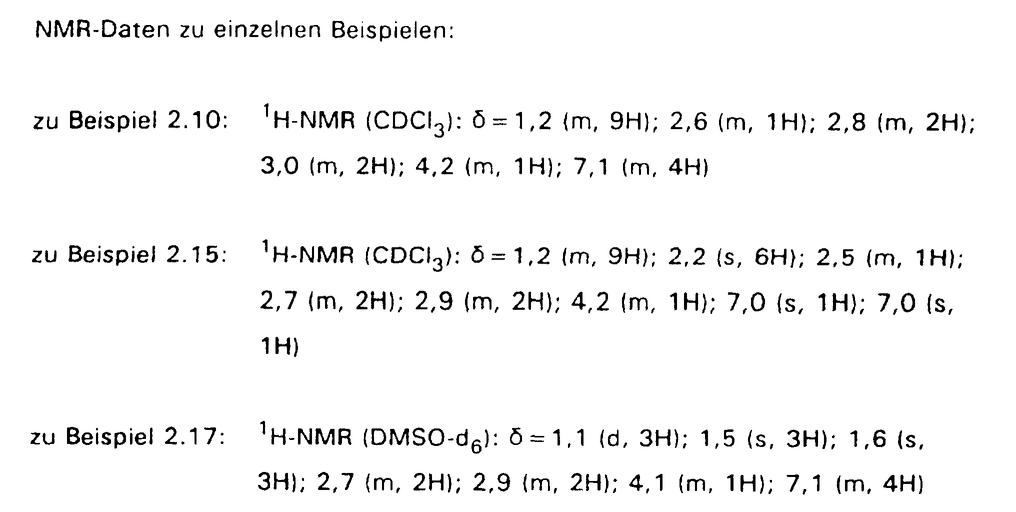

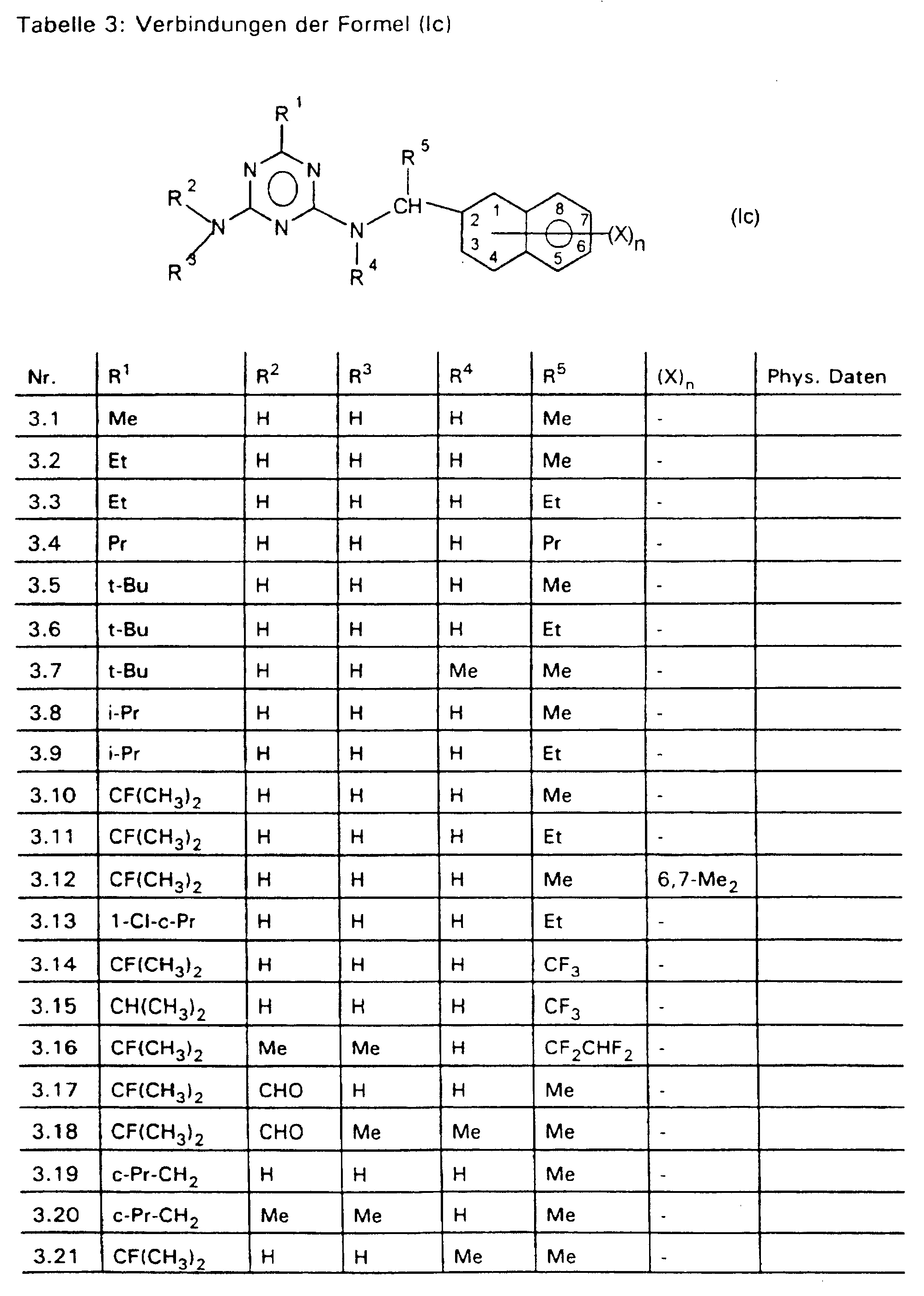

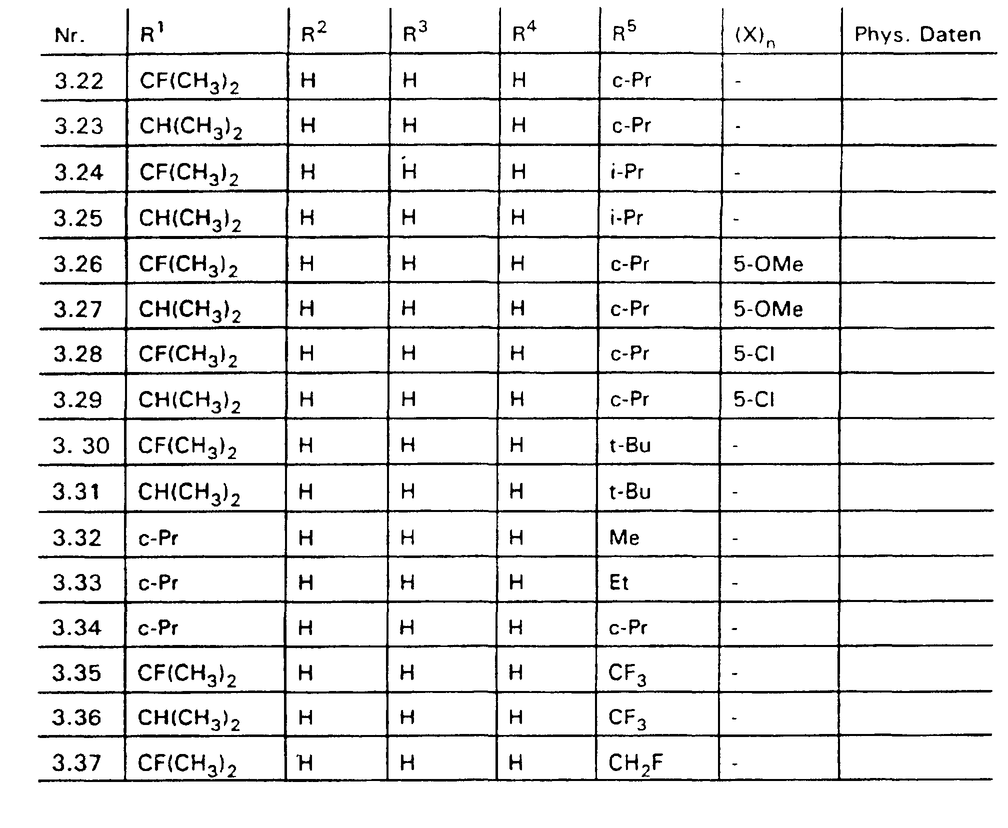

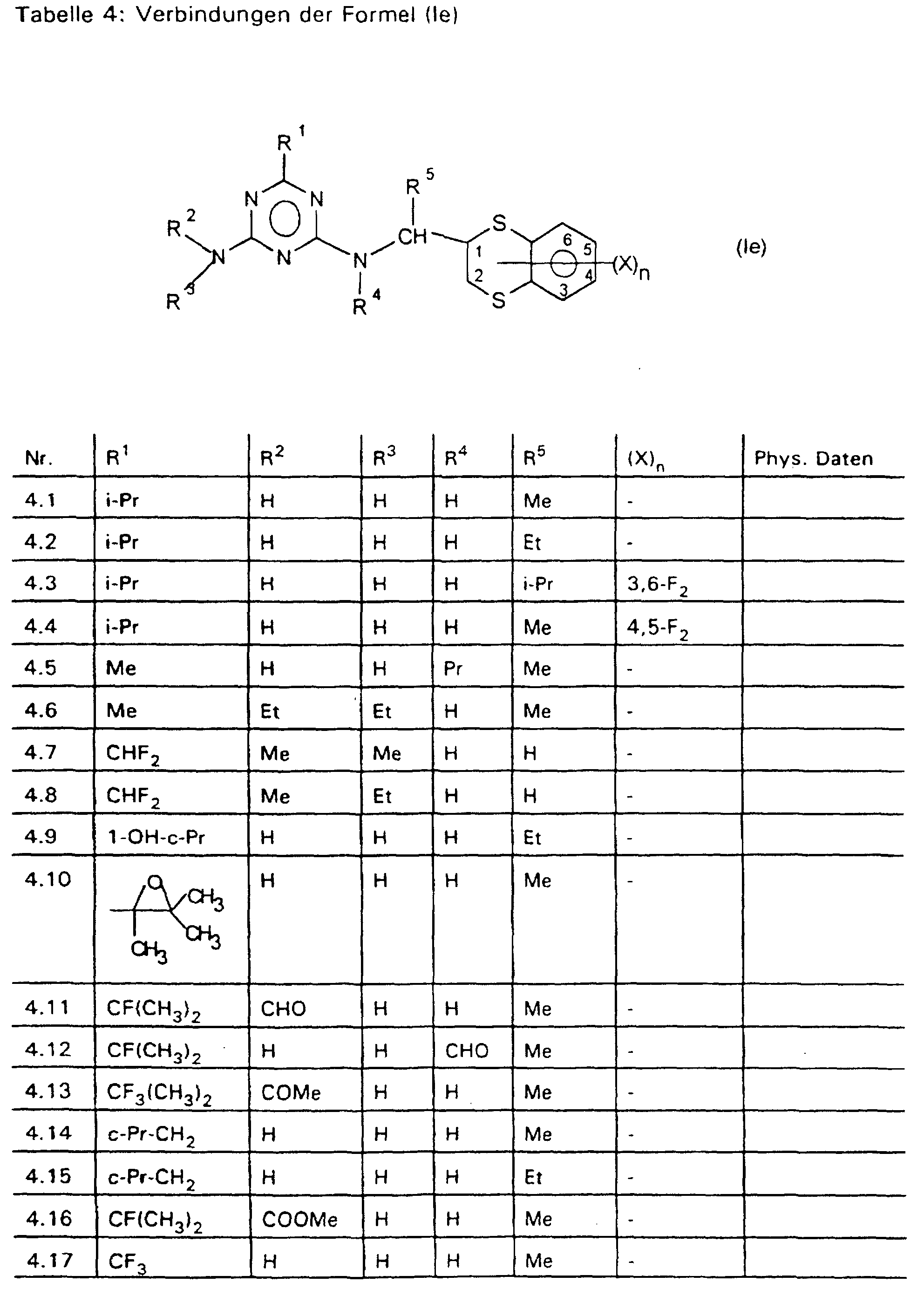

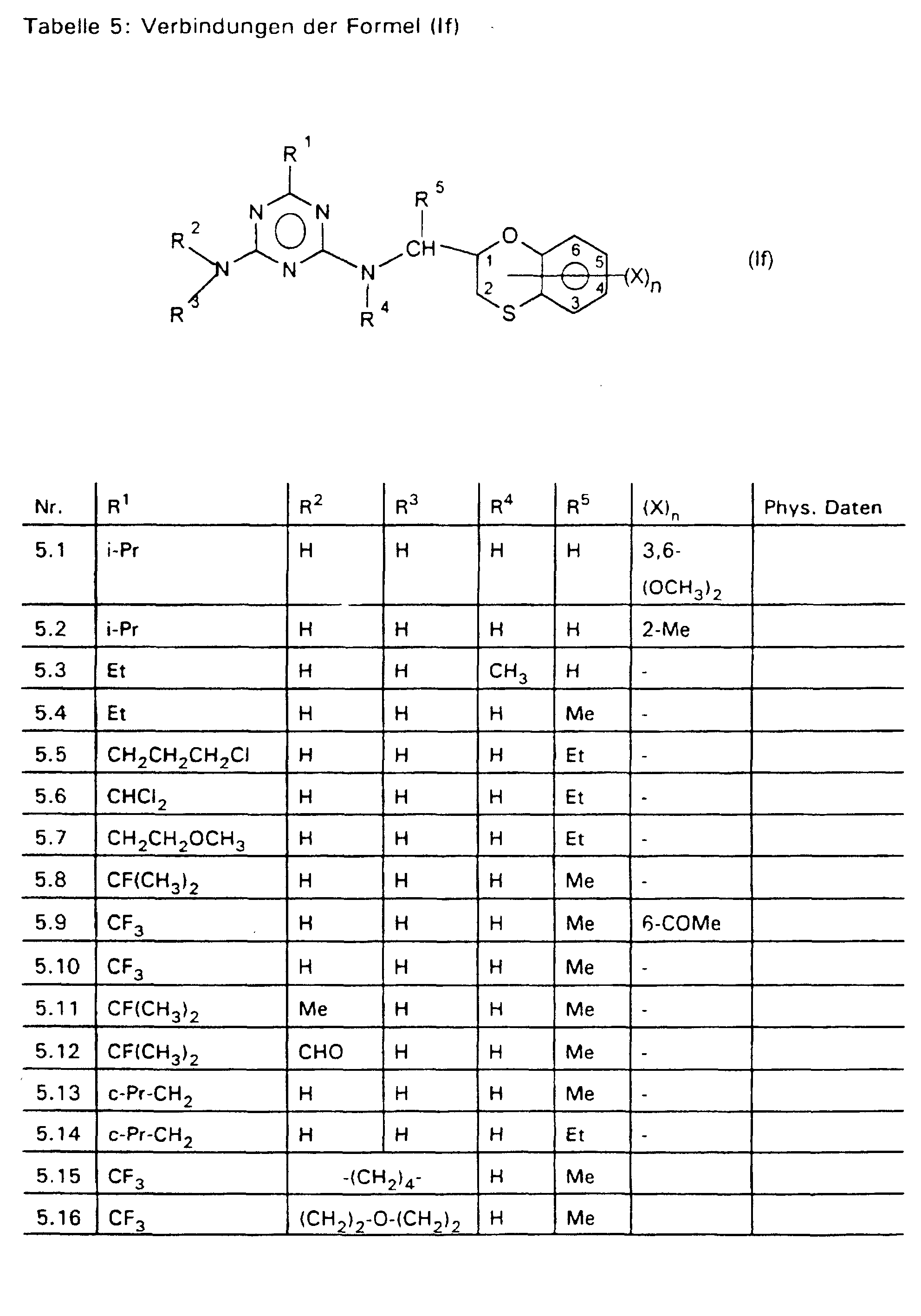

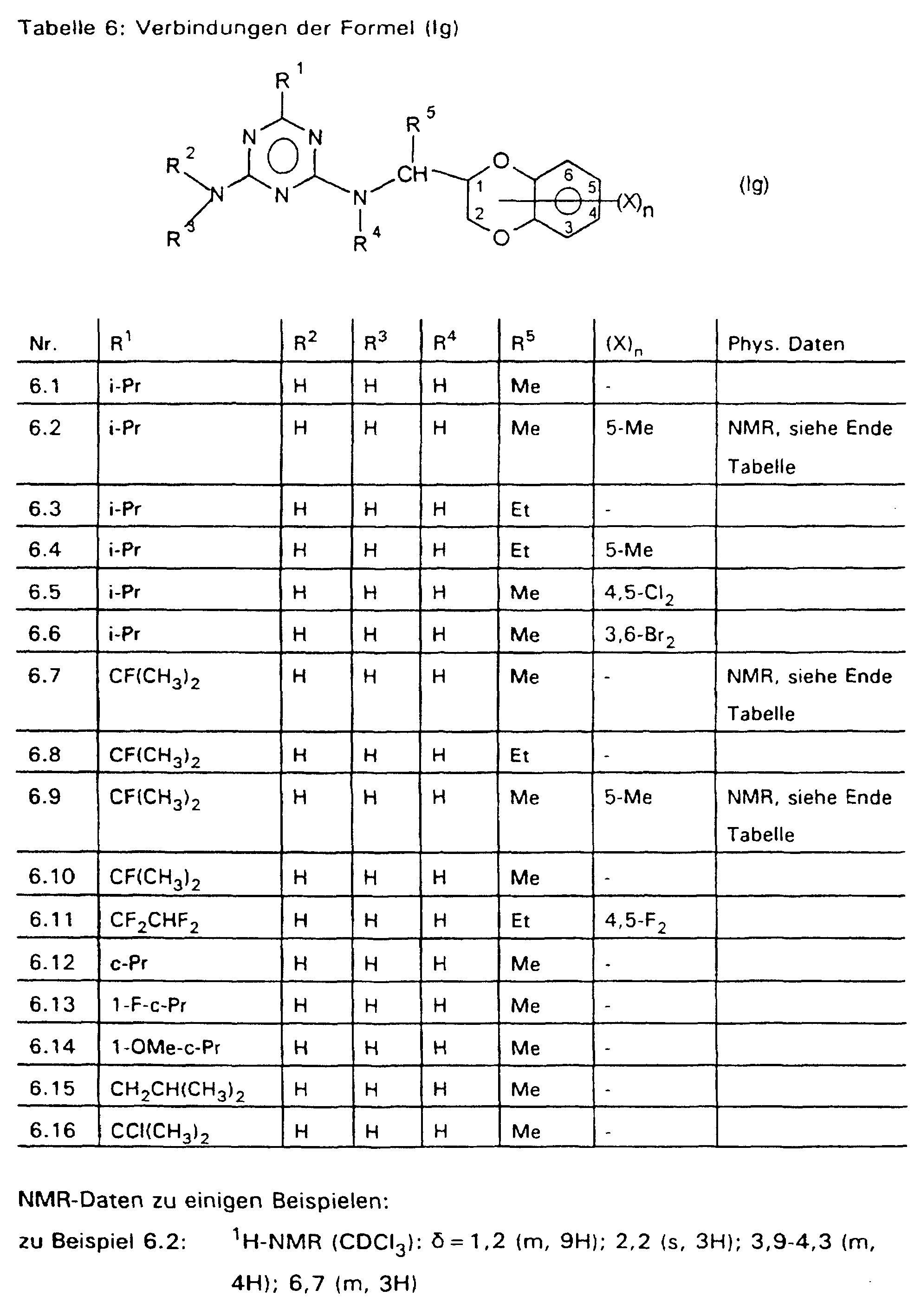

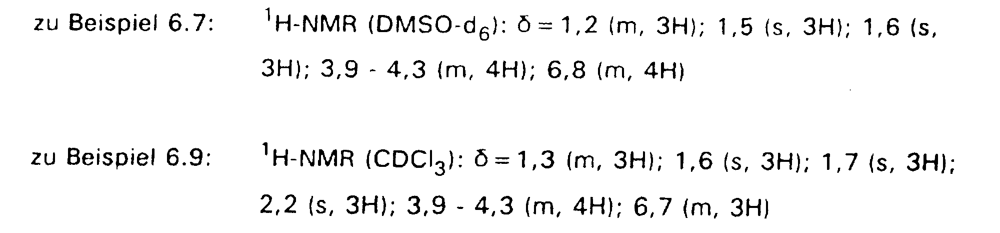

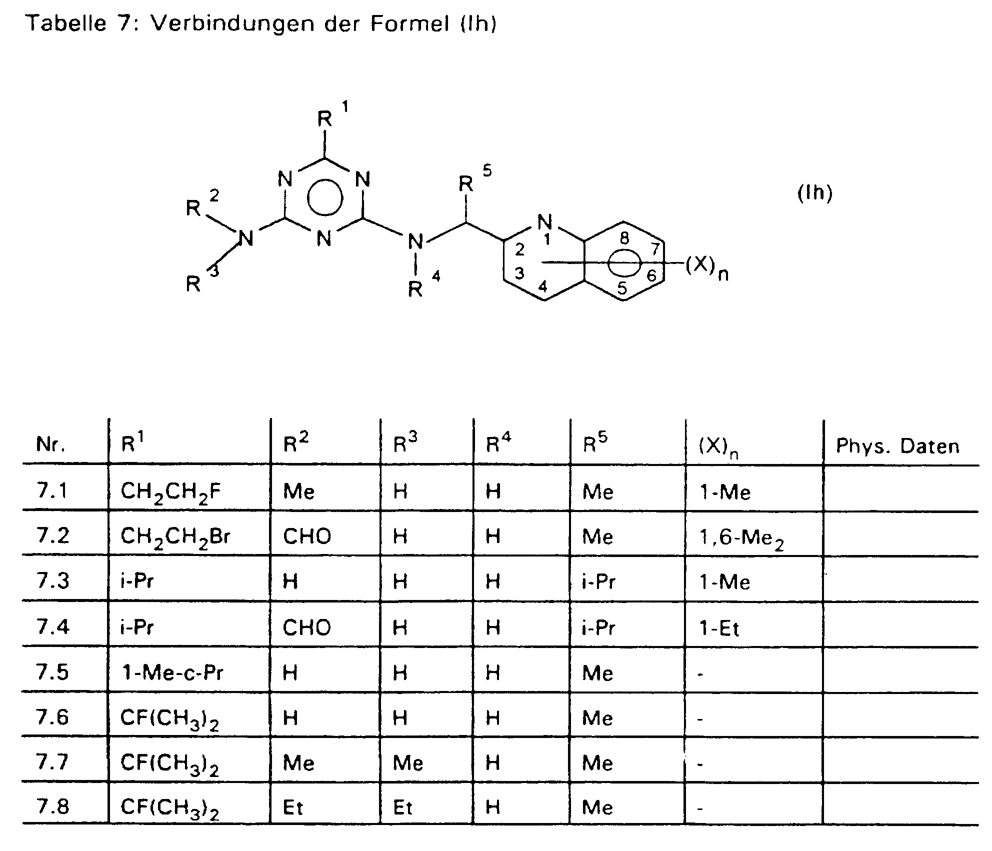

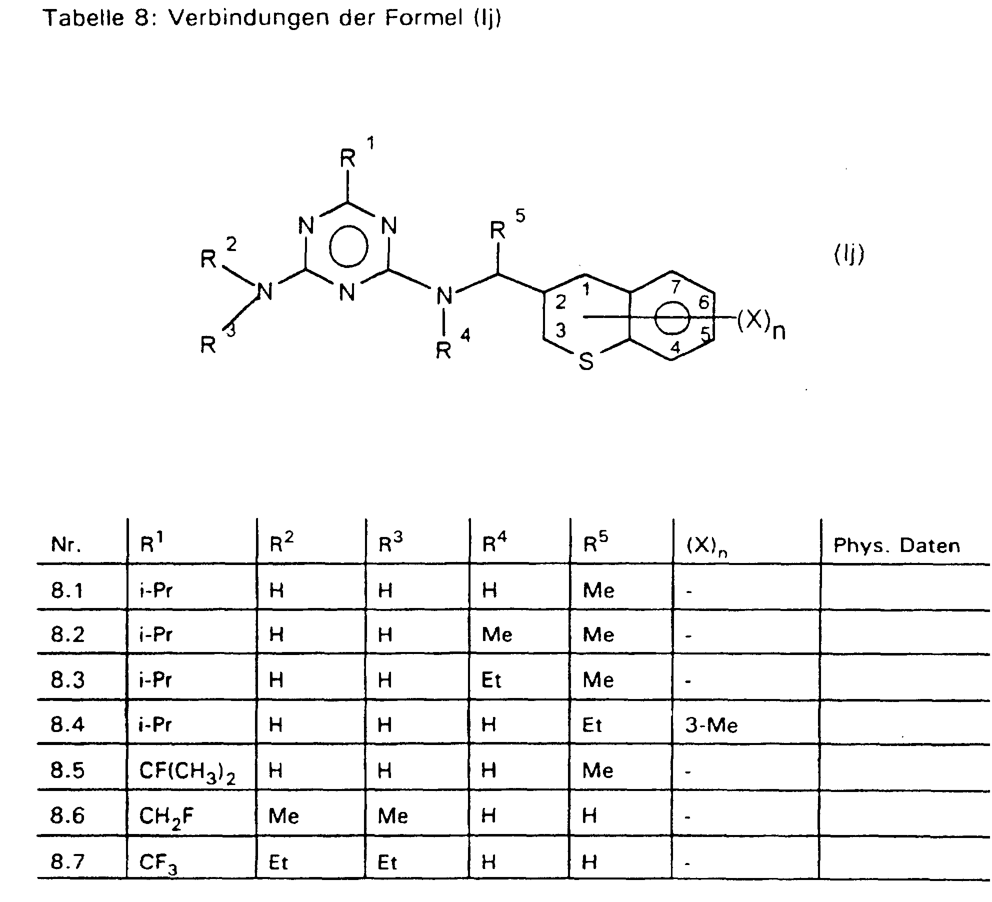

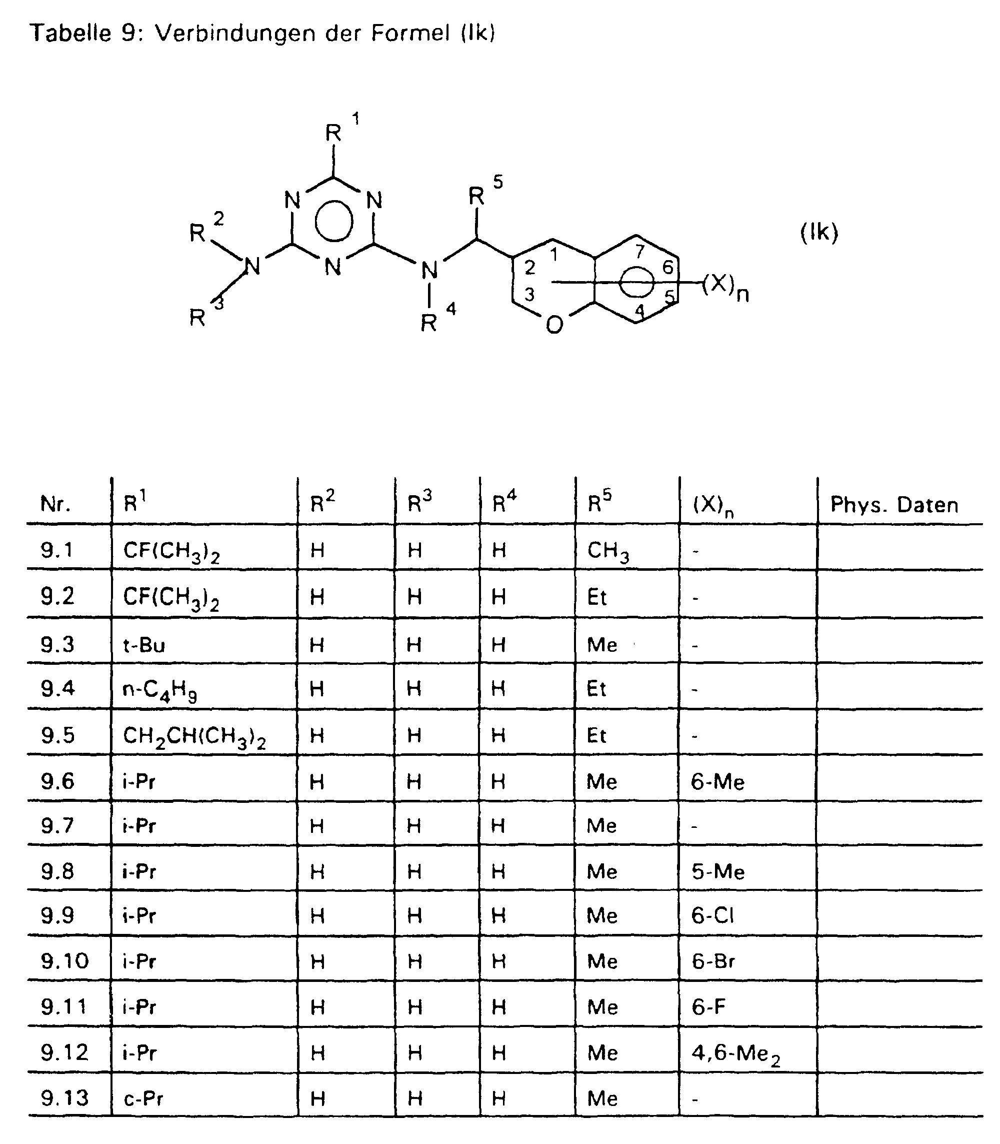

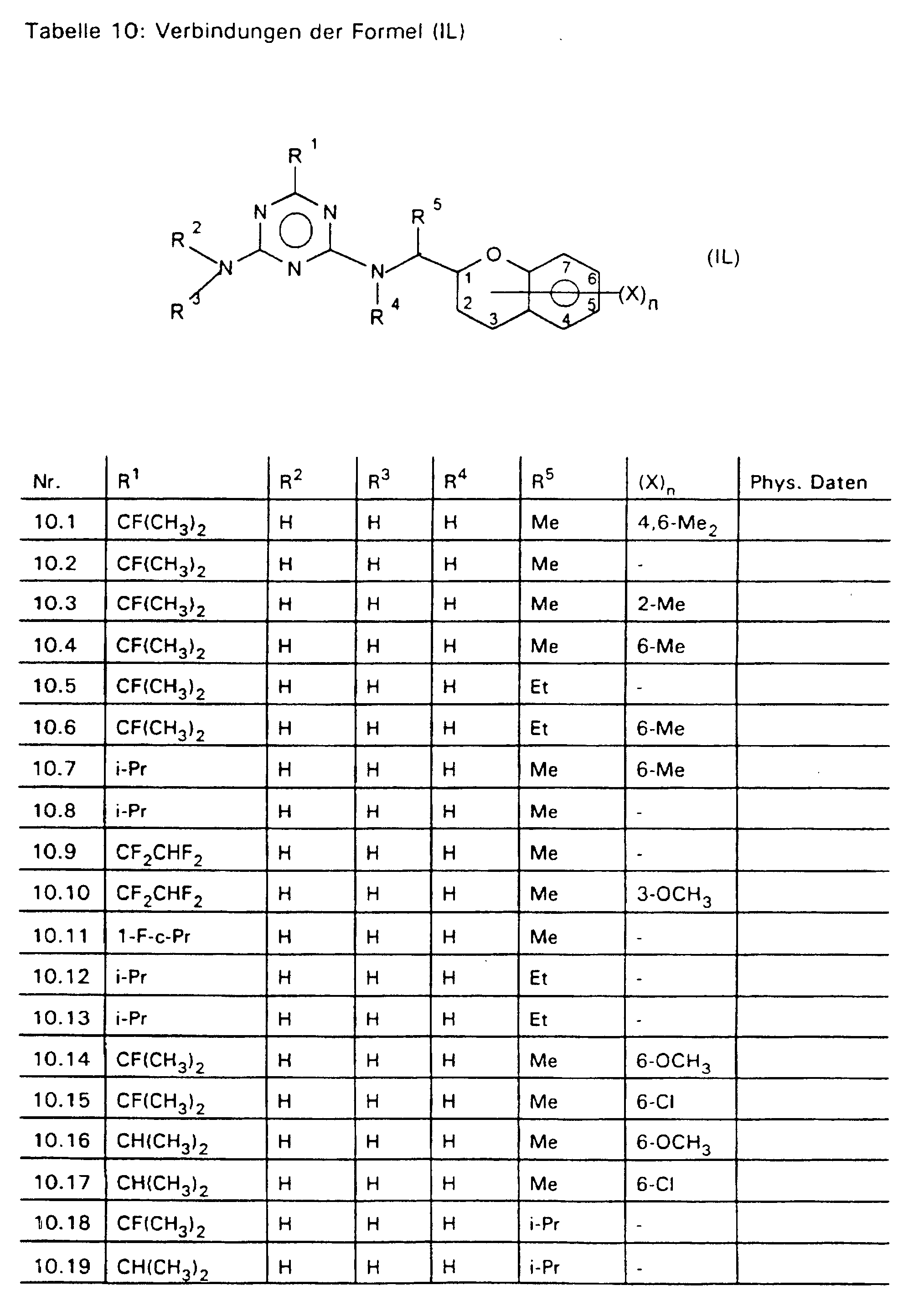

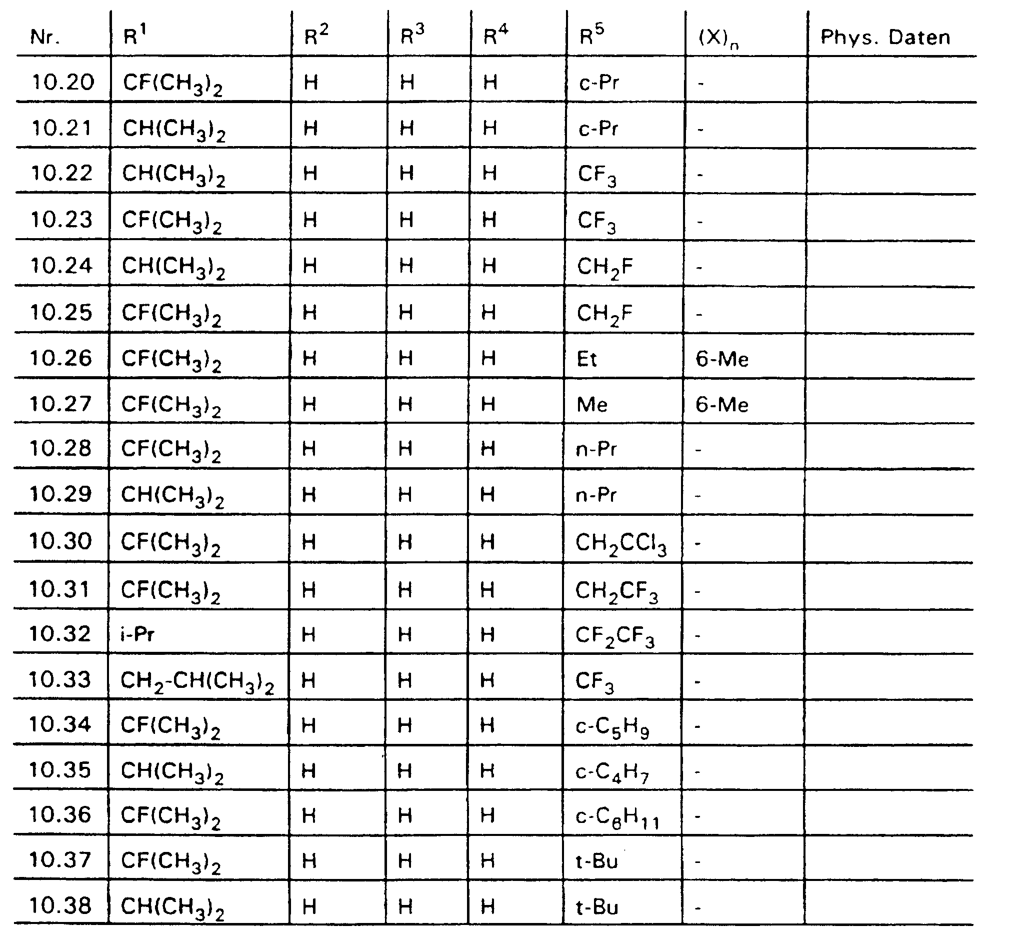

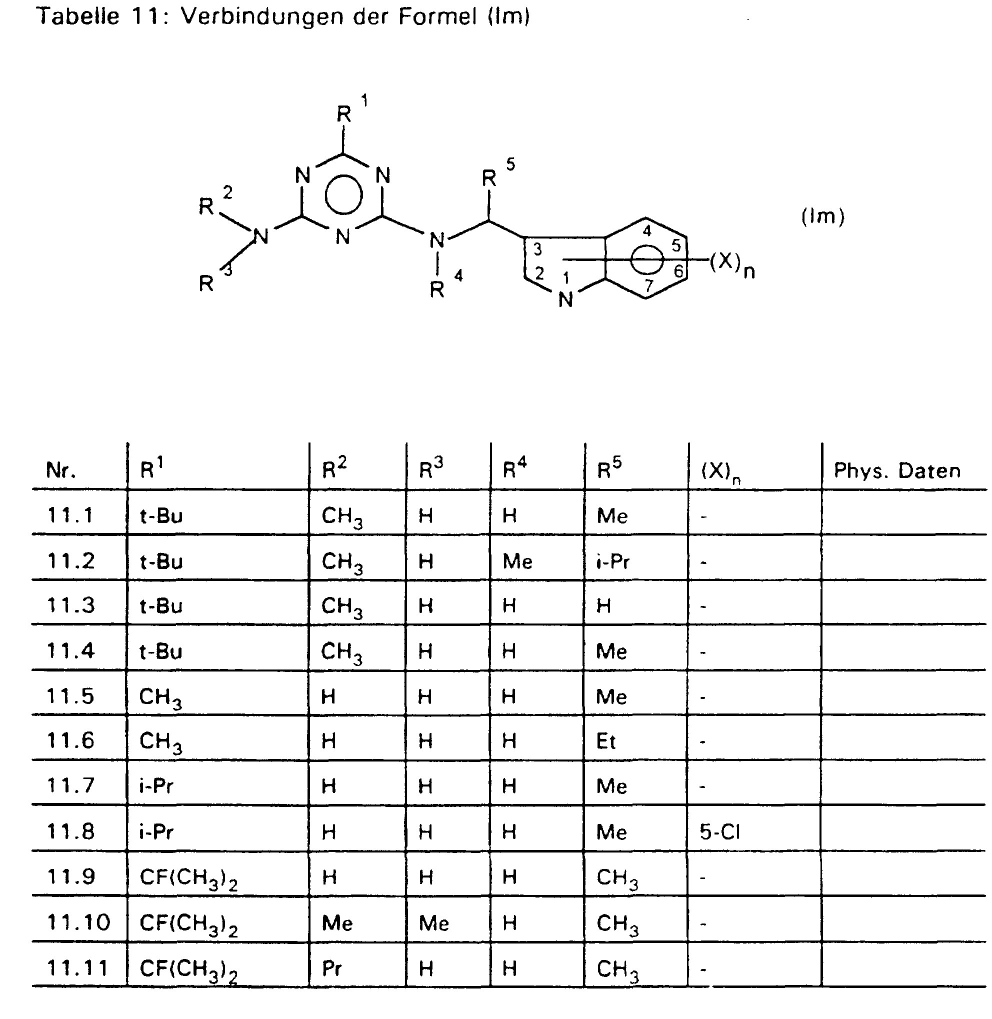

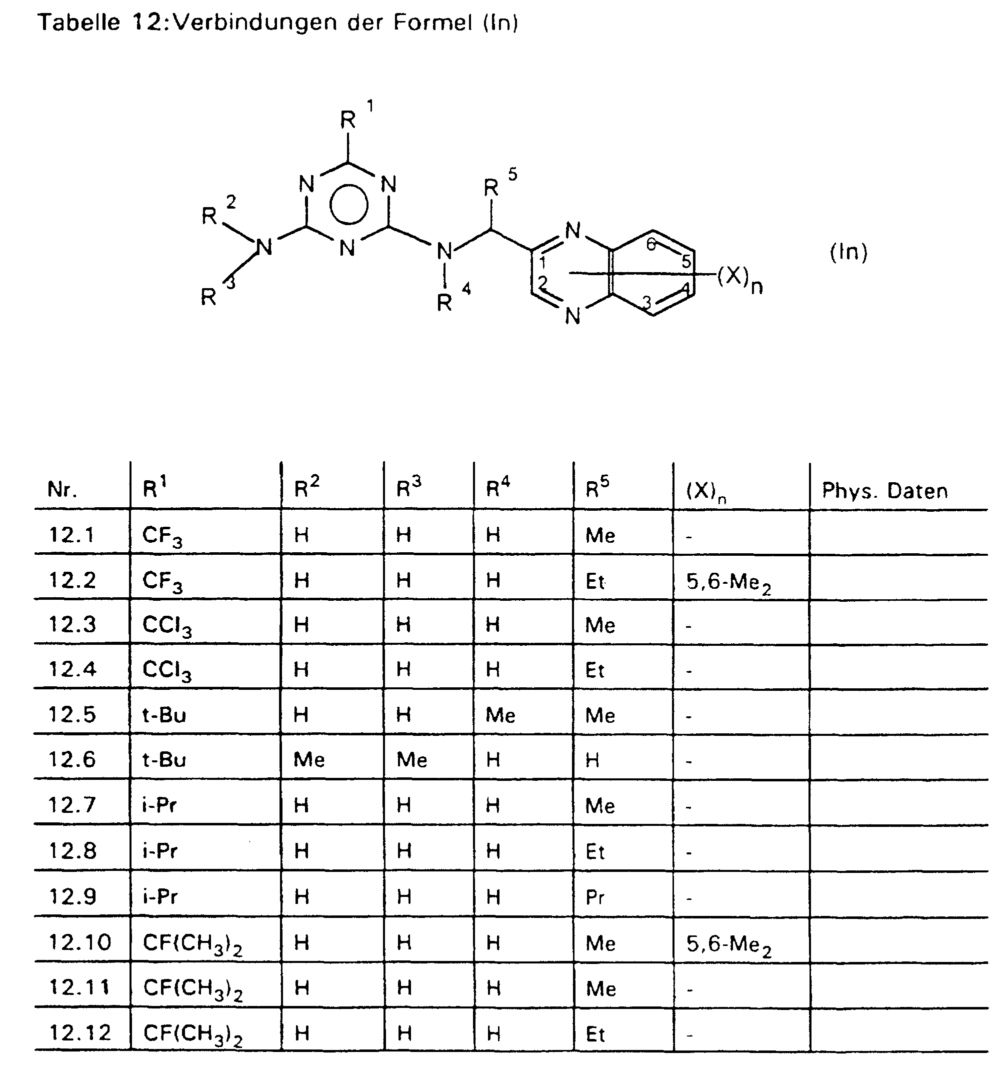

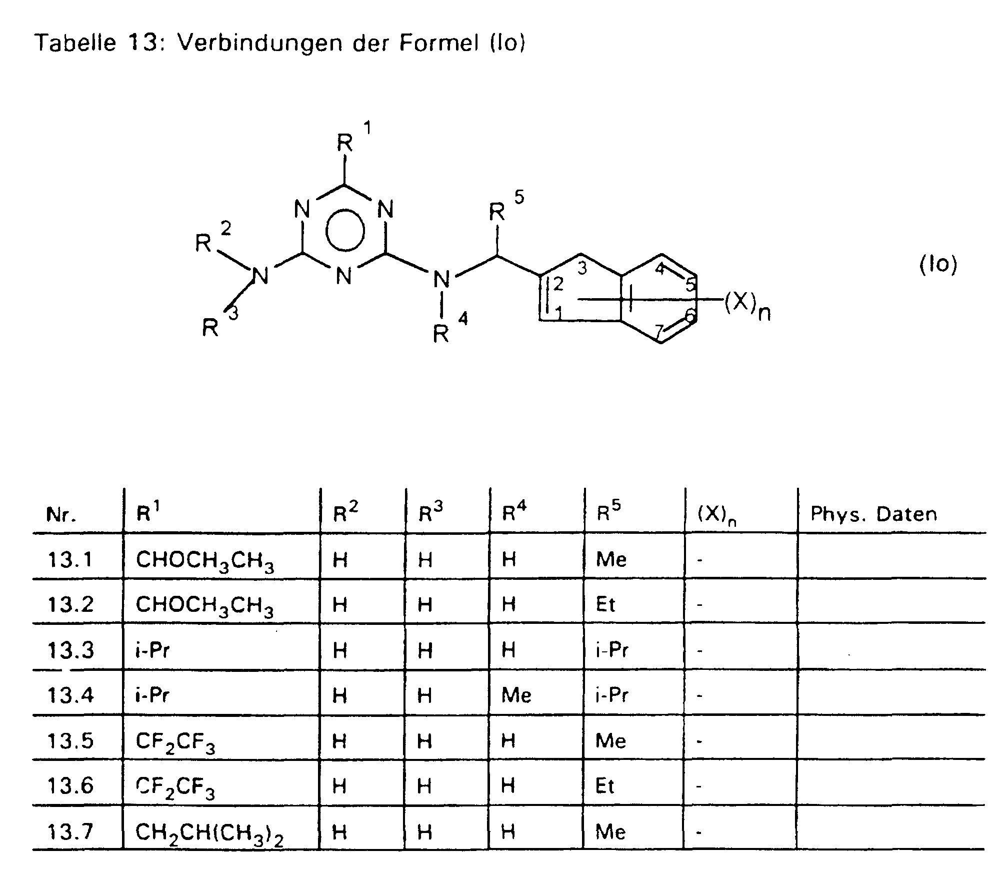

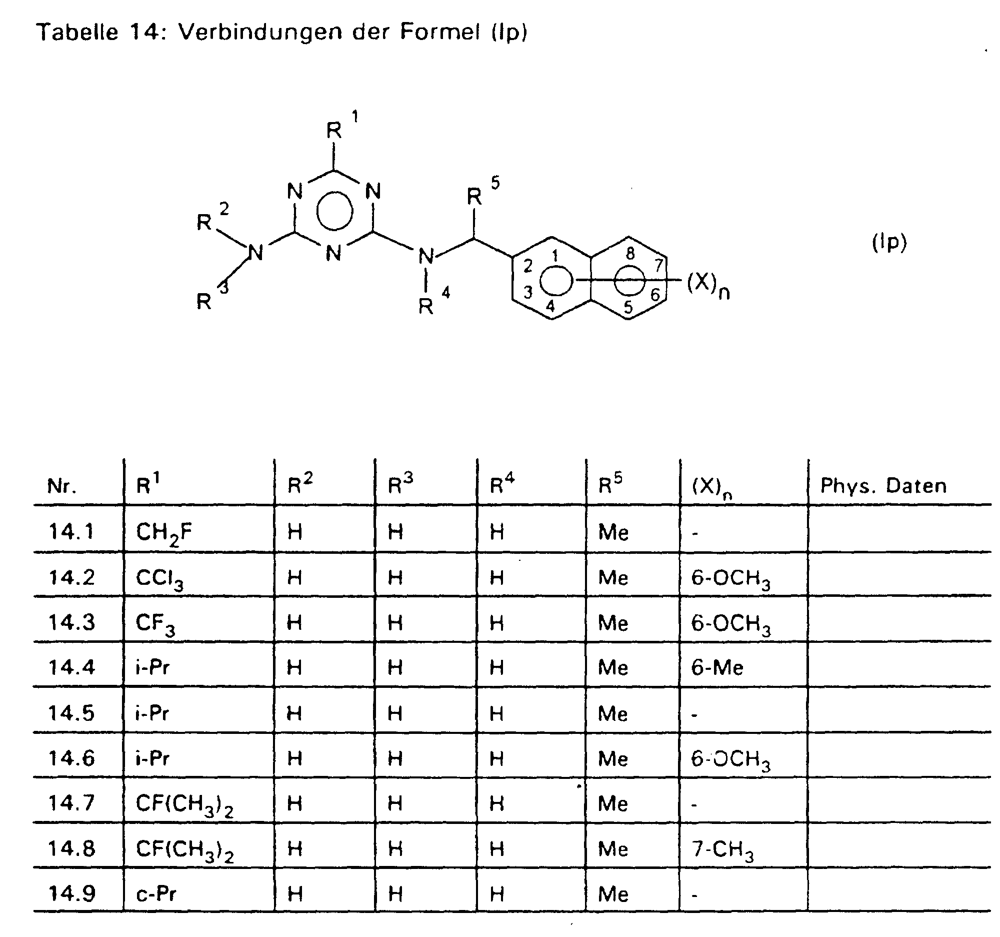

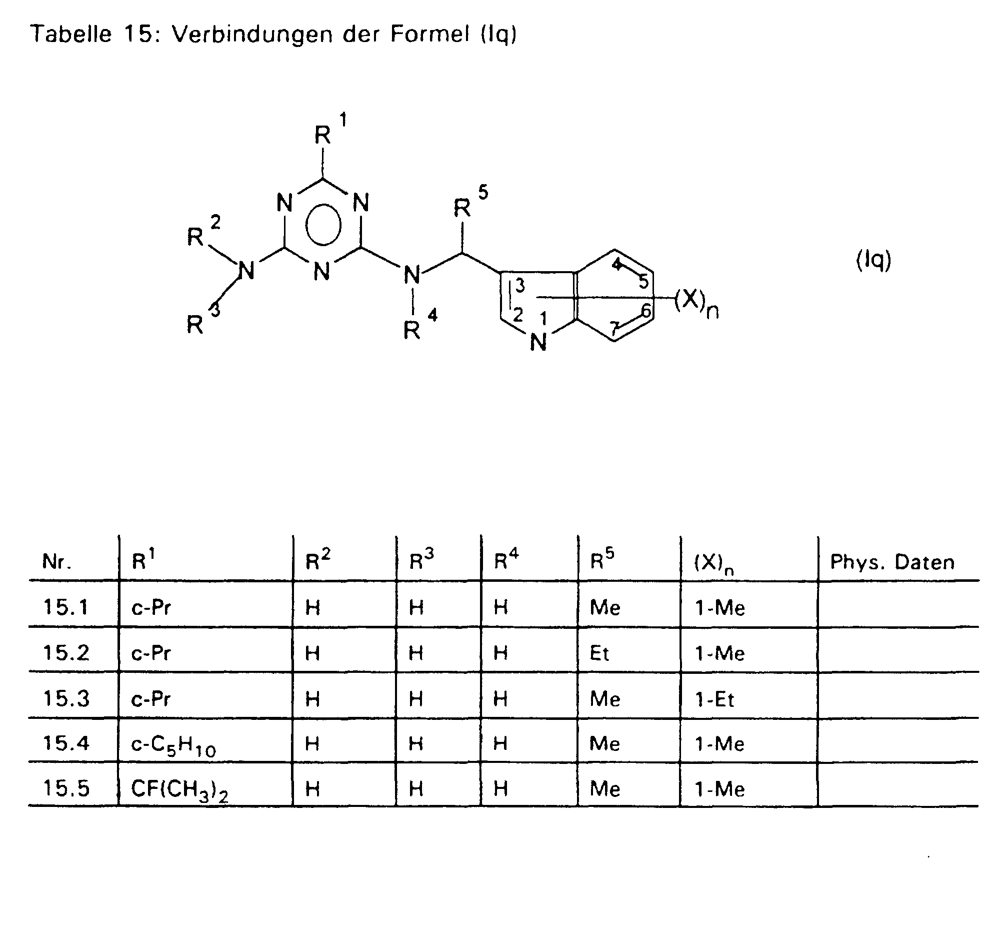

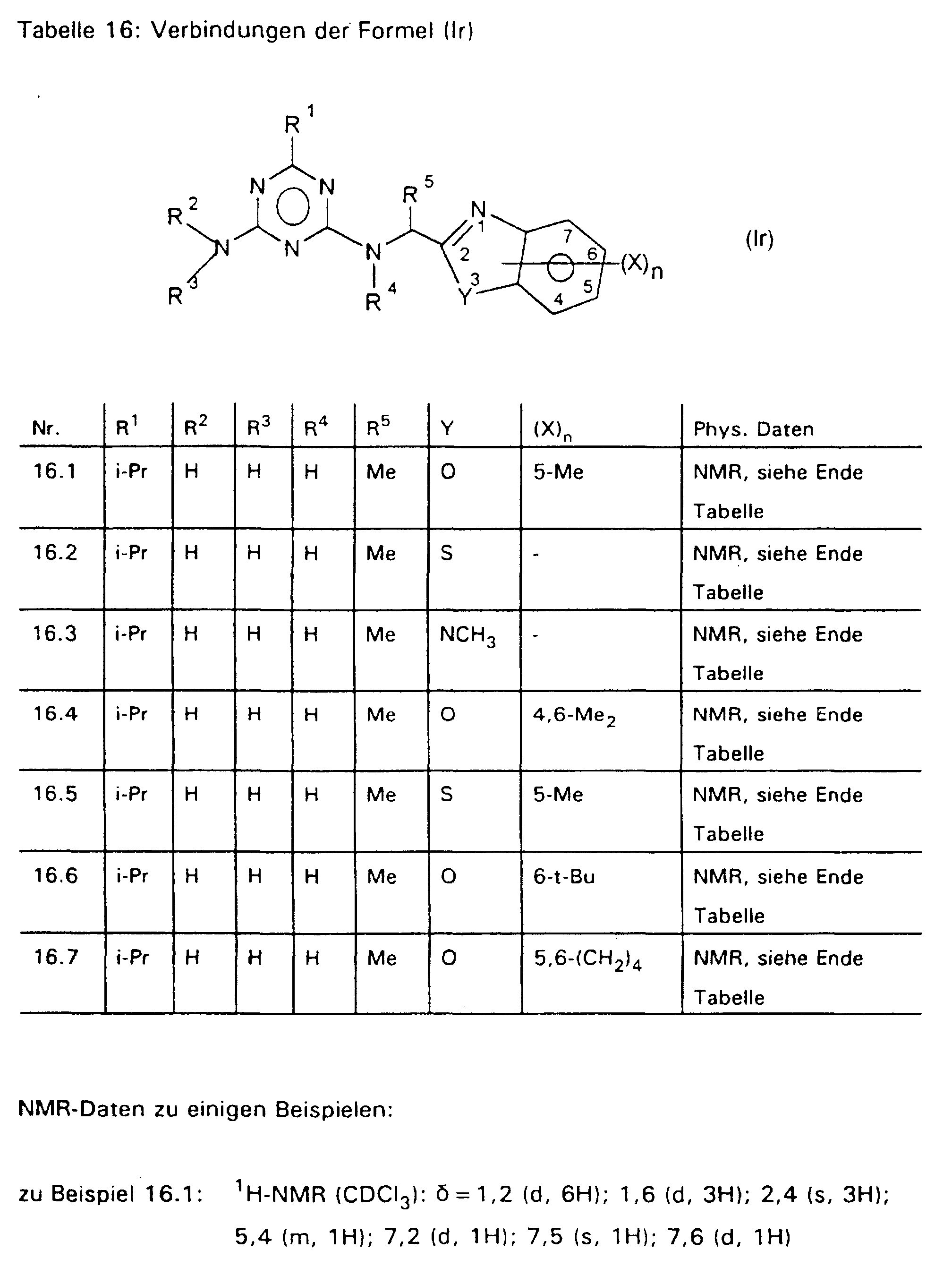

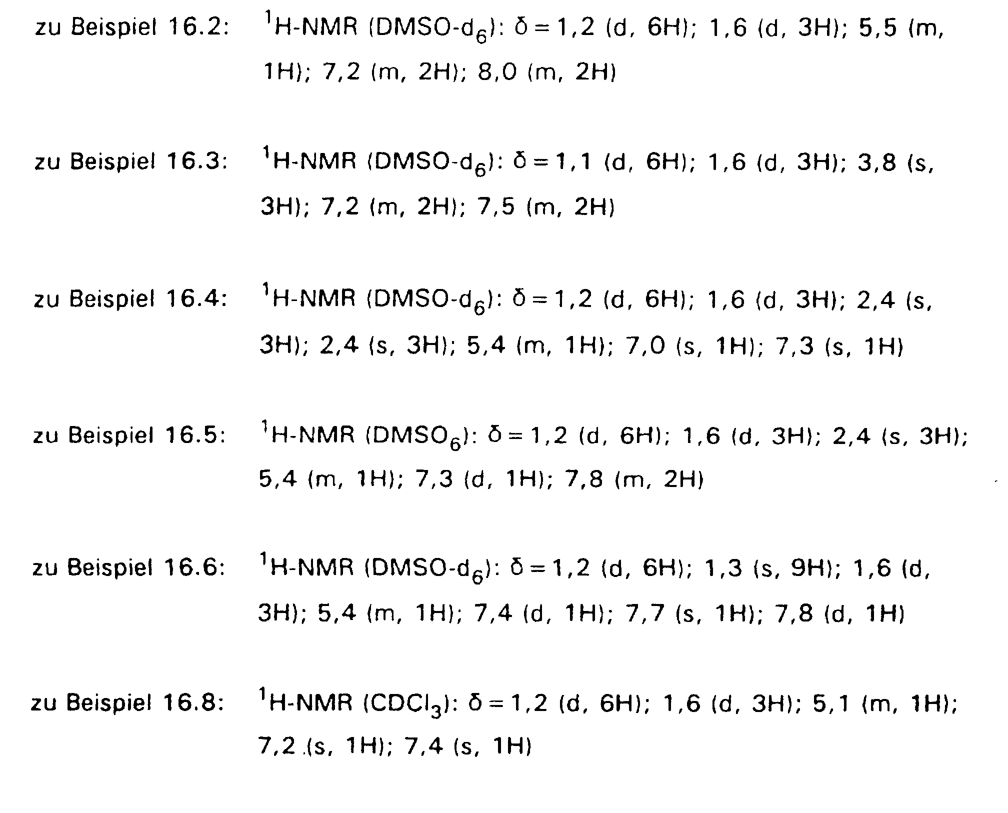

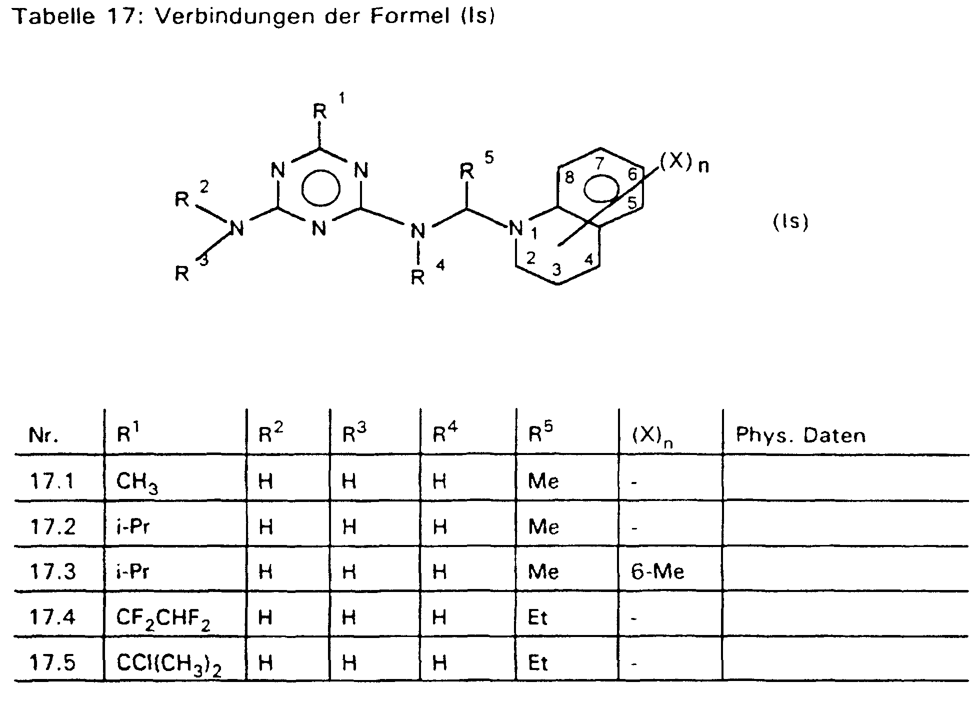

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | | |
|---|---|---|
| 75 | Gewichtsteile | einer Verbindung der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | | |
|---|---|---|
| 25 | Gewichtsteile | einer Verbindung der Formel (I), |
| 5 | " | 2,2'-dinaphthylmethan-6,6'-disulfonsaures Natrium |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise zeigen im Test die Beispiele Nr. 2.5, 2.10, 2.17, 2.18, 2.20, 2.30, 2.68, 2.70, 2.71, 2.72, 3.8, 3.10, 6.1, 6.7, 6.9, 6.16, 10.2, 10.8, 16.1 und 16.2 der Tabellen 2, 3, 6, 10 und 16 sehr gute herbizide Wirkung gegen Schadpflanzen wie Stellaria media, Lolium multiflorum, Matricaria inodora, Echinochloa crus-galli, Sinapis alba, Avena sativa, Cyperus esculentus und Cyperus iria im Vorauflaufverfahren bei einer Aufwandmenge von 1,25 kg oder weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise zeigen im Test die Beispiele Nr. 2.5, 2.10, 2.17, 2.18, 3.10, 6.1, 6.2, 6.7, 6.9, 16.1 und 16.2 der Tabellen 2, 3, 6 und 16 sehr gute herbizide Wirkung gegen Schadpflanzen wie Sinapis alba, Echinochloa crus-galli, Lolium multiflorum, Stellaria media, Matricaria inodora, Cyperus esculentus, Cyperus iria und Avena sativa im Nachauflaufverfahren bei einer Aufwandmenge von 1,25 kg und weniger Aktivsubstanz pro Hektar.

### 3. Wirkung auf Schadpflanzen in Reis

Verpflanzter und gesäter Reis sowie typische Reisunkräuter und -ungräser werden im Gewächshaus bis zum Dreiblattstadium (Echinochloa 1,5-Blatt) unter Paddyreis-Bedingungen (Anstauhöhe des Wassers: 2 - 3 cm) in geschlossenen Plastiktöpfen angezogen. Danach erfolgt die Behandlung mit den erfindungsgemäßen Verbindungen. Hierzu werden die formulierten Wirkstoffe in Wasser suspendiert, gelöst bzw. emulgiert und mittels Gießapplikation in das Anstauwasser der Test-pflanzen in unterschiedlichen Dosierungen ausgebracht. Nach der so durchgeführten Behandlung werden die Versuchspflanzen im Gewächshaus unter optimalen Wachstumsbedingungen aufgestellt und während der gesamten Versuchszeit so gehalten.

Etwa drei Wochen nach der Applikation erfolgt die Auswertung mittels optischer Bonitur der Pflanzenschäden im Vergleich zu unbehandelten Kontrollen. Die erfindungsgemäßen Verbindungen weisen sehr gute herbizide Wirkung gegen Schadpflanzen auf. Beispielsweise zeigen Verbindungen der Beispiele 2.10, 2.17, 2.18, 2.20, 2.30, 2.68, 2.70, 2.71, 2.72, 3.10, 6.1, 6.7, 6.16, 10.2 und 10.8 der Tabellen 2, 3, 6 bzw.10 im Test sehr gute Wirkung gegen Schadpflanzen, die typisch für Reiskulturen sind, wie z.B. Cyperus monti, Echinochloa crus-galli und Sagittaria pygmaea.

### 4. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vorund Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum-Hirsen, Mais oder Reis. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) und deren Salze, 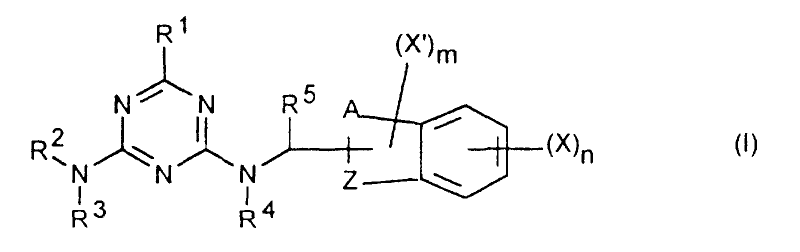 worin
R¹ (C₁-C₆)Alkyl,
das unsubstituiert oder durch einen oder mehrere Reste aus der Gruppe Halogen, Cyano, Nitro, Thiocyanato, Hydroxy, (C₁-C₄)-Alkoxy, (C₁-C₄)Alkylthio, (C₁-C₄)Alkylsulfinyl, (C₁-C₄)Alkylsulfonyl, (C₂-C₄)Alkenyl, (C₂-C₄)Alkinyl, Phenyl, das unsubstituiert oder substituiert ist, und (C₃-C₉)Cycloalkyl, das unsubstituiert oder substituiert ist, und Heterocyclyl mit 3 bis 6 Ringatomen und 1 bis 3 Heteroringatomen aus der Gruppe N, O und S, wobei der Ring unsubstituiert oder substituiert ist, substituiert ist, oder
einen carbocyclischen oder heterocyclischen Rest mit 3 bis 6 Ringatomen, der unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist, wobei Substituenten paarweise auch einen carbocylischen oder heterocyclischen Ring mit 3 bis 6 Ringatomen bilden können, der unsubstituiert oder substituiert ist,
R² und R³ jeweils unabhängig voneinander Wasserstoff, Amino oder Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen, oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest oder
R² und R³ gemeinsam mit dem Stickstoffatom der Gruppe NR²R³ einen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder substituiert ist,
R⁴ Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 10 C-Atomen, oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder substituiert ist, oder einen Acylrest,
R⁵ Wasserstoff, Nitro, Cyano, Thiocyanato oder einen Rest der Formel -B²-Y², wobei B² und Y² wie weiter unten definiert sind,
A-Z eine divalente Brücke der Formel
-(CH₂)ₐ- ,
worin a die ganze Zahl 2, 3 oder 4 ist,
-W¹-(CH₂)_{b}-W²- ,
worin b die ganze Zahl 1 oder 2 ist und W¹, W² unabhängig voneinander O oder S bedeuten,
-CH₂CH₂CH₂-W³- ,
worin W³ O oder S bedeutet,
(X')ₘ m Substituenten X' und dabei X' jeweils unabhängig voneinander Halogen, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder substituiert sind, oder die Oxogruppe,
(X)ₙ n Substituenten X und dabei X jeweils unabhängig voneinander Halogen, Nitro, Cyano, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder substituiert sind, oder einen Rest der Formel -B³-Y³, wobei B³ und Y³ wie unten definiert sind,
oder zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
B¹,B²,B³ jeweils unabhängig voneinander eine direkte Bindung oder eine divalente Gruppe der Formel -O-, -S(O)ₚ-, -S(O)ₚ-O-, -O-S(O)ₚ-, -CO-, -O-CO-, -CO-O-, -NR'-, -0-NR'-, -NR'-O-, -NR'-CO- oder -CO-NR'-, wobei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist,
Y¹,Y² jeweils unabhängig voneinander H oder einen acyclischen Kohlenwasserstoffrest oder einen cyclischen Kohlenwasserstoffrest mit 3 bis 8 C-Atomen oder einen heterocyclischen Rest mit 3 bis 9 Ringatomen und 1 bis 3 Heteroringatomen aus der Gruppe N, O und S, wobei jeder der drei letztgenannten Reste unsubstituiert oder substituiert ist,
Y³ einen aromatischen, gesättigten oder teilgesättigten carbocyclischen oder heterocyclischen Rest bedeutet, wobei der cyclische Rest substituiert oder unsubstituiert ist,
m eine ganze Zahl von Null bis zur Anzahl der Wasserstoffatome im Grundkörper der divalenten Brücke,
n 0, 1, 2, 3 oder 4,
bedeuten.

2. Verbindungen der Formel (I) oder deren Salze nach Anspruch 1, **dadurch gekennzeichnet, daß**
R¹ (C₁-C₄)Alkyl,
das unsubstituiert oder durch einen oder mehrere Reste aus der Gruppe Halogen, (C₁-C₄)Alkoxy, (C₁-C₄)Alkylthio, (C₁-C₄)Alkylsulfonyl, Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl und (C₁-C₄)Alkylsulfonyl substituiert ist, und (C₃-C₉)Cycloalkyl, das unsubstituiert oder durch einen oder mehrere Reste (C₁-C₄)Alkyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, (C₁-C₄)Alkyl und Oxo substituiert ist,
substituiert ist, oder
(C₃-C₇)Cycloalkyl, (C₃-C₇)Cycloalkenyl oder einen heterocyclischen Rest mit 3 bis 6 Ringatomen und 1 bis 3 Heteroringatomen aus der Gruppe N, O und S, wobei der Cycloalkylrest oder der heterocyclische Rest jeweils unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist und/oder Substituenten aufweist, die paarweise einen ankondensierten Benzolring oder einen ankondensierten oder in Spiroform verknüpften Ring aus der Gruppe (C₃-C₇)Cycloalkyl, (C₃-C₇)Cycloalkenyl und einen heterocyclischen Ring mit 3 bis 6 Ringatomen bilden können, der unsubstituiert oder durch einen oder mehrere Reste aus der Gruppe Halogen, (C₁-C₄)Alkyl, (C₁-C₄)Haloalkyl und Oxo substituiert ist, oder Phenyl, das unsubstituiert oder substituiert ist,
R² und R³ jeweils unabhängig voneinander Wasserstoff, Amino oder Alkylamino oder Dialkylamino mit jeweils 1 bis 4 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest oder
R² und R³ gemeinsam mit dem Stickstoffatom der Gruppe NR²R³ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
R⁴ Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
oder einen Acylrest,
R⁵ Wasserstoff, Nitro, Cyano, Thiocyanato oder einen Rest der Formel -B²-Y², wobei B² und Y² wie weiter unten definiert sind,
A-Z eine divalente Brücke der Formel
-(CH₂)ₐ- ,
worin a die ganze Zahl 2, 3 oder 4 ist,
-W¹-(CH₂)_{b}-W²- ,
worin b die ganze Zahl 1 oder 2 ist und W¹, W² unabhängig voneinander S oder O bedeuten,
-CH₂CH₂CH₂-W³- ,
worin W³ O oder S bedeutet,
(X')ₘ m Substituenten X' und dabei X' jeweils unabhängig voneinander Halogen, (C₁-C₆)Alkyl, (C₂-C₆)Alkenyl, (C₂-C₆)Alkinyl, (C₁-C₆)Alkoxy, (C₂-C₆)Alkenyloxy, (C₂-C₆)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder durch einen oder mehrere Reste aus der Gruppe Halogen, (C₁-C₄)Alkoxy und (C₁-C₄)Alkylthio substituiert sind, oder die Oxogruppe,
(X)ₙ n Substituenten X und dabei X jeweils unabhängig voneinander Halogen, Nitro, Cyano, (C₁-C₆)Alkyl, (C₁-C₆)Alkoxy, (C₁-C₄)Alkylcarbonyl, (C₁-C₄)Alkyloxycarbonyl oder (C₁-C₄)Alkylthiocarbonyl, wobei die letztgenannten fünf Reste unsubstituiert oder durch Halogen oder (C₁-C₄)Alkoxy substituiert sind, einen Rest der Formel -B³-Y³, wobei B³ und Y³ wie unten definiert sind, oder
zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
B¹,B²,B³ jeweils unabhängig voneinander eine direkte Bindung oder eine divalente Gruppe der Formel -O-, -S-, -CO-, -O-CO-, -CO-O-, -NR'-, -NR'-CO- oder -CO-NR'-, wobei R' H oder (C₁-C₄)Alkyl ist,
Y¹,Y² jeweils unabhängig voneinander H oder einen acyclischen Kohlenwasserstoffrest mit 1 bis 6 C-Atomen, einen cyclischen Kohlenwasserstoffrest mit 3 bis 6 C-Atomen oder einen heterocyclischen Rest mit 3 bis 9 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₂-C₄)Alkenyl, (C₂-C₄)Alkinyl, (C₂-C₄)Alkenyloxy, (C₂-C₄)Alkinyloxy, Hydroxy, Amino, Acylamino, Mono- und Dialkylamino, Nitro, Carboxy, Cyano, Azido, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
Y³ (C₃-C₆)Cycloalkyl, das unsubstituiert oder durch Reste aus der Gruppe (C₁-C₄)Alkyl und (C₁-C₄)Haloalkyl substituiert ist, oder Phenyl, das unsubstituiert oder durch Reste aus der Gruppe Halogen, (C₁-C₄)Alkyl, (C₁-C₄)Haloalkyl und (C₁-C₄)Alkoxy substituiert ist,
m 0, 1 oder 2,
n 0, 1, 2, 3 oder 4,
bedeuten.

3. Verbindungen der Formel (I) oder deren Salze nach Anspruch 1 oder 2, **dadurch gekennzeichnet, daß**
R¹ (C₁-C₄)Alkyl,
das unsubstituiert oder durch einen oder mehrere Reste aus der Gruppe Halogen, (C₁-C₄)Alkoxy, (C₁-C₄)Alkylthio und Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Nitro, Cyano, [(C₁-C₂)Alkyl]carbonyl, Formyl, Carbamoyl, Mono- und Di-[(C₁-C₂)alkyl]aminocarbonyl und (C₁-C₄)Alkylsulfonyl und (C₃-C₇)Cycloalkyl, das unsubstituiert oder durch einen oder mehrere Reste (C₁-C₄)Alkyl substituiert ist,
substituiert ist, oder
(C₃-C₇)Cycloalkyl oder einen heterocyclischen Rest mit 3 bis 6 Ringatomen und 1 oder 2 Heteroringatomen aus der Gruppe N, O und S, wobei der Cycloalkylrest oder de: heterocyclische Rest jeweils unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist und/oder Substituenten aufweist, die paarweise einen in Spiroform verknüpften Ring aus der Gruppe (C₃-C₇)Cycloalkyl bilden können, der unsubstituiert oder durch einen oder mehrere Reste aus der Gruppe Halogen, (C₁-C₄)Alkyl, (C₁-C₄)Haloalkyl und Oxo substituiert ist,
R² und R³ unabhängig voneinander Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocyclyl-(C₁-C₄)alkyl, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder
Phenyl, Phenoxy, Phenylcarbonyl, Phenoxy-carbonyl, Phenylcarbonyl-(C₁-C₄)alkyl, (C₁-C₄)Alkoxy-carbonyl-(C₁-C₄)alkyl, (C₁-C₄)Alkylaminocarbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 17 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
R² und R³ gemeinsam mit dem Stickstoffatom der Gruppe NR²R³ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist,
R⁴ Wasserstoff, Amino, Formyl, (C₁-C₄)Alkyl, Cyano-(C₁-C₄)alkyl, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, (C₃-C₉)Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder Phenyl, Phenoxy, Phenylcarbonyl, Phenoxycarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio, oder einen der letztgenannten 17 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxycarbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält,
R⁵ Wasserstoff, (C₁-C₄)Alkyl, (C₁-C₄)Haloalkyl oder (C₃-C₇)Cycloalkyl,
A-Z eine divalente Brücke der Formel
-CH₂CH₂-, -CH₂CH₂CH₂-, -CH₂CH₂CH₂CH₂-,
-S-CH₂S-, -S-CH₂-O-, -O-CH₂-O-,
-S-CH₂CH₂S-, -S-CH₂CH₂-O-, -O-CH₂CH₂-O-,
-CH₂CH₂CH₂-O-, -CH₂CH₂CH₂-S-,
(X')ₘ m Substituenten X' und dabei X' jeweils unabhängig voneinander Halogen, (C₁-C₄)Alkyl, (C₂-C₄)Alkenyl, (C₂-C₄)Alkinyl, (C₁-C₄)Alkoxy, (C₂-C₄)Alkenyloxy, (C₂-C₄)Alkinyloxy, [(C₁-C₄)Alkyl]-carbonyl, [(C₁-C₄)Alkoxy]-carbonyl oder [(C₁-C₄)Alkylthio]-carbonyl, wobei die kohlenwasserstoffhaltigen Teile in den letztgenannten 9 Resten unsubstituiert oder durch einen oder mehrere Reste aus der Gruppe Halogen substituiert sind, oder die Oxogruppe,
(X)ₙ n Substituenten X und dabei X jeweils unabhängig voneinander Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder
Phenyl, Phenoxy, Phenylcarbonyl, Phenoxycarbonyl,
Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 17 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxycarbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
B¹ eine direkte Bindung oder eine divalente Gruppe der Formel -O-, -S-, -CO-, -O-CO-, -CO-O-, -NR'-, -NR'-CO- oder -CO-NR'-, wobei R' H oder (C₁-C₄)Alkyl ist,
Y¹ H oder einen acyclischen Kohlenwasserstoffrest mit 1 bis 4 C-Atomen, einen cyclischen Kohlenwasserstoffrest mit 3 bis 6 C-Atomen oder einen heterocyclischen Rest mit 3 bis 6 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, (C₁-C₄)Alkoxy, (C₁-C₄)Haloalkoxv, (C₁-C₄)Alkylthio, Hydroxy, Amino, Mono- und Dialkylamino, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, (C₁-C₄)Alkylsulfinyl, (C₁-C₄)Haloalkylsulfinyl, (C₁-C₄)Alkylsulfonyl, (C₁-C₄)Haloalkylsulfonyl und, im Falle cyclischer Reste, auch (C₁-C₄)Alkyl und (C₁-C₄)Haloalkyl substituiert ist,
bedeuten.

4. Verbindungen der Formel (I) und deren Salze, nach einem der Ansprüche 1 bis 3, **dadurch gekennzeichnet, daß**
R¹ (C₁-C₄)Alkyl, (C₁-C₄)Haloalkyl, Benzyl, [(C₃-C₆)Cycloalkyl]-(C₁-C₂)alkyl, (C₃-C₆)Cycloalkyl oder einen heterocyclischen Rest mit 3 bis 6 Ringatomen und 1 oder 2 Heteroringatom aus der Gruppe N und O, vorzugsweise ein Sauerstoffatom als Heteroringatom, wobei der Cycloalkylrest oder der heterocyclische Rest jeweils unsubstituiert oder durch einen oder mehrere Reste aus der Gruppe Halogen, Nitro, Cyano, Thiocyanato und einen Rest der Formel B¹-Y¹, wobei B¹ und Y¹ wie weiter unten definiert sind, substituiert ist und/oder Substituenten aufweist, die paarweise einen in Spiroform verknüpften Ring aus der Gruppe (C₃-C₇)Cycloalkyl bilden können, der unsubstituiert oder durch einen oder mehrere Reste aus der Gruppe Halogen, (C₁-C₄)Alkyl, (C₁-C₄)Haloalkyl und Oxo substituiert ist,
R² und R³ unabhängig voneinander Wasserstoff, Amino, Formyl, (C₁-C₄)Alkyl, (C₁-C₄)Alkoxy-(C₁-C₄)alkyl, (C₃-C₆)Alkenyl, (C₃-C₆)Alkinyl, (C₁-C₄)Alkylamino-(C₁-C₄)alkyl, Di-[(C₁-C₄)alkyl]-amino-(C₁-C₄)alkyl oder Phenyl, Phenyl-(C₁-C₄)alkyl, Phenylcarbonyl oder Phenoxycarbonyl oder einen der letztgenannten vier Reste, der im Phenylteil bis zu dreifach durch Reste aus der Gruppe Halogen, (C₁-C₄)Alkyl, (C₁-C₄)Alkoxy und (C₁-C₄)Alkoxy-carbonyl substituiert ist, oder
R² und R³ gemeinsam mit dem Stickstoffatom der Gruppe NR²R³ einen heterocyclischen Rest mit 3 bis 6 Ringatomen und 1 bis 2 Heteroringatomen, wobei neben dem N-Atom das gegebenenfalls weitere Heteroringatom aus der Gruppe N und O ausgewählt ist und der Rest unsubstituiert oder durch einen oder mehrere Reste aus der Gruppe Halogen, (C₁-C₄)Alkyl und Oxo substituiert ist,
R⁴ Wasserstoff, Amino, Formyl, (C₁-C₄)Alkyl, Di-[(C₁-C₄)alkyl]-amino, (C₁-C₄)Alkoxy-(C₁-C₄)alkyl, (C₃-C₆)Alkenyl, (C₃-C₆)Alkinyl, (C₁-C₄)Dialkylamino-(C₁-C₄)alkyl, Phenyl, Phenoxy-(C₁-C₄)alkyl, Phenyl-(C₁-C₄)alkyl, Phenoxy-carbonyl, Phenylamino-carbonyl oder einen der letztgenannten fünf Reste, der im Phenylteil einfach bis dreifach durch Reste aus der Gruppe Halogen, (C₁-C₄)Alkyl, (C₁-C₄)Alkoxy und (C₁-C₄)Alkoxy-carbonyl substituiert ist,
R⁵ Wasserstoff, (C₁-C₄)Alkyl, (C₁-C₄)Haloalkyl oder (C₃-C₇)Cycloalkyl,
A-Z eine divalente Brücke der Formel
-CH₂CH₂-, -CH₂CH₂CH₂-, -CH₂CH₂CH₂CH₂-,
-S-CH₂CH₂S-, -S-CH₂CH₂-O-, -O-CH₂CH₂-O-,
-CH₂CH₂CH₂-O-, -CH₂CH₂CH₂-S-,
(X')ₘ m Substituenten X' und dabei X' jeweils unabhängig voneinander Halogen, (C₁-C₄)Alkyl oder (C₁-C₄)Alkoxy,
(X)ₙ n Substituenten X und dabei X jeweils unabhängig voneinander Halogen, Hydroxy, (C₁-C₄)Alkyl oder (C₁-C₄)Alkoxy
B¹ eine direkte Bindung oder eine divalente Gruppe der Formel -O-, -S- oder -O-CO-,
Y¹ H, (C₁-C₄)Alkyl, (C₃-C₆)Cycloalkyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, Mono- und Dialkylamino substituiert ist,
bedeuten.

5. Verbindungen der Formel (I) und deren Salze nach einem der Ansprüche 1 bis 4, **dadurch gekennzeichnet, daß**
R¹ (C₁-C₄)Alkyl, (C₁-C₄)Haloalkyl oder [(C₃-C₆)Cycloalkyl]-methyl,
R² und R³ unabhängig voneinander Wasserstoff, Formyl oder (C₁-C₄)Alkyl oder
R² und R³ gemeinsam mit dem Stickstoffatom der Gruppe NR²R³ einen heterocyclischen Rest mit 4 bis 6 Ringatomen und 1 bis 2 Heteroringatomen, wobei neben dem N-Atom das gegebenenfalls weitere Heteroringatom aus der Gruppe N und O ausgewählt ist,
R⁴ Wasserstoff oder (C₁-C₄)Alkyl,
R⁵ H oder (C₁-C₄)Alkyl,
A-Z eine divalente Brücke der Formel
-CH₂CH₂-, -CH₂CH₂CH₂-, -CH₂CH₂CH₂CH₂-,
-S-CH₂CH₂S, -O-CH₂CH₂-O-,
-CH₂CH₂CH₂-O-, -CH₂CH₂CH₂-S-,
(X')ₘ m Substituenten X' und dabei X' jeweils unabhängig voneinander Halogen oder (C₁-C₄)Alkyl,
(X)ₙ n Substituenten X und dabei X jeweils unabhängig voneinander Halogen, Hydroxy, (C₁-C₄)Alkyl oder (C₁-C₄)Alkoxy
bedeuten.

6. Verfahren zur Herstellung der Verbindungen der allgemeinen Formel (I) oder deren Salze wie sie in Anspruch 1 definiert sind, **dadurch gekennzeichnet, daß** man
a) eine Verbindung der Formel (II),
R¹ - Fu (II)
worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet,
mit einem Biguanidid der Formel (III) oder einem Säureadditionssalz hiervon 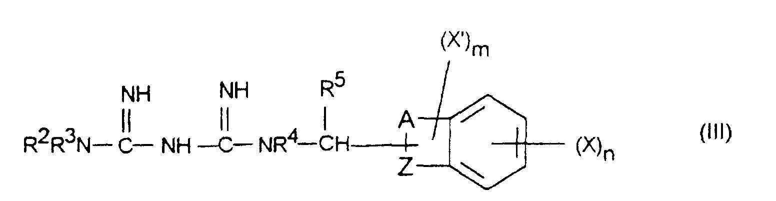 umsetzt oder
b) eine Verbindung der Formel (IV), 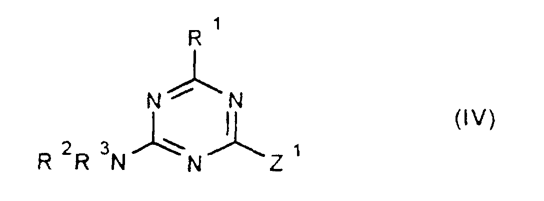 worin Z¹ einen austauschfähigen Rest oder eine Abgangsgruppe, z.B. Chlor, Trichlormethyl, (C₁-C₄)Alkylsulfonyl und unsubstituiertes oder substituiertes Phenyl-(C₁-C₄)alkylsulfonyl oder (C₁-C₄)Alkylphenylsulfonyl, bedeutet, mit einem geeigneten Amin der Formel (V) oder einem Säureadditionssalz hiervon 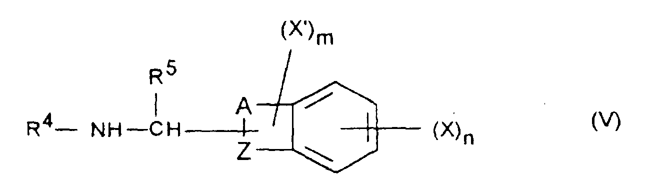 umsetzt,
wobei in den Formeln (II), (III), (IV) und (V) die Reste R¹, R², R³, R⁴, R⁵, A, X', X, m und n wie in Formel (I) definiert sind.

7. Herbizides oder pflanzenwachstumsregulierende Mittel, **dadurch gekennzeichnet, daß** es mindestens eine Verbindung der Formel (I) oder deren Salz nach einem der Ansprüche 1 bis 5 und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 5 auf die Pflanzen, Pflanzensamen oder die Anbaufläche appliziert.

9. Verwendung von Verbindungen der Formel (I) oder deren Salze nach einem der Ansprüche 1 bis 5 als Herbizide und Pflanzenwachstumsregulatoren.

## Claims

1. A compound of the formula (I) or a salt thereof 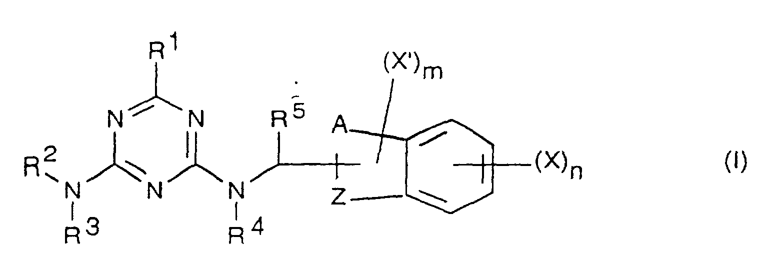 in which
R¹ is (C₁-C₆)alkyl
which is unsubstituted or substituted by one or more radicals selected from the group consisting of halogen, cyano, nitro, thiocyanato, hydroxyl, (C₁-C₄)alkoxy, (C₁-C₄)alkylthio, (C₁-C₄)alkylsulfinyl, (C₁-C₄)alkylsulfonyl, (C₂-C₄)alkenyl, (C₂-C₄)alkynyl, phenyl which is unsubstituted or substituted, (C₃-C₉)cycloalkyl which is unsubstituted or substituted, and heterocyclyl having 3 to 6 ring atoms and 1 to 3 hetero ring atoms selected from the group consisting of N, O and S, the ring being unsubstituted or substituted, or
a carbocyclic or heterocyclic radical having 3 to 6 ring atoms which is unsubstituted or substituted by one or more radicals selected from the group consisting of halogen, nitro, cyano, thiocyanato and a radical of the formula B¹-Y¹, where B¹ and Y¹ are as defined further below, it also being possible for pairs of substituents to form a carbocyclic or heterocyclic ring having 3 to 6 ring atoms which is unsubstituted or substituted,
R² and R³ are in each case independently of one another hydrogen, amino or alkylamino or dialkylamino, each of which has 1 to 6 carbon atoms in the alkyl radical, an acyclic or cyclic hydrocarbon radical or hydrocarbon-oxy radical having in each case 1 to 10 carbon atoms, or a heterocyclyl radical, heterocyclyloxy radical or heterocyclylamino radical having in each case 3 to 6 ring atoms and 1 to 3 hetero ring atoms selected from the group consisting of N, O and S, each of the five last-mentioned radicals being unsubstituted or substituted, or an acyl radical, or
R² and R³ together with the nitrogen atom of the group NR²R³ are a heterocyclic radical having 3 to 6 ring atoms and 1 to 4 hetero ring atoms, where, besides the nitrogen atom, the optional further hetero ring atoms are selected from the group consisting of N, O and S and the radical is unsubstituted or substituted,
R⁴ is hydrogen, amino, alkylamino or dialkylamino, each of which has 1 to 6 carbon atoms in the alkyl radical, an acyclic or cyclic hydrocarbon radical or hydrocarbon-oxy radical having in each case 1 to 10 carbon atoms, or a heterocyclyl radical, heterocyclyloxy radical or heterocyclylamino radical having in each case 3 to 6 ring atoms and 1 to 3 hetero ring atoms selected from the group consisting of N, O and S, each of the five last-mentioned radicals being unsubstituted or substituted, or an acyl radical,
R⁵ is hydrogen, nitro, cyano, thiocyanato or a radical of the formula -B²-Y², where B² and Y² are as defined further below,
A-Z is a divalent bridge of the formula
-(CH₂)ₐ-,
where a is the integer 2, 3 or 4,
-W¹-(CH₂)_{b}-W²-,
where b is the integer 1 or 2 and W¹, W² independently of one another are O or S,
-CH₂CH₂CH₂-W³-,
where W³ is O or S,
(X')ₘ denotes m substituents X' where the X' in each case independently of one another are halogen, (C₁-C₆)alkyl, (C₂-C₆)alkenyl, (C₂-C₆)alkynyl, (C₁-C₆)alkoxy, (C₂-C₆)alkenyloxy, (C₂-C₆)alkynyloxy, [(C₁-C₄)alkyl]carbonyl, [(C₁-C₄)alkoxy]carbonyl or [(C₁-C₄)alkylthio]-carbonyl, the hydrocarbon- containing moieties in the last-mentioned 9 radicals being unsubstituted or substituted, or the oxo group,
(X)ₙ denotes n substituents X where the X in each case independently of one another are halogen, nitro, cyano, (C₁-C₆)alkyl, (C₂-C₆)alkenyl, (C₂-C₆)alkynyl, (C₁-C₆)alkoxy, (C₂-C₆)alkenyloxy, (C₂-C₆)alkynyloxy, [(C₁-C₄)alkyl]carbonyl, [(C₁-C₄)alkoxy]carbonyl or [(C₁-C₄)alkylthio]carbonyl, where the hydrocarbon- containing moities in the last-mentioned 9 radicals are unsubstituted or substituted, or a radical of the formula -B³-Y³, where B³ and Y³ are as defined below,
or two adjacent radicals X together are a fused cycle having 4 to 6 ring atoms which is carbocyclic or contains hetero ring atoms from the group consisting of O, S and N and which is unsubstituted or substituted by one or more radicals selected from the group consisting of halogen, (C₁-C₄)alkyl and oxo,
B¹,B²,B³ in each case independently of one another are a direct bond or a divalent group of the formula -O-, -S(O)ₚ-, -S(O)ₚ-O-, -O-S(O)ₚ-, -CO-, -O-CO-, -CO-O-, -NR'-, -O-NR'-, -NR'-O-, -NR'-CO- or -CO-NR'-, where p = 0, 1 or 2 and R' is hydrogen, alkyl having 1 to 6 carbon atoms, phenyl, benzyl, cycloalkyl having 3 to 6 carbon atoms or alkanoyl having 1 to 6 carbon atoms,
Y¹,Y² in each case independently of one another are H or an acyclic hydrocarbon radical or a cyclic hydrocarbon radical having 3 to 8 carbon atoms, or a heterocyclic radical having 3 to 9 ring atoms and 1 to 3 hetero ring atoms selected from the group consisting of N, O and S, each of the three last-mentioned radicals being unsubstituted or substituted,
Y³ is an aromatic, saturated or partially saturated carbocyclic or heterocyclic radical, the cyclic radical being substituted or unsubstituted,
m is an integer from zero up to the number of the hydrogen atoms in the skeleton of the divalent bridge,
n is 0, 1, 2, 3 or 4.

2. A compound of the formula (I) or a salt thereof as claimed in claim 1, wherein
R¹ is (C₁-C₄)alkyl,
which is unsubstituted or substituted by one or more radicals selected from the group consisting of halogen, (C₁-C₄)alkoxy, (C₁-C₄)alkylthio, (C₁-C₄)alkylsulfonyl, phenyl which is unsubstituted or substituted by one or more radicals selected from the group consisting of halogen, (C₁-C₄)alkyl and (C₁-C₄)haloalkyl, (C₁-C₄)alkoxy, (C₁-C₄)haloalkoxy, (C₁-C₄)alkylthio, amino, mono- and di[(C₁-C₄)alkyl]amino, (C₁-C₄)alkanoylamino, benzoylamino, nitro, cyano, [(C₁-C₄)alkyl]carbonyl, formyl, carbamoyl, mono- and di- [(C₁-C₄)alkyl]aminocarbonyl and (C₁-C₄)alkylsulfonyl, and (C₃-C₉)cycloalkyl which is unsubstituted or substituted by one or more radicals (C₁-C₄)alkyl, and heterocyclyl having 3 to 6 ring atoms and 1 to 3 hetero ring atoms selected from the group consisting of N, O and S, the ring being unsubstituted or substituted by one or more radicals selected from the group consisting of halogen, (C₁-C₄)alkyl and oxo,
or
(C₃-C₇)cycloalkyl, (C₃-C₇)cycloalkenyl or a heterocyclic radical having 3 to 6 ring atoms and 1 to 3 hetero ring atoms selected from the group consisting of N, O and S, the cycloalkyl radical or the heterocyclic radical in each case being unsubstituted or substituted by one or more radicals selected from the group consisting of halogen, nitro, cyano, thiocyanato and a radical of the formula B¹-Y¹, where B¹ and Y¹ are as defined further below, and/or has substituents which, in pairs, can form a fused benzene ring or a fused or spiro-linked ring selected from the group consisting of (C₃-C₇)cycloalkyl, (C₃-C₇)cycloalkenyl and a heterocyclic ring having 3 to 6 ring atoms which is unsubstituted or substituted by one or more radicals selected from the group consisting of halogen, (C₁-C₄)alkyl, (C₁-C₄)haloalkyl and oxo, or phenyl which is unsubstituted or substituted,
R² and R³ in each case independently of one another are hydrogen, amino or alkylamino or dialkylamino, each of which has 1 to 4 carbon atoms in the alkyl radical, an acyclic or cyclic hydrocarbon radical or hydrocarbon-oxy radical, each of which has 1 to 6 carbon atoms, or a heterocyclyl radical, heterocyclyloxy radical or heterocyclylamino radical, each of which has 3 to 6 ring atoms and 1 to 3 hetero ring atoms selected from the group consisting of N, O and S, where each of the five last-mentioned radicals is unsubstituted or substituted by one or more radicals selected from the group consisting of halogen, (C₁-C₄)alkoxy, (C₁-C₄)haloalkoxy, (C₁-C₄)alkylthio, (C₂-C₄)alkenyl, (C₂-C₄)alkynyl, (C₂-C₄)alkenyloxy, (C₂-C₄)alkynyloxy, hydroxyl, amino, acylamino, mono- and dialkylamino, nitro, carboxyl, cyano, azido, [(C₁-C₄)alkoxy]carbonyl, [(C₁-C₄)alkyl]carbonyl, formyl, carbamoyl, mono- and di [(C₁-C₄)alkyl]aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl,
or an acyl radical, or
R² and R³ together with the nitrogen atom of the group NR²R³ are a heterocyclic radical having 3 to 6 ring atoms and 1 to 2 hetero ring atoms where, besides the nitrogen atom, the optional other hetero ring atom is selected from the group consisting of N, O and S and the radical is unsubstituted or substituted by one or more radicals selected from the group consisting of halogen, (C₁-C₄)alkyl and oxo,
R⁴ is hydrogen, amino, alkylamino or dialkylamino, each of which has 1 to 6 carbon atoms in the alkyl radical, an acyclic or cyclic hydrocarbon radical or hydrocarbon-oxy radical, each of which has 1 to 6 carbon atoms, or a heterocyclyl radical, heterocyclyloxy radical or heterocyclylamino radical, each of which has 3 to 6 ring atoms and 1 to 3 hetero ring atoms selected from the group consisting of N, O and S, where each of the five last-mentioned radicals is unsubstituted or substituted by one or more radicals selected from the group consisting of halogen, (C₁-C₄)alkoxy, (C₁-C₄)haloalkoxy, (C₁-C₄)alkylthio, (C₂-C₄)alkenyl, (C₂-C₄)alkynyl, (C₂-C₄)alkenyloxy, (C₂-C₄)alkynyloxy, hydroxyl, amino, acylamino, mono- and dialkylamino, nitro, carboxyl, cyano, azido, [(C₁-C₄)alkoxy]carbonyl, [(C₁-C₄)alkyl]carbonyl, formyl, carbamoyl, mono- and di[(C₁-C₄)alkyl]aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl, or an acyl radical,
R⁵ is hydrogen, nitro, cyano, thiocyanato or a radical of the formula
-B²-Y², where B² and Y² are as defined further below,
A-Z is a divalent bridge of the formula
-(CH₂)ₐ- ,
where a is the integer 2, 3 or 4,
-W¹-(CH₂)_{b}-W²- ,
where b is the integer 1 or 2 and W¹, W² independently of one another are S or O,
-CH₂CH₂CH₂-W³- ,
where W³ is O or S,
(X')ₘ denotes m substituents X' where the X' in each case independently of one another are halogen, (C₁-C₆)alkyl, (C₂-C₆)alkenyl, (C₂-C₆)alkynyl, (C₁-C₆)alkoxy, (C₂-C₆)alkenyloxy, (C₂-C₆)alkynyloxy, [(C₁-C₄)alkyl]carbonyl, [(C₁-C₄)alkoxy]carbonyl or [(C₁-C₄)alkylthio)carbonyl, the hydrocarbon- containing moieties in the last-mentioned 9 radicals being unsubstituted or substituted by one or more radicals selected from the group consisting of halogen, (C₁-C₄)alkoxy and (C₁-C₄)alkylthio, or the oxo group,
(X)ₙ denotes n substituents X where the X in each case independently of one another are halogen, nitro, cyano, (C₁-C₆)alkyl, (C₁-C₆)alkoxy, (C₁-C₄)alkylcarbonyl, (C₁-C₄)alkyloxycarbonyl or (C₁-C₄)alkylthiocarbonyl, the last-mentioned five radicals being unsubstituted or substituted by halogen or (C₁-C₄)alkoxy, a radical of the formula -B³-Y³, where B³ and Y³ are as defined below, or two adjacent radicals X together are a fused cycle having 4 to 6 ring atoms which is carbocyclic or contains hetero ring atoms selected from the group consisting of O, S and N and which is unsubstituted or substituted by one or more radicals selected from the group consisting of halogen, (C₁-C₄)alkyl and oxo,
B¹,B² and B³ in each case independently of one another are a direct bond or a divalent group of the formula -O-, -S-, -CO-, -O-CO-, -CO-O-, -NR'-, -NR'-CO- or -CO-NR'-, where R' is H or (C₁-C₄)alkyl,
Y¹ and Y² in each case independently of one another are H or an acyclic hydrocarbon radical having 1 to 6 carbon atoms, a cyclic hydrocarbon radical having 3 to 6 carbon atoms or a heterocyclic radical having 3 to 9 ring atoms and 1 to 3 hetero ring atoms selected from the group consisting of N, O and S, each of the three last-mentioned radicals being unsubstituted or substituted by one or more radicals selected from the group consisting of halogen, (C₁-C₄)alkoxy, (C₁-C₄)haloalkoxy, (C₁-C₄)alkylthio, (C₂-C₄)alkenyl, (C₂-C₄)alkynyl, (C₂-C₄)alkenyloxy, (C₂-C₄)alkynyloxy, hydroxyl, amino, acylamino, mono- and dialkylamino, nitro, carboxyl, cyano, azido, [(C₁-C₄)alkoxy]carbonyl, [(C₁-C₄)alkyl]carbonyl, formyl, carbamoyl, mono- and di[(C₁-C₄)alkyl]aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl,
Y³ is (C₃-C₆)cycloalkyl which is unsubstituted or substituted by radicals selected from the group consisting of (C₁-C₄)alkyl and (C₁-C₄)haloalkyl, or phenyl which is unsubstituted or substituted by radicals selected from the group consisting of halogen, (C₁-C₄)alkyl, (C₁-C₄)haloalkyl and (C₁-C₄)alkoxy,
m is 0, 1 or 2, and
n is 0, 1, 2, 3 or 4.

3. A compound of the formula (I) or a salt thereof as claimed in claim 1 or 2, wherein
R¹ is (C₁-C₄)alkyl,
which is unsubstituted or substituted by one or more radicals selected from the group consisting of halogen, (C₁-C₄)alkoxy, (C₁-C₄)alkylthio and phenyl which is unsubstituted or substituted by one or more radicals selected from the group consisting of halogen, (C₁-C₄)alkyl and (C₁-C₄)haloalkyl, (C₁-C₄)alkoxy, (C₁-C₄)haloalkoxy, (C₁-C₄)alkylthio, nitro, cyano, [(C₁-C₂)alkyl]carbonyl, formyl, carbamoyl, mono- and di[(C₁-C₂)alkyl]aminocarbonyl, (C₁-C₄)alkylsulfonyl and (C₃-C₇) cycloalkyl which is unsubstituted or substituted by one or more radicals (C₁-C₄)alkyl,
or
(C₃-C₇)cycloalkyl or a heterocyclic radical having 3 to 6 ring atoms and 1 or 2 hetero ring atoms selected from the group consisting of N, O and S, the cycloalkyl radical or the heterocyclic radical in each case being unsubstituted or substituted by one or more radicals selected from the group consisting of halogen, nitro, cyano, thiocyanato and a radical of the formula B¹-Y¹, B¹ and Y¹ being as defined further below, and/or having substituents which, in pairs, can form a spiro-linked ring selected from the (C₃-C₇)cycloalkyl group which is unsubstituted or substituted by one or more radicals selected from the group consisting of halogen, (C₁-C₄)alkyl, (C₁-C₄)haloalkyl and oxo,
R² and R³ independently of one another are hydrogen, amino, formyl, (C₁-C₄)alkyl, cyano (C₁-C₄)alkyl, (C₁-C₄)alkylamino, di[(C₁-C₄)alkyl]-amino, halo(C₁-C₄)alkyl, hydroxy(C₁-C₄)alkyl, (C₁-C₄)alkoxy-(C₁-C₄)alkyl, halo(C₁-C₄)alkoxy-(C₁-C₄)alkyl, (C₂-C₆)alkenyl, halo(C₂-C₆)alkenyl, (C₂-C₆)alkynyl, halo-(C₂-C₆)alkynyl, (C₁-C₄)alkylamino-(C₁-C₄)alkyl, di[(C₁-C₄)alkyl]-amino-(C₁-C₄)alkyl, (C₃-C₉)cycloalkylamino-(C₁-C₄)alkyl, (C₃-C₉)cycloalkyl, (C₃-C₉)heterocyclyl-(C₁-C₄)alkyl, the cyclic groups in the last-mentioned 3 radicals being unsubstituted or substituted by one or more radicals selected from the group consisting of (C₁-C₄)alkyl, halogen and cyano, or phenyl, phenoxy, phenylcarbonyl, phenoxycarbonyl, phenylcarbonyl-(C₁-C₄)alkyl, (C₁-C₄)alkoxycarbonyl-(C₁-C₄)alkyl, (C₁-C₄)alkylaminocarbonyl-(C₁-C₄)alkyl, (C₁-C₄)alkylcarbonyl, (C₁-C₄)alkoxycarbonyl, aminocarbonyl, (C₁-C₄)alkylaminocarbonyl, phenoxy-(C₁-C₄)alkyl, phenyl-(C₁-C₄)alkyl, heterocyclyl, heterocyclylamino, heterocyclyloxy, heterocyclylthio, or one of the last-mentioned 17 radicals which is substituted in the acyclic moiety or in the cyclic moiety by one or more radicals selected from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkylcarbonyl, (C₁-C₄)alkoxycarbonyl and (C₁-C₄)alkoxy, heterocyclyl in the radicals in each case containing 3 to 9 ring atoms and 1 to 3 hetero ring atoms selected from the group consisting of N, O and S, or
R² and R³ together with the nitrogen atom of the group NR²R³ are a heterocyclic radical having 3 to 6 ring atoms and 1 to 2 hetero ring atoms, where, besides the nitrogen atom, the optional further hetero ring atom is selected from the group consisting of N, O and S and the radical is unsubstituted or substituted by one or more radicals selected from the group consisting of halogen, (C₁-C₄)alkyl and oxo,
R⁴ is hydrogen, amino, formyl, (C₁-C₄)alkyl, cyano-(C₁-C₄)alkyl, (C₁-C₄)alkylamino, di[(C₁-C₄)alkyl]amino, halo-(C₁-C₄)alkyl, hydroxy- (C₁-C₄)alkyl, (C₁-C₄)alkoxy-(C₁-C₄)alkyl, halo(C₁-C₄alkoxy-(C₁-C₄)alkyl, (C₂-C₆)alkenyl, halo-(C₂-C₆)alkenyl, (C₂-C₆)alkynyl, halo-(C₂-C₆)alkynyl, (C₁-C₄)alkylamino-(C₁-C₄)alkyl, di[(C₁-C₄)alkyl]amino-(C₁-C₄)alkyl, (C₃-C₉)cycloalkylamino-(C₁-C₄)alkyl, (C₃-C₉)cycloalkyl, (C₃-C₉)heterocyclyl-(C₁-C₄)alkyl having 3 to 9 ring members, the cyclic groups in the last-mentioned 3 radicals being unsubstituted or substituted by one or more radicals selected from the group consisting of (C₁-C₄)alkyl, halogen and cyano, or
phenyl, phenoxy, phenylcarbonyl, phenoxycarbonyl, phenylcarbonyl-(C₁-C₄)alkyl, (C₁-C₄)alkoxycarbonyl-(C₁-C₄)alkyl, (C₁-C₄)alkylaminocarbonyl-(C₁-C₄)alkyl, (C₁-C₄)alkylcarbonyl, (C₁-C₄)alkoxycarbonyl, aminocarbonyl, (C₁-C₄)alkylaminocarbonyl, phenoxy-(C₁-C₄)alkyl, phenyl-(C₁-C₄)alkyl, heterocyclyl, heterocyclylamino, heterocyclyloxy, heterocyclylthio, or one of the last-mentioned 17 radicals which is substituted in the acyclic moiety or in the cyclic moiety by one or more radicals selected from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkylcarbonyl, (C₁-C₄)alkoxycarbonyl, (C₁-C₄)alkoxy, where heterocyclyl in the radicals contains in each case 3 to 9 ring atoms and 1 to 3 hetero ring atoms selected from the group consisting of N, O and S,
R⁵ is hydrogen, (C₁-C₄)alkyl, (C₁-C₄)haloalkyl or (C₃-C₇)cycloalkyl,
A-Z is a divalent bridge of the formula
-CH₂CH₂-, -CH₂CH₂CH₂-, -CH₂CH₂CH₂CH₂-,
-S-CH₂S-, -S-CH₂-O-, -O-CH₂-O-,
-S-CH₂CH₂S-, -S-CH₂CH₂-O-, -O-CH₂CH₂-O-,
-CH₂CH₂CH₂-O-, -CH₂CH₂CH₂-S-,
(X')ₘ denotes m substituents X' where the X' in each case independently of one another are halogen, (C₁-C₄)alkyl, (C₂-C₄)alkenyl, (C₂-C₄)alkynyl, (C₁-C₄)alkoxy, (C₂-C₄)alkenyloxy, (C₂-C₄)alkynyloxy, [(C₁-C₄)alkyl]carbonyl, [(C₁-C₄)alkoxy]carbonyl or [(C₁-C₄)alkylthio]-carbonyl, the hydrocarbon-b containing moieties in the last-mentioned 9 radicals being unsubstituted or substituted by one or more radicals selected from the halogen group, or are the oxo group,
(X)ₙ denotes n substituents X where the X in each case independently of one another are halogen, hydroxyl, amino, nitro, formyl, carboxyl, cyano, thiocyanato, (C₁-C₄)alkyl, cyano(C₁-C₄)alkyl, (C₁-C₄)alkoxy, (C₁-C₄)alkylamino, di[(C₁-C₄)alkyl]amino, halo(C₁-C₄)alkyl, hydroxy- (C₁-C₄)alkyl, (C₁-C₄)alkoxy- (C₁-C₄)alkyl, halo(C₁-C₄)alkoxy-(C₁-C₄)alkyl, (C₁-C₄)alkylthio, halo(C₁-C₄)alkylthio, (C₂-C₆)alkenyl, halo(C₂-C₆)alkenyl, (C₂-C₆)alkynyl, halo(C₂-C₆)alkynyl, (C₁-C₄)alkylamino-(C₁-C₄)alkyl, di[(C₁-C₄)alkyl)amino-(C₁-C₄)alkyl, (C₃-C₉)cycloalkylamino-(C₁-C₄)alkyl, (C₃-C₉)cycloalkyl, heterocyclyl-(C₁-C₄)alkyl having 3 to 9 ring members, the cyclic groups in the last-mentioned 3 radicals being unsubstituted or substituted by one or more radicals selected from the group consisting of (C₁-C₄)alkyl, halogen and cyano, or
phenyl, phenoxy, phenylcarbonyl, phenoxycarbonyl, phenylcarbonyl-(C₁-C₄)alkyl, (C₁-C₄)alkoxycarbonyl- (C₁-C₄)alkyl, (C₁-C₄)alkylaminocarbonyl-(C₁-C₄)alkyl, (C₁-C₄)alkylcarbonyl, (C₁-C₄)alkoxycarbonyl, aminocarbonyl, (C₁-C₄)alkylaminocarbonyl, phenoxy- (C₁-C₄)alkyl, phenyl-(C₁-C₄)alkyl, heterocyclyl, heterocyclylamino, heterocyclyloxy, heterocyclylthio or one of the last-mentioned 17 radicals which is substituted in the acyclic moiety or in the cyclic moiety by one or more radicals selected from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkylcarbonyl, (C₁-C₄)alkoxycarbonyl and (C₁-C₄)alkoxy, where heterocyclyl in the radicals contains in each case 3 to 9 ring atoms and 1 to 3 hetero ring atoms selected from the group consisting of N, O and S, or
two adjacent radicals X together are a fused cycle having 4 to 6 ring atoms which is carbocyclic or contains hetero ring atoms selected from the group consisting of O, S and N and which is unsubstituted or substituted by one or more radicals selected from the group consisting of halogen, (C₁-C₄)alkyl and oxo,
B¹ is a direct bond or a divalent group of the formula -O-, -S-, -CO-, -O-CO-, -CO-O-, -NR'-, -NR'-CO- or -CO-NR'-, where R' is H or (C₁-C₄)alkyl,
Y¹ is H or an acyclic hydrocarbon radical having 1 to 4 carbon atoms, a cyclic hydrocarbon radical having 3 to 6 carbon atoms or a heterocyclic radical having 3 to 6 ring atoms and 1 to 3 hetero ring atoms selected from the group consisting of N, O and S, where each of the three last-mentioned radicals is unsubstituted or substituted by one or more radicals selected from the group consisting of halogen, (C₁-C₄)alkoxy, (C₁-C₄)haloalkoxy, (C₁-C₄)alkylthio, hydroxyl, amino, mono- and dialkylamino, [(C₁-C₄)alkoxy]carbonyl, [(C₁-C₄)alkyl]carbonyl, formyl, carbamoyl, mono- and di[(C₁-C₄)alkyl]aminocarbonyl, (C₁-C₄)alkylsulfinyl, (C₁-C₄)haloalkylsulfinyl, (C₁-C₄)alkylsulfonyl, (C₁-C₄)haloalkylsulfonyl and, in the case of cyclic radicals, also (C₁-C₄)alkyl and (C₁-C₄)haloalkyl.

4. A compound of the formula (I) or a salt thereof as claimed in any of claims 1 to 3, wherein
R¹ is (C₁-C₄)alkyl, (C₁-C₄)haloalkyl, benzyl, [(C₃-C₆)cycloalkyl]-(C₁-C₂)-alkyl, (C₃-C₆)cycloalkyl or a heterocyclic radical having 3 to 6 ring atoms and 1 or 2 hetero ring atoms selected from the group consisting of N and 0, preferably an oxygen atom as hetero ring atom, the cycloalkyl radical or the heterocyclic radical being in each case unsubstituted or substituted by one or more radicals selected from the group consisting of halogen, nitro, cyano, thiocyanato and a radical of the formula B¹-Y¹, where B¹ and Y¹ are as defined further below, and/or having substituents which, in pairs, can form a spiro-linked ring selected from the (C₃-C₇)cycloalkyl group which is unsubstituted or substituted by one or more radicals selected from the group consisting of halogen, (C₁-C₄)alkyl, (C₁-C₄)haloalkyl and oxo,
R² and R³ independently of one another are hydrogen, amino, formyl, (C₁-C₄)alkyl, (C₁-C₄)alkoxy-(C₁-C₄)alkyl, (C₃-C₆)alkenyl, (C₃-C₆)alkynyl, (C₁-C₄)alkylamino-(C₁-C₄)alkyl, di[(C₁-C₄)alkyl]-amino-(C₁-C₄)alkyl or phenyl, phenyl-(C₁-C₄)alkyl, phenylcarbonyl or phenoxycarbonyl or one of the last-mentioned four radicals which is up to trisubstituted in the phenyl moiety by radicals selected from the group consisting of halogen, (C₁-C₄)alkyl, (C₁-C₄)alkoxy and (C₁-C₄)alkoxycarbonyl, or
R² and R³ together with the nitrogen atom of the group NR²R³ are a heterocyclic radical having 3 to 6 ring atoms and 1 to 2 hetero ring atoms, where, besides the nitrogen atom, the optional further hetero ring atom is selected from the group consisting of N and 0 and the radical is unsubstituted or substituted by one or more radicals selected from the group consisting of halogen, (C₁-C₄)alkyl and oxo,
R⁴ is hydrogen, amino, formyl, (C₁-C₄)alkyl, di[(C₁-C₄)alkyl]amino, (C₁-C₄)alkoxy-(C₁-C₄)alkyl, (C₃-C₆)alkenyl, (C₃-C₆)alkynyl, (C₁-C₄)dialkylamino-(C₁-C₄)alkyl, phenyl, phenoxy- (C₁-C₄)alkyl, phenyl-(C₁-C₄)alkyl, phenoxycarbonyl, phenylaminocarbonyl or one of the last-mentioned five radicals which is monosubstituted to trisubstituted in the phenyl moiety by radicals selected from the group consisting of halogen, (C₁-C₄)alkyl, (C₁-C₄)alkoxy and (C₁-C₄)alkoxycarbonyl,
R⁵ is hydrogen, (C₁-C₄)alkyl, (C₁-C₄)haloalkyl or (C₃-C7) cycloalkyl,
A-Z is a divalent bridge of the formula
-CH₂CH₂-, -CH₂CH₂CH₂-, -CH₂CH₂CH₂CH₂-,
-S-CH₂CH₂S-, -S-CH₂CH₂-O-, -O-CH₂CH₂-O-,
-CH₂CH₂CH₂-O-, -CH₂CH₂CH₂-S-,
(X')ₘ denotes m substituents X' where the X' in each case independently of one another are halogen, (C₁-C₄)alkyl or (C₁-C₄)alkoxy,
(X)ₙ denotes n substituents X where the X in each case independently of one another are halogen, hydroxyl, (C₁-C₄)alkyl or (C₁-C₄)alkoxy,
B¹ is a direct bond or a divalent group of the formula -O-, -S- or -O-CO-,
Y¹ is H, (C₁-C₄)alkyl , (C₃-C₆)cycloalkyl or phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, mono- and dialkylamino.

5. A compound of the formula (I) or a salt thereof as claimed in any of claims 1 to 4, wherein
R¹ is (C₁-C₄)alkyl, (C₁-C₄)haloalkyl or [(C₃-C₆)cycloalkyl]methyl,
R² and R³ independently of one another are hydrogen, formyl or (C₁-C₄)alkyl or
R² and R³ together with the nitrogen atom of the group NR²R³ are a heterocyclic radical having 4 to 6 ring atoms and 1 to 2 hetero ring atoms, where, besides the nitrogen atom, the optional further hetero ring atom is selected from the group consisting of N and O,
R⁴ is hydrogen or (C₁-C₄)alkyl,
R⁵ is H or (C₁-C₄)alkyl,
A-Z is a divalent bridge of the formula
-CH₂CH₂-, -CH₂CH₂CH₂-, -CH₂CH₂CH₂CH₂-,
-S-CH₂CH₂S, -O-CH₂CH₂-O-,
-CH₂CH₂CH₂-O-, -CH₂CH₂CH₂-S-,
(X')ₘ denotes m substituents X' where the X' in each case independently of one another are halogen or (C₁-C₄)alkyl, and
(X)ₙ denotes n substituents X where the X in each case independently of one another are halogen, hydroxyl, (C₁-C₄)alkyl or (C₁-C₄)alkoxy.

6. A process for the preparation of a compound of the formula (I) or a salt thereof as they are defined in claim 1, which comprises
a) reacting a compound of the formula (II)
R¹ - Fu (II)
where Fu is a functional group selected from the group consisting of carboxylic ester, carboxylic orthoester, carboxylic acid chloride, carboxamide, carboxylic anhydride and trichloromethyl,
with a biguanidide of the formula (III) or an acid addition salt hereof 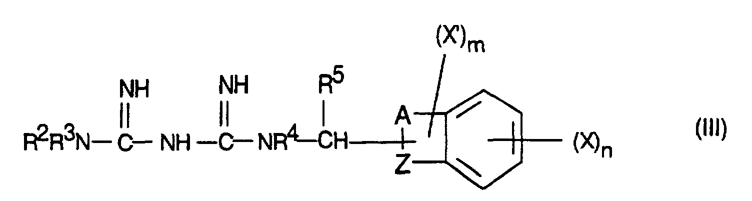 or
b) reacting a compound of the formula (IV) 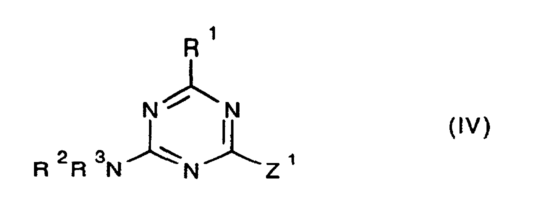 where Z¹ is an exchangeable radical or a leaving group, for example chlorine, trichloromethyl, (C₁-C₄)alkylsulfonyl and unsubstituted or substituted phenyl-(C₁-C₄)alkylsulfonyl or (C₁-C₄)alkylphenylsulfonyl, with a suitable amine of the formula (V) or an acid addition salt hereof 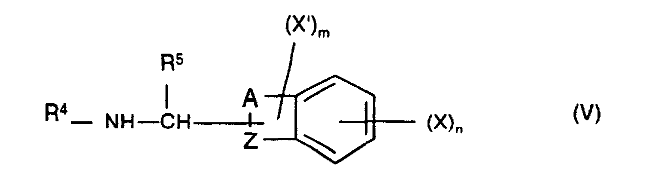
the radicals R¹, R², R³, R⁴, R⁵, A, X', X, m and n in formulae (II), (III), (IV) and (V) being as defined in formula (I).

7. A herbicidal or plant growth-regulating composition, which comprises at least one compound of the formula (I) or a salt thereof as claimed in any of claims 1 to 5 and formulation auxiliaries conventionally used in crop protection.

8. A method of controlling harmful plants or of regulating the growth of plants, which comprises applying an effective amount of at least one compound of the formula (I) or a salt thereof as claimed in any of claims 1 to 5 to the plants, the seeds of the plants or the area under cultivation.

9. The use of a compound of the formula (I) or a salt thereof as claimed in any of claims 1 to 5 as herbicides and plant growth regulators.

## Revendications

1. Composés de formule (I) et leurs sels, 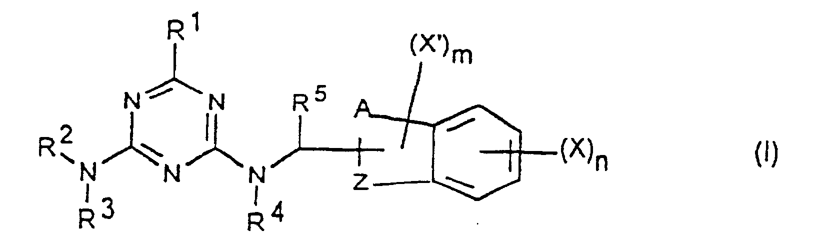 où
R¹ représente un groupe alkyle en C₁-C₆,
qui est non substitué ou substitué par un ou plusieurs restes pris dans le groupe comprenant un atome d'halogène, un groupe cyano, nitro, thiocyanato, hydroxy, alkoxy en C₁-C₄, (alkyl en C₁-C₄)thio, (alkyl en C₁-C₄)-sulfinyle, (alkyl en C₁-C₄)sulfonyle, alcényle en C₂-C₄, alcynyle en C₂-C₄, phényle, qui est non substitué ou substitué, et un groupe cycloalkyle en C₃-C₉ qui est non substitué ou substitué, et un groupe hétérocyclyle présentant 3 à 6 atomes nucléaires et 1 à 3 hétéroatomes nucléaires pris parmi les atomes de N, O et S, le cycle étant non substitué ou substitué, ou
un reste carbocyclique ou hétérocyclique présentant 3 à 6 atomes nucléaires, qui est non substitué ou substitué par un ou plusieurs restes pris dans le groupe comprenant un atome d'halogène, un groupe nitro, cyano, thiocyanato et un reste de formule B¹-Y¹, B¹ et Y¹ sont définis comme ci-dessous, les substituants pouvant aussi former par paires un cycle carbocyclique ou hétérocyclique présentant 3 à 6 atomes nucléaires qui est non substitué ou substitué,
R² et R³ représentent, indépendamment l'un de l'autre, un atome d'hydrogène, un groupe amino ou alkylamino ou dialkylamino présentant chacun 1 à 6 atomes de carbone dans le reste alkyle, un reste hydrocarboné ou un reste hydrocarboxy acyclique ou cyclique présentant chacun 1 à 10 atomes de carbone, ou un reste hétérocyclyle, un reste hétérocyclyloxy ou reste hétérocyclylamino présentant chacun 3 à 6 atomes nucléaires et 1 à 3 hétéroatomes nucléaires pris parmi les atomes de N, O et S, chacun des cinq derniers restes cités pouvant être non substitué ou substitué, ou représente un reste acyle ou
R² et R³ forment conjointement avec l'atome d'azote du groupe NR²R³ un reste hétérocyclique présentant 3 à 6 atomes nucléaires et 1 à 4 hétéroatomes nucléaires, les autres hétéroatomes nucléaires éventuels au côté de l'atome de N sont pris parmi les atomes de N, O et S et le reste est non substitué ou substitué,
R⁴ représente un atome d'hydrogène, un groupe amino, alkylamino ou dialkylamino présentant chacun 1 à 6 atomes de carbone dans le reste alkyle, un reste hydrocarboné ou un reste hydrocarboxy acyclique ou cyclique présentant chacun 1 à 10 atomes de carbone, ou un reste hétérocyclyle, un reste hétérocyclyloxy ou reste hétérocyclylamino présentant chacun 3 à 6 atomes nucléaires et 1 à 3 hétéroatomes nucléaires pris parmi les atomes de N, O et S, chacun des cinq derniers restes cités pouvant être non substitué ou substitué, ou représente un reste acyle ou
R⁵ représente un atome d'hydrogène, un groupe nitro, cyano, thiocyanato ou un reste de formule -B²-Y², B² et Y² sont définis comme ci-dessous,
A-Z représente une liaison bivalente de formule
-(CH₂)ₐ-
où a est un entier 2, 3 ou 4,
-W¹-(CH₂)_{b}-W²-,
où b est un entier de 1 ou 2, et W¹, W² représentent, indépendamment l'un de l'autre, un atome de O ou S,
-CH₂CH₂CH₂-W³-
où W³ représente un atome de O ou S,
(X')ₘ représente m substituants X' et dans ce contexte, chaque X' représente indépendamment un atome d'halogène, en groupe alkyle en C₁-C₆, alcényle en C₂-C₆, alcynyle en C₂-C₆, alkoxy en C₁-C₆, alcényloxy en C₂-C₆, alcynyloxy en C₂-C₆, (alkyl en C₁-C₄)carbonyle, (alkoxy en C₁-C₄)-carbonyle ou [(alkyl en C₁-C₄)thio]carbonyle, les fragments contenant des restes hydrocarbonés dans les 9 derniers restes cités sont non substitués ou substitués, ou représente le groupe oxo,
(X)ₙ représente n substituants X et dans ce contexte, chaque X représente indépendamment un atome d'halogène, des groupe groupe nitro, cyano, alkyle en C₁-C₆, alcényle en C₂-C₆, alcynyle en C₂-C₆, alkoxy en C₁-C₆, alcényloxy en C₂-C₆, alcynyloxy en C₂-C₆, (alkyl en C₁-C₄)carbonyle, (alkoxy en C₁-C₄)-carbonyle ou [(alkyl en C₁-C₄)-thio]carbonyle, les fragments contenant des restes hydrocarbonés dans les 9 derniers restes cités sont non substitués ou substitués, ou un reste de formule -B³-Y³, B³ et Y³ étant définis ci-dessous,
ou deux restes X adjacents forment conjointement un cycle condensé présentant 4 à 6 atomes nucléaires, qui est carbocyclique ou renferme des hétéroatomes nucléaires pris parmi les atomes de O, S et N, et qui est non substitué ou substitué par un ou plusieurs restes pris dans le groupe comprenant un atome d'halogène, un groupe alkyle en C₁-C₄ et un groupe oxo,
B¹, B², B³ représentent chacun, indépendamment les uns des autres, une liaison directe ou un groupe bivalent de formule -O-, -S(O)ₚ-, -S(O)ₚ-O-, -O-S(O)ₚ-, -CO-, -O-CO-, -CO-O-, -NR'-, -O-NR'-, -NR'-O-, -NR'-CO- ou -CO-NR'-, p = 0, 1 ou 2 et R' représente un atome d'hydrogène, un groupe alkyle présentant 1 à 6 atomes de carbone, phényle, benzyle, cycloalkyle présentant 3 à 6 atomes de carbone ou alcanoyle présentant 1 à 6 atomes de carbone,
Y¹, Y² représentent chacun, indépendamment l'un de l'autre, un atome d'hydrogène ou un reste hydrocarboné acyclique ou un reste hydrocarboné cyclique présentant 3 à 8 atomes de carbone ou un reste hétérocyclique présentant 3 à 9 atomes nucléaires et 1 à 3 hétéroatomes nucléaires pris parmi les atomes N, O et S, chacun des trois derniers restes cités étant non substitué ou substitué,
Y³ représente un reste carbocyclique ou hétérocyclique aromatique, saturé ou partiellement saturé, le reste cyclique étant substitué ou non substitué,
m est un entier de 0 jusqu'au nombre d'atomes d'hydrogène dans le corps fondamental du pont bivalent,
n vaut 0, 1, 2, 3 ou 4.

2. Composés de formule (I) ou leurs sels selon la revendication 1, **caractérisés en ce que**
R¹ représente un groupe alkyle en C₁-C₄,
qui est non substitué ou substitué par un ou plusieurs restes pris dans le groupe comprenant un atome d'halogène, un groupe alkoxy en C₁-C₄, (alkyl en C₁-C₄)thio, (alkyl en C₁-C₄)sulfonyle, phényle, qui est non substitué ou substitué par un ou plusieurs restes pris dans le groupe comprenant un atome d'halogène, un groupe alkyle en C₁-C₄ et haloalkyle en C₁-C₄, alkoxy en C₁-C₄, haloalkoxy en C₁-C₄, (alkyl en C₁-C₄)thio, amino, mono- et di(alkyl en C₁-C₄)amino, (alcanoyl en C₁-C₄)amino, benzoylamino, nitro, cyano, (alkyl en C₁-C₄)carbonyle, formyle, carbamoyle, mono- et di-(alkyl en C₁-C₄)aminocarbonyle et (alkyl en C₁-C₄)-sulfonyle, et cycloalkyle en C₃-C₉ qui est non substitué ou substitué par un ou plusieurs restes alkyle en C₁-C₄, et hétérocyclyle présentant 3 à 6 atomes nucléaires et 1 à 3 hétéroatomes nucléaires pris parmi les atomes de N, O et S, le cycle pouvant être non substitué ou substitué par un ou plusieurs restes pris dans le groupe comprenant un atome d'halogène, un groupe alkyle en C₁-C₄ et un groupe oxo, ou
un groupe cycloalkyle en C₃-C₇, cycloalcényle en C₃-C₇ ou un reste hétérocyclique présentant 3 à 6 atomes nucléaires et 1 à 3 hétéroatomes nucléaires pris parmi les atomes de N, O et S, le reste cycloalkyle ou le reste hétérocyclique étant chacun non substitué ou substitué par un ou plusieurs restes pris dans un groupe comprenant un atome d'halogène, un groupe nitro, cyano, thiocyanato et un reste de formule B¹-Y¹, B¹ et Y¹ sont définis comme ci-dessous, et/ou présente des substituants qui peuvent former par paires un cycle benzène condensé ou un cycle condensé ou relié sous forme spiro pris dans le groupe comprenant un groupe comprenant un groupe cycloalkyle en C₃-C₇, cycloalcényle en C₃-C₇ ou un reste hétérocyclique présentant 3 à 6 atomes nucléaires, qui est non substitué ou substitué par un ou plusieurs restes pris dans le groupe comprenant un atome d'halogène, un groupe alkyle en C₁-C₄, haloalkyle en C₁-C₄ et oxo, ou phényle, qui est non substitué ou substitué,
R² et R³ représentent chacun, indépendamment l'un de l'autre, un atome d'hydrogène, un groupe amino ou alkylamino ou dialkylamino présentant chacun 1 à 4 atomes de carbone dans le reste alkyle, ou un reste hydrocarboné ou un reste hydrocarboxy acyclique ou cyclique présentant chacun 1 à 6 atomes de carbone, ou un reste hétérocyclyle, un reste hétérocyclyloxy ou reste hétérocyclylamino présentant chacun 3 à 6 atomes nucléaires et 1 à 3 hétéroatomes nucléaires pris parmi les atomes de N, O et S, chacun des cinq derniers restes cités pouvant être non substitué ou substitué par un ou plusieurs restes pris dans le groupe comprenant un atome d'halogène, un groupe alkoxy en C₁-C₄, haloalkoxy en C₁-C₄, (alkyl en C₁-C₄)thio, alcényle en C₂-C₄, alcynyle en C₂-C₄, alcényloxy en C₂-C₄, alcynyloxy en C₂-C₄, hydroxy, amino, acylamino, mono- et dialkylamino, nitro, carboxy, cyano, azido, (alkoxy en C₁-C₄)carbonyle, (alkyl en C₁-C₄)carbonyle, formyle, carbamoyle, mono- et di-(alkyl en C₁-C₄)aminocarbonyle et (alkyl en C₁-C₄)sulfinyle, halo(alkyl en C₁-C₄)-sulfinyle, (alkyl en C₁-C₄)sulfonyle, halo(alkyl en C₁-C₄)sulfonyle, et dans le cas de restes cyclique, également par alkyle en C₁-C₄ et haloalkyle en C₁-C₄,
ou un reste acyle ou
R² et R³ forment conjointement avec l'atome d'azote du groupe NR²R³ un reste hétérocyclique présentant 3 à 6 atomes nucléaires et 1 ou 2 hétéroatomes nucléaires, présentant éventuellement d'autres hétéroatomes au côté de l'atome de N pris parmi les atomes de N, O et S et le reste est non substitué ou substitué par un ou plusieurs restes pris dans le groupe comprenant un atome d'halogène, un groupe alkyle en C₁-C₄ et un groupe oxo,
R⁴ représente un atome d'hydrogène, un groupe amino, alkylamino ou dialkylamino présentant chacun 1 à 6 atomes de carbone dans le reste alkyle, un reste hydrocarboné ou un reste hydrocarboxy acyclique ou cyclique présentant chacun 1 à 6 atomes de carbone, ou un reste hétérocyclyle, un reste hétérocyclyloxy ou reste hétérocyclylamino présentant chacun 3 à 6 atomes nucléaires et 1 à 3 hétéroatomes nucléaires pris parmi les atomes de N, O et S, chacun des cinq derniers restes cités pouvant être non substitué ou substitué par un ou plusieurs restes pris parmi le groupe comprenant un atome d'halogène, alkoxy en C₁-C₄, haloalkoxy en C₁-C₄ (alkyl en C₁-C₄)thio, alcényle en C₂-C₄, alcynyle en C₂-C₄, (alcényl en C₂-C₄)oxy, (alcynyl en C₂-C₄)oxy, hydroxy, amino, acylamino, mono- et dialkylamino, nitro, carboxy, cyano, azido, (alkoxy en C₁-C₄)carbonyle, (alkyl en C₁-C₄)-carbonyle, formyle, carbamoyle, mono- et di-(alkyl en C₁-C₄)aminocarbonyle, (alkyl en C₁-C₄)-sulfinyle, halo(alkyl en C₁-C₄)sulfinyle, (alkyl en C₁-C₄)sulfonyle, halo(alkyl en C₁-C₄)-sulfonyle et, dans le cas de restes cycliques, aussi par alkyle en C₁-C₄ et haloalkyle en C₁-C₄,
ou représente un reste acyle ou
R⁵ représente un atome d'hydrogène, un groupe nitro, cyano, thiocyanato ou un reste de formule -B²-Y², B² et Y² sont définis comme ci-dessous,
A-Z représente une liaison bivalente de formule
-(CH₂)ₐ-
où a est un entier qui vaut 2, 3 ou 4,
-W¹-(CH₂)_{b}-W²-,
où b est un entier qui vaut 1 ou 2, et W¹, W² représentent, indépendamment l'un de l'autre, un atome de O ou S,
-CH₂CH₂CH₂-W³-
où W³ représente un atome de O ou S,
(X')ₘ représente m substituants X' et dans ce contexte, chaque X' représente indépendamment l'un de l'autre, un atome d'halogène, un groupe alkyle en C₁-C₆, alcényle en C₂-C₆, alcynyle en C₂-C₆, alkoxy en C₁-C₆, alcényloxy en C₂-C₆, alcynyloxy en C₂-C₆, (alkyl en C₁-C₄)carbonyle, (alkoxy en C₁-C₄)-carbonyle ou [(alkyl en C₁-C₄)thio]carbonyle, les fragments contenant des restes hydrocarbonés dans les 9 derniers restes cités sont non substitués ou substitués par un ou plusieurs restes pris dans le groupe comprenant un atome d'halogène, un groupe alkoxy en C₁-C₄ et un groupe (alkyl en C₁-C₄)thio, ou le groupe oxo,
(X)ₙ représente n substituants X et dans ce contexte, X étant chaque fois, indépendamment les uns des autres, un atome d'halogène, un groupe nitro, cyano, alkyle en C₁-C₆, alkoxy en C₁-C₆, (alkyl en C₁-C₄)carbonyle, (alkoxy en C₁-C₄)-carbonyle ou (alkyl en C₁-C₄)-thiocarbonyle, les 5 derniers restes cités sont non substitués ou substitués par un substituant halogène ou alkoxy en C₁-C₄, ou représente un reste de formule -B³-Y³, B³ et Y³ étant définis ci-dessous, ou
deux restes X adjacents forment conjointement un cycle condensé présentant 4 à 6 atomes de carbone nucléaires, qui est carbocyclique ou renferme des hétéroatomes nucléaires pris parmi les atomes de O, S et N, et qui est non substitué ou substitué par un ou plusieurs restes pris dans le groupe comprenant les atomes d'halogène, un groupe alkyle en C₁-C₄, et oxo,
B¹, B², B³ représentent chacun, indépendamment les uns des autres, une liaison directe ou un groupe bivalent de formule -O-, -S-, -CO-, -O-CO-, -CO-O-, -NR'-, -NR'-CO- ou -CO-NR'-, R' représente un atome d'hydrogène ou un groupe alkyle en C₁-C₄,
Y¹, Y² représentent chacun, indépendamment l'un de l'autre, un atome d'hydrogène ou un reste hydrocarboné acyclique présentant 1 à 6 atomes de carbone, un reste hydrocarboné cyclique présentant 3 à 6 atomes de carbone ou un reste hétérocyclique présentant 3 à 9 atomes nucléaires et 1 à 3 hétéroatomes nucléaires pris parmi les atomes N, O et S, chacun des trois derniers restes cités étant non substitué ou substitué par un ou plusieurs restes pris dans le groupe comprenant un atome d'halogène, un groupe alkoxy en C₁-C₄, haloalkoxy en C₁-C₄, (alkyl en C₁-C₄)-thio, alcényle en C₂-C₄, alcynylé en C₂-C₄, (alcényl en C₂-C₄)oxy, (alcynyl en C₂-C₄)oxy, hydroxy, amino, acylamino, mono- et dialkylamino, nitro, carboxy, cyano, azido, (alkoxy en C₁-C₄)-carbonyle, (alkyl en C₁-C₄)carbonyle, formyle, carbamoyle, mono- et di-(alkyl en C₁-C₄)-aminocarbonyle, (alkyl en C₁-C₄)sulfinyle, halo(alkyl en C₁-C₄)sulfinyle, (alkyl en C₁-C₄)-sulfonyle, halo(alkyl en C₁-C₄)sulfonyle et, dans le cas de restes cycliques, également par alkyle en C₁-C₄ et haloalkyle en C₁-C₄,
Y³ représente un reste cycloalkyle en C₃-C₆, qui est non substitué ou substitué par des restes pris dans le groupe comprenant un groupe alkyle en C₁-C₄ et haloalkyle en C₁-C₄, ou représente phényle, qui est non substitué ou substitué par des restes pris dans le groupe comprenant un atome d'halogène, un groupe alkyle en C₁-C₄, haloalkyle en C₁-C₄ et alkoxy en C₁-C₄,
m vaut 0, 1 ou 2,
n vaut 0, 1, 2, 3 ou 4.

3. Composés de formule (I) ou leurs sels selon la revendication 1 ou 2, **caractérisés en ce que**
R¹ représente un groupe alkyle en C₁-C₄,
qui est non substitué ou substitué par un ou plusieurs restes pris dans le groupe comprenant un atome d'halogène, un groupe alkoxy en C₁-C₄, (alkyl en C₁-C₄)thio et phényle, qui est non substitué ou substitué par un ou plusieurs restes pris dans le groupe comprenant un atome d'halogène, un groupe alkyle en C₁-C₄ et haloalkyle en C₁-C₄, alkoxy en C₁-C₄, haloalkoxy en C₁-C₄, (alkyl en C₁-C₄)thio, nitro, cyano, (alkyl en C₁-C₂)carbonyle, formyle, carbamoyle, mono- et di-(alkyl en C₁-C₂)aminocarbonyle et (alkyle en C₁-C₄)sulfonyle, et cycloalkyle en C₃-C₇ qui est non substitué ou substitué par un ou plusieurs restes alkyle en C₁-C₄, ou
un groupe cycloalkyle en C₃-C₇ ou un reste hétérocyclique présentant 3 à 6 atomes nucléaires et 1 ou 2 hétéroatomes nucléaires pris parmi les atomes de N, O et S, le reste cycloalkyle ou le reste hétérocyclique étant chacun non substitué ou substitué par un ou plusieurs restes pris dans le groupe comprenant un atome d'halogène, un groupe nitro, cyano, thiocyanato et un reste de formule B¹-Y¹, B¹ et Y¹ sont définis comme ci-dessous, et/ou présente des substituants qui peuvent former par paires un cycle relié en forme spiranique pris dans le groupe de cycloalkyle en C₃-C₇, qui est non substitué ou substitué par un ou plusieurs restes pris dans le groupe comprenant un atome d'halogène, un groupe alkyle en C₁-C₄, haloalkyle en C₁-C₄ et oxo,
R² et R³ représentent chacun, indépendamment l'un de l'autre, un atome d'hydrogène, un groupe amino, formyle, alkyle en C₁-C₄, cyanoalkyle en C₁-C₄, (alkyl en C₁-C₄)amino ou di-(alkyl en C₁-C₄)amino, haloalkyle en C₁-C₄, hydroxyalkyle en C₁-C₄, (alkoxy en C₁-C₄)-(alkyle en C₁-C₄), (haloalkoxy en C₁-C₄)-(alkyle en C₁-C₄), alcényle en C₂-C₆, haloalcényle en C₂-C₆, alcynyle en C₂-C₆, haloalcynyle en C₂-C₆, (alkyl en C₁-C₄)amino-(alkyle en C₁-C₄), di-[(alkyl en C₁-C₄)-amino]-(alkyle en C₁-C₄), cyclo(alkyl en C₁-C₄)amino-(alkyle en C₁-C₄), cycloalkyle en C₃-C₉, (hétérocyclyle en C₃-C₉)-(alkyle en C₁-C₄), les groupes cycliques dans les trois derniers restes cités pouvant être non substitués ou substitués par un ou plusieurs restes pris dans le groupe comprenant un groupe alkyle en C₁-C₄, halogène et cyano, ou
un groupe phényle, phénoxy, phényl-carbonyle, phénoxycarbonyle, phénylcarbonyl-alkyle en C₁-C₄, (alkoxy en C₁-C₄)-carbonyl-(alkyle en C₁-C₄), (alkyl en C₁-C₄)aminocarbonyl-(alkyle en C₁-C₄), (alkyl en C₁-C₄)carbonyle, (alkoxy en C₁-C₄)-carbonyle, aminocarbonyle, (alkyl en C₁-C₄)-aminocarbonyle, phénoxyalkyle en C₁-C₄, phénylalkyle en C₁-C₄, hétérocyclyle, hétérocyclylamino, hétérocyclyloxy, hétérocyclylthio ou un des 17 derniers restes cités, qui est substitué dans le fragment acyclique ou dans le fragment cyclique par un ou plusieurs restes pris dans le groupe comprenant un atome un atome d'halogène, un groupe nitro, cyano, alkyle en C₁-C₄, alkoxy en C₁-C₄, (alkyl en C₁-C₄)thio, haloalkyle en C₁-C₄, haloalkoxy en C₁-C₄, formyle, (alkyl en C₁-C₄)carbonyle, (alkoxy en C₁-C₄)-carbonyle, alkoxy en C₁-C₄, le fragment hétérocyclyle dans les restes présentant chacun 3 à 9 atomes nucléaires et 1 à 3 hétéroatomes nucléaires pris parmi les atomes N, O et S,
R² et R³ forment conjointement avec l'atome d'azote du groupe NR²R³ un reste hétérocyclique présentant 3 à 6 atomes nucléaires et 1 ou 2 hétéroatomes nucléaires, présentant éventuellement d'autres hétéroatomes au côté de l'atome de N pris parmi les atomes de N, O et S et le reste est non substitué ou substitué par un ou plusieurs restes pris dans le groupe comprenant un atome d'halogène, un groupe alkyle en C₁-C₄ et oxo,
R⁴ représente un atome d'hydrogène, un groupe amino, formyle, alkyle en C₁-C₄, cyanoalkyle en C₁-C₄, (alkyl en C₁-C₄)amino, di-(alkyl en C₁-C₄)amino, haloalkyle en C₁-C₄, hydroxyalkyle en C₁-C₄, (alkoxy en C₁-C₄)-(alkyle en C₁-C₄), halo(alkoxy en C₁-C₄)-(alkyle en C₁-C₄), alcényle en C₂-C₆, alcynyle en C₂-C₆, haloalcényle en C₂-C₆, haloalcynyle en C₂-C₆, (alkyl en C₁-C₄)amino-(alkyle en C₁-C₄), di-(alkyl en C₁-C₄)amino-(alkyle en C₁-C₄), cyclo(alkyl en C₃-C₉)amino-(alkyle en C₁-C₄), cycloalkyle en C₃-C₉, hétérocyclyle en C₃-C₉)-(alkyle en C₁-C₄) présentant 3 à 9 chaînons, les groupes cycliques dans les trois derniers restes cités sont non substitués ou substitués par un ou plusieurs restes pris parmi le groupe comprenant un groupe alkyle en C₁-C₄, halogène et cyano, ou phényle, phénoxy, phénylcarbonyle, phénoxycarbonyle, phénylcarbonylalkyle en C₁-C₄, (alkoxy en C₁-C₄)-carbonyl-(alkyle en C₁-C₄), (alkyl en C₁-C₄)aminocarbonyl-(alkyle en C₁-C₄), (alkyl en C₁-C₄)carbonyle, (alkoxy en C₁-C₄)-carbonyle, aminocarbonyle, (alkyl en C₁-C₄)-aminocarbonyle, phénoxyalkyle en C₁-C₄, phénylalkyle en C₁-C₄, hétérocyclyle, hétérocyclylamino, hétérocyclyloxy, hétérocyclylthio, ou un des 17 derniers restes cités qui est substitué dans le fragment acyclique ou dans le fragment cyclique par un ou plusieurs restes pris dans le groupe comprenant un atome d'halogène, un groupe nitro, cyano, alkyle en C₁-C₄, alkoxy en C₁-C₄, (alkyl en C₁-C₄)thio, haloalkyle en C₁-C₄, haloalkoxy en C₁-C₄, formyle, (alkyl en C₁-C₄)-carbonyle, (alkoxy en C₁-C₄)carbonyle, alkoxy en C₁-C₄, le fragment hétérocyclyle dans les restes présentant chacun 3 à 9 atomes nucléaires et 1 à 3 hétéroatomes nucléaires pris parmi les atomes N, O et S,
R⁵ représente un atome d'hydrogène, un groupe alkyle en C₁-C₄, haloalkyle en C₁-C₄ ou cycloalkyle en C₃-C₉,
A-Z représente une liaison bivalente de formule
-CH₂-CH₂-, -CH₂CH₂CH₂-, -CH₂CH₂CH₂CH₂-,
-S-CH₂-S-, -S-CH₂-O-, -O-CH₂-O-,
-S-CH₂CH₂-S-, -S-CH₂CH₂-O-, -O-CH₂CH₂-O-,
-CH₂CH₂CH₂-O-, -CH₂CH₂CH₂-S-,
(X')ₘ représente m substituants X' et dans ce contexte, chaque X' représente, indépendamment l'un de l'autre, un atome d'halogène, un groupe alkyle en C₁-C₄, alcényle en C₂-C₄, alcynyle en C₂-C₄, alkoxy en C₁-C₄, alcényloxy en C₂-C₄, alcynyloxy en C₂-C₄, (alkyl en C₁-C₄)carbonyle, (alkoxy en C₁-C₄)-carbonyle ou (alkyl en C₁-C₄)thiocarbonyle, les fragments contenant des restes hydrocarbonés dans les 9 derniers restes cités sont non substitués ou substitués par un ou plusieurs restes pris dans le groupe comprenant un atome d'halogène, ou le groupe oxo,
(X)ₙ représente n substituants X et dans ce contexte, chaque X représente, indépendamment l'un de l'autre, un atome d'halogène, un groupe hydroxy, amino, nitro, formyle, carboxy, cyano, thiocyanato, alkyle en C₁-C₄, cyanoalkyle en C₁-C₄, alkoxy en C₁-C₄, (alkyl en C₁-C₄)amino, di-(alkyl en C₁-C₄)amino, haloalkyle en C₁-C₄, hydroxyalkyle en C₁-C₄, (alkoxy en C₁-C₄)-(alkyl en C₁-C₄), halo(alkoxy en C₁-C₄)-(alkyle en C₁-C₄), (alkyl en C₁-C₄)thio, halo(alkyl en C₁-C₄)thio, alcényle en C₂-C₆, haloalcényle en C₂-C₆, alcynyle en C₂-C₆, haloalcynyle en C₂-C₆, (alkyl en C₁-C₄)amino-(alkyle en C₁-C₄), di-(alkyl en C₁-C₄)amino-(alkyle en C₁-C₄), cyclo(alkyl en C₃-C₉) amino-(alkyle en C₁-C₄), cycloalkyle en C₃-C₉, hétérocyclylalkyle en C₁-C₄ présentant 3 à 9 chaînons, les groupes cycliques dans les 3 derniers restes cités sont non substitués ou substitués par un ou plusieurs restes pris dans le groupe comprenant un groupe alkyle en C₁-C₄, halogène et cyano, ou
un groupe phényle, phénoxy, phénylcarbonyle, phénoxy-carbonyle, phénylcarbonylalkyle en C₁-C₄, (alkoxy en C₁-C₄)-carbonyl-(alkyle en C₁-C₄), (alkyl en C₁-C₄)aminocarbonyl-(alkyle en C₁-C₄), (alkyl en C₁-C₄)carbonyle, (alkoxy en C₁-C₄)-carbonyle, aminocarbonyle, (alkyl en C₁-C₄)-aminocarbonyle, phénoxyalkyle en C₁-C₄, phénylalkyle en C₁-C₄, hétérocyclyle, hétérocyclylamino, hétérocyclyloxy, hétérocyclothio, ou un des 17 derniers restes cités qui est substitué dans le fragment acyclique ou dans le fragment cyclique par un ou plusieurs restes pris dans le groupe comprenant un atome d'halogène, un groupe nitro, cyano, alkyle en C₁-C₄, alkoxy en C₁-C₄, (alkyl en C₁-C₄)thio, haloalkyle en C₁-C₄, haloalkoxy en C₁-C₄, formyle, (alkyl en C₁-C₄)-carbonyle, (alkoxy en C₁-C₄)carbonyle, alkoxy en C₁-C₄, le fragment hétérocyclyle dans les restes présentant chacun 3 à 9 atomes nucléaires et 1 à 3 hétéroatomes nucléaires pris parmi les atomes N, O et S,
deux restes X adjacents forment conjointement un cycle condensé présentant 4 à 6 atomes de carbone nucléaires, qui est carbocyclique ou renferme des hétéroatomes nucléaires pris parmi les atomes de O, S et N, et qui est non substitué ou est substitué par un ou plusieurs restes pris dans le groupe comprenant des atomes d'halogène, un groupe alkyle en C₁-C₄ et oxo,
B¹ représente une liaison directe ou un groupe bivalent de formule -O-, -S-, -CO-, -O-CO-, -CO-O-, -NR'-, -NR'-CO- ou -CO-NR'-, R' représente un atome d'hydrogène ou un groupe alkyle en C₁-C₄,
Y¹ représente un atome d'hydrogène ou un reste hydrocarboné acyclique présentant 1 à 4 atomes de carbone, un reste hydrocarboné cyclique présentant 3 à 6 atomes de carbone ou un reste hétérocyclique présentant 3 à 6 atomes nucléaires et 1 à 3 hétéroatomes nucléaires pris parmi les atomes de N, O et S, chacun des trois derniers restes cités étant non substitué ou substitué par un ou plusieurs restes pris dans le groupe comprenant un atome d'halogène, un groupe alkoxy en C₁-C₄, halo(alkoxy en C₁-C₄), (alkyl en C₁-C₄)-thio, hydroxy, amino, mono- et dialkylamino, (alkoxy en C₁-C₄)carbonyle, (alkyl en C₁-C₄)-carbonyle, formyle, carbamoyle, mono- et di-(alkyl en C₁-C₄)aminocarbonyle et (alkyl en C₁-C₄)-sulfinyle, halo(alkyl en C₁-C₄)sulfinyle, (alkyl en C₁-C₄)sulfonyle, halo(alkyl en C₁-C₄)sulfonyle, et dans le cas de restes cycliques, également par alkyle en C₁-C₄ et haloalkyle en C₁-C₄.

4. Composés de formule (I) et leurs sels selon l'une des revendications 1 à 3, **caractérisés en ce que**
R¹ représente un groupe alkyle en C₁-C₄, haloalkyle en C₁-C₄, benzyle, (cycloalkyl en C₃-C₆)-(alkyle en C₁-C₂), cycloalkyle en C₃-C₆ ou un reste hétérocyclique présentant 3 à 6 atomes nucléaires et 1 ou 2 hétéroatomes nucléaires pris parmi les atomes de N et O, de préférence un atome d'oxygène en tant que hétéroatome nucléaire, le reste cycloalkyle ou le reste hétérocyclique étant chacun non substitué ou substitué par un ou plusieurs restes pris dans le groupe comprenant un atome d'halogène, un groupe nitro, cyano, thiocyanato et un reste de formule B¹-Y¹, B¹ et Y¹ sont définis comme ci-dessous, et/ou présente des substituants qui peuvent former par paires un cycle relié en forme spiranique pris dans le groupe de cycloalkyle en C₃-C₇, qui est non substitué ou substitué par un ou plusieurs restes pris dans le groupe comprenant un atome d'halogène, un groupe alkyle en C₁-C₄, haloalkyle en C₁-C₄ et oxo,
R² et R³ représentent chacun, indépendamment l'un de l'autre, un atome d'hydrogène, un groupe amino, formyle, alkyle en C₁-C₄, (alkoxy en C₁-C₄)-(alkyle en C₁-C₄), alcényle en C₃-C₆, alcynyle en C₃-C₆, (alkyl en C₁-C₄)amino-(alkyle en C₁-C₄), di(alkyl en C₁-C₄)amino-(alkyle en C₁-C₄) ou phényle, phénylalkyle en C₁-C₄, phénylcarbonyle ou phénoxycarbonyle ou un des derniers quatre restes cités qui est substitué dans le fragment phényle une à trois fois par des restes pris dans le groupe comprenant un atome d'halogène, halogène, un groupe alkyle en C₁-C₄, alkoxy en C₁-C₄ et (alkoxy en C₁-C₄)carbonyle, ou
R² et R³ forment conjointement avec l'atome d'azote du groupe NR²R³ un reste hétérocyclique présentant 3 à 6 atomes nucléaires et 1 ou 2 hétéroatomes nucléaires, présentant éventuellement d'autres hétéroatomes au côté de l'atome de N pris parmi les atomes de N, O et S, et le reste est non substitué ou substitué par un ou plusieurs restes pris dans le groupe comprenant un atome d'halogène, un groupe alkyle en C₁-C₄ et oxo,
R⁴ représente un atome d'hydrogène, un groupe amino, formyle, alkyle en C₁-C₄, di-(alkyl en C₁-C₄)amino, (alkoxy en C₁-C₄)-(alkyle en C₁-C₄), alcényle en C₃-C₆, alcynyle en C₃-C₆, di-(alkyl en C₁-C₄)amino-(alkyle en C₁-C₄), phényle, phénoxyalkyle en C₁-C₄, phénylalkyle en C₁-C₄, phénoxycarbonyle, phénylaminocarbonyle, ou un des cinq derniers restes cités qui est substitué dans le fragment phényle une à trois fois par des restes pris dans le groupe comprenant un atome d'halogène, un groupe alkyle en C₁-C₄, alkoxy en C₁-C₄ et (alkoxy en C₁-C₄) carbonyle,
R⁵ représente un atome d'hydrogène, un groupe alkyle en C₁-C₄, haloalkyle en C₁-C₄ ou cycloalkyle en C₃-C₉,
A-Z représente une liaison bivalente de formule
-CH₂-CH₂-, -CH₂CH₂CH₂-, -CH₂CH₂CH₂CH₂-,
-S-CH₂CH₂-S-, -S-CH₂CH₂-O-, -O-CH₂CH₂-O-,
-CH₂CH₂CH₂-O-, -CH₂CH₂CH₂-S-,
(X')ₘ représente m substituants X' et dans ce contexte, chaque X' représente indépendamment l'un de l'autre, un atome d'halogène, un groupe alkyle en C₁-C₄ ou alkoxy en C₁-C₄,
(X)ₙ représente n substituants X et dans ce contexte, chaque X représente, indépendamment l'un de l'autre, un atome d'halogène, un groupe hydroxy, alkyle en C₁-C₄ ou alkoxy en C₁-C₄,
B¹ représente une liaison directe ou un groupe bivalent de formule -O-, -S- ou -O-CO-,
Y¹ représente un atome d'hydrogène, un groupe alkyle en C₁-C₄, cycloalkyle en C₃-C₆ ou phényle, qui est non substitué ou substitué par un ou plusieurs restes pris dans le groupe comprenant un atome d'halogène, un groupe alkyle en C₁-C₄, haloalkyle en C₁-C₄, alkoxy en C₁-C₄, haloalkoxy en C₁-C₄, mono- et dialkylamino.

5. Composés de formule (I) et leurs sels selon l'une des revendications 1 à 4, **caractérisés en ce que**
R¹ représente un groupe alkyle en C₁-C₄, haloalkyle en C₁-C₄, (cycloalkyl en C₃-C₆)méthyle,
R² et R³ représentent chacun, indépendamment l'un de l'autre, un atome d'hydrogène, un groupe formyle ou alkyle en C₁-C₄,
R² et R³ forment conjointement avec l'atome d'azote du groupe NR²R³ un reste hétérocyclique présentant 4 à 6 atomes nucléaires et 1 à 2 hétéroatomes nucléaires, présentant éventuellement d'autres hétéroatomes au côté de l'atome de N pris parmi les atomes de N et O,
R⁴ représente un atome d'hydrogène ou un groupe alkyle en C₁-C₄,
R⁵ représente un atome d'hydrogène ou un groupe alkyle en C₁-C₄,
A-Z représente une liaison bivalente de formule
-CH₂-CH₂-, -CH₂CH₂CH₂-, -CH₂CH₂CH₂CH₂-,
-S-CH₂CH₂-S-, -O-CH₂CH₂-O-,
-CH₂CH₂CH₂-O-, -CH₂CH₂CH₂-S-,
(X')ₘ représente m substituants X' et dans ce contexte, chaque X' représente, indépendamment l'un de l'autre, un atome d'halogène ou un groupe alkyle en C₁-C₄,
(X)ₙ représente n substituants X et dans ce contexte, chaque X représente, indépendamment l'un de l'autre, un atome d'halogène, un groupe hydroxy, alkyle en C₁-C₄ ou alkoxy en C₁-C₄,

6. Procédé pour la préparation des composés de formule générale (I) ou de leurs sels tels que définis à la revendication 1, **caractérisés en ce qu'**on fait réagir
a) un composé de formule (II)
**R**^{**1**}**-Fu (II)**
où Fu est un groupe fonctionnel pris parmi le groupe comprenant des ester d'acide carboxylique, orthoester d'acide carboxylique, chlorure d'acide carboxylique, amide d'acide carboxylique, anhydride d'acide carboxylique et trichlorométhyle,
sur un biguanidide de formule (III) ou un sel d'addition d'acide de celui-ci 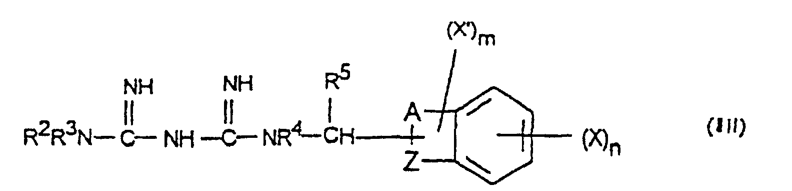 ou
b) un composé de formule (IV) 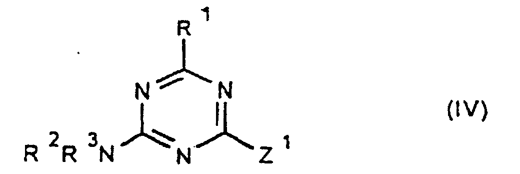 où Z¹ représente un reste échangeable ou un groupe partant, par exemple chlore, trichlorométhyle, (alkyl en C₁-C₄)sulfonyle et phényl(alkyl en C₁-C₄)sulfonyle ou (alkyl en C₁-C₄)phénylsulfonyle non substitué ou substitué, sur une amine appropriée de formule (V) ou un sel d'addition d'acide de celui-ci 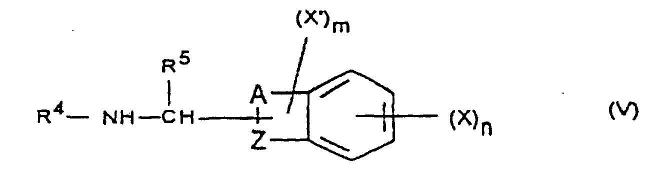
dans les formules (II), (III), (IV) et (V) les restes R¹, R², R³, R⁴, R⁵, A, X', X, m et n sont définis comme à la formule (I).

7. Agent herbicide ou de croissance de plantes, **caractérisé en ce qu'**il renferme au moins un composé de formule (I) ou son sel selon l'une des revendications 1 à 5 et des adjuvants de formulation usuels dans le domaine phytosanitaire.

8. Procédé pour la lutte contre les plantes adventices ou pour la régulation de la croissance des plantes, **caractérisé en ce qu'**on applique une quantité efficace d'au moins un composé de formule (I) ou leurs sels selon l'une des revendications 1 à 5 sur les plantes, les graines de plantes ou la surface cultivable.

9. Utilisation des composés de formule (I) ou leurs sels selon l'une des revendications 1 à 5 en tant qu'herbicides et régulateurs de croissance de plantes.
